# EUROPEAN PATENT APPLICATION

(11) **EP 4 389 758 A1**
(43) Date of publication of application: **26.06.2024**
(21) Application number: 22858549.3
(22) Date of filing: 19.08.2022
(51) Int. Cl.: C07K 7/08, A61K 38/10, A61K 38/12, A61K 47/64, A61K 47/65, A61K 47/66, A61K 49/00, A61P 25/00, C07K 19/00

(54) **HUMAN TRANSFERRIN RECEPTOR?BINDING PEPTIDE**

(30) Priority: 19.08.2021 JP 2021134377
(71) Applicant: PeptiDream Inc., Kawasaki-shi, Kanagawa 210-0821 (JP); JCR Pharmaceuticals Co., Ltd., Ashiya-shi, Hyogo 659-0021 (JP)
(72) Inventor: OHUCHI Masaki, Kawasaki-shi, Kanagawa 210-0821 (JP); TAKUWA Masatoshi, Kawasaki-shi, Kanagawa 210-0821 (JP); YAMAKOSHI Shuhei, Kawasaki-shi, Kanagawa 210-0821 (JP); TAKAHASHI Kenichi, Kobe-shi, Hyogo 651-2241 (JP); YODEN Eiji, Kobe-shi, Hyogo 651-2241 (JP); HASHIMOTO Hidehiko, Kobe-shi, Hyogo 651-2241 (JP); ONOUCHI Takashi, Kobe-shi, Hyogo 651-2241 (JP); FUJIYAMA Saki, Kobe-shi, Hyogo 651-2241 (JP)
(74) Representative: van Dam, Vincent
(86) International application number: PCT/JP2022/031422
(87) International publication number: WO 2023/022234

(57) **Abstract**

Novel peptides that bind to the human transferrin receptor (hTfR) are provided. The peptide is a peptide comprising an amino acid sequence described in Ala-Val-MePhe-Val-Trp-Asn-Tyr-Tyr-Ile-Ile-K(MePEG4c)-Arg-Phe-MeTyr-Cys (SEQ ID NO: 1), or the peptide comprising one or more predetermined substitutions in the amino acid sequence described in SEQ ID NO:1.

## Description

### Technical Field

The present invention relates to a peptide that can bind to the human transferrin receptor (hTfR).

### Background of the Invention

In capillaries that supply blood to most tissues of the brain, except for some areas including the circumventricular organs (pineal gland, pituitary gland, area postrema, etc.), unlike capillaries in muscles and other tissues, the endothelial cells that form their endothelium are tightly attached to each other by strong intercellular junctions. Therefore, passive transport of substances from the blood to the brain is prevented, and although there are exceptions, substances other than highly fat-soluble substances or substances with small molecular weight (less than 200 to 500 Daltons) and electrically neutral near physiological pH are difficult to be transported from the capillaries to the brain. Such a mechanism that restricts the exchange of substances between blood and brain tissue fluid via the capillary endothelium in the brain is called the blood-brain barrier (BBB). The BBB also restricts the exchange of substances between blood and tissue fluids of the central nervous system, including the brain and spinal cord, as well as the brain. The presence of the BBB allows most of the cells of the central nervous system to maintain their biochemical homeostasis without being affected by fluctuations in the concentrations of hormones, lymphokines and other substances in the blood.

As a method to allow macromolecular substances to reach the brain through the BBB, various methods have been reported to modify the macromolecular substances so that they have affinity with a transferrin receptor, which are membrane proteins existing on endothelial cells of capillaries in the brain (Patent Documents 1 to 3). For example, as Patent Document 1 describes a blood-brain barrier shuttle that has affinity to the transferrin receptor and can bind to the receptor, it is known that a substance that can bind to the transferrin receptor has the probability of passing through the blood-brain barrier.

### Prior Art Documents

### Patent Documents

[Patent Document 1] JP 2015-528452 A (Translation of PCT Application)
[Patent Document 2] JP H06-228199 A
[Patent Document 3] WO2016/208695 A
[Patent Document 4] WO2019/151539 A

### Summary of the Invention

### Problems to be Solved by the Invention

One invention described in this specification aims to provide a novel peptide that binds to the human transferrin receptor (hTfR).

Another invention aims to provide various uses for the above novel peptide.

### Solution to Problem

A certain invention described in this specification relates to a peptide that binds to the transferrin receptor.

The peptide comprises an amino acid sequence described in Ala-Val-MePhe-Val-Trp-Asn-Tyr-Tyr-Ile-Ile-K(MePEG4c)-Arg-Phe-MeTyr-Cys (SEQ ID NO: 1), or the peptide comprises an amino acid sequence with one or more substitutions selected from:
(I) substitution of the 1st alanine residue in the SEQ ID NO: 1 for a MeA residue;
(II) substitution of the 3rd N-methylphenylalanine residue in the SEQ ID NO: 1 for an amino acid which contain an aromatic ring, which may have a substituent, in a side chain, or an N-methylamino acid;
(III) substitution of the 5th tryptophan residue in the SEQ ID NO: 1 for tryptophan which has a substituent or N-methyltryptophan;
(IV) substitution of the 6th asparagine residue of the SEQ ID NO: 1 for alanine or a positively charged amino acid;
(V) substitution of the 7th tyrosine residue of the SEQ ID NO: 1 for an amino acid having an aromatic ring, which may have a substituent, in a side chain, or an alkyl group of C₄ or more;
(VI) substitution of the 8th tyrosine residue of the SEQ ID NO: 1 for an amino acid having an aromatic ring, which may have a substituent which may have a linker, in a side chain, or N-methylamino acid;
(VII) substitution of the 9th isoleucine residue of the SEQ ID NO: 1 for an aliphatic amino acid;
(VIII) substitution of the 10th isoleucine residue of the SEQ ID NO: 1 for an amino acid having a butyl group, which may have a substituent, in a side chain;
(IX) substitution of the 11th K(MePEG4c) residue of the SEQ ID NO: 1 for Aib, or a hydrophilic amino acid which may have a linker or N-methylamino acid;
(X) substitution of the 12th arginine residue of the SEQ ID NO: 1 for a hydrophilic amino acid (including D-body) which may have a linker, or N-methylamino acid;
(XI) substitution of the 13th phenylalanine of the SEQ ID NO: 1 for an amino acid having an aromatic ring, which may have a linker, in a side chain; or
(XII) substitution of the 14th N-methyltyrosine residue of the SEQ ID NO: 1 for N-methylamino acid having an aromatic ring, which may have a linker, in the side chain.

### Advantageous Effects of the Invention

The invention described in this specification provides the peptide that binds to the human transferrin receptor (hTfR) and others, as demonstrated by the examples.

### Detailed Description of Embodiments

The following is a description of embodiments for carrying out the present invention. The present invention is not limited to the embodiments described below, but also includes modifications made from the following embodiments as appropriate within the range obvious to those skilled in the art.

A certain invention described in this specification relates to a peptide that binds to a transferrin receptor.

### Transferrin Receptor

The transferrin receptor (also called transferrin acceptor) is a receptor that binds to transferrin, a protein which is found in plasma and binds to iron ions, and has the function of taking them into cells, and also represents a protein known to take a dimeric structure. Transferrin receptor is expressed on various cells such as reticulocytes, placental trophoblasts, and lymphocytes, and it has been suggested that transferrin receptor is particularly expressed on tumor cells. Since the transferrin receptor has the property of triggering cellular endocytosis by stimulating binding of iron ion in plasma, research is underway to use antibodies or the like that bind to the transferrin receptor as a DDS to allow desired substances to pass through the BBB. There are two types of transferrin receptors, type I and type II, and type I (Gene ID: 7037) is preferred as the transferrin receptor in the present invention. In this specification, the human-type transferrin receptor is referred to as the human TfR, hTfR, or simply TfR, unless otherwise noted.

### Peptides That Bind to Transferrin Receptor

Binding to the transferrin receptor (also referred to as having binding activity or affinity) means binding specifically to the transferrin receptor.

Affinity is expressed by the equilibrium constant (KD) for dissociation between the transferrin receptor and the binding peptide, which is a measure of the binding strength between the transferrin receptor and the binding peptide, and as the value of KD decreases, the binding strength between the transferrin receptor and the binding peptide becomes stronger (alternatively, affinity can be expressed as an affinity constant (KA), which is 1/KD). As will be clear to those skilled in the art (e.g., based on further disclosure herein), affinity can be determined in a manner that is known per se, depending on the type and nature of the substance to be bound. Binding activity is also a measure of the strength of the binding between the transferrin receptor and the binding peptide. Binding activity is related to both the affinity between the transferrin receptor and its binding site on the binding peptide and the number of relevant binding sites present on the binding molecule.

Specific binding of the transferrin receptor and the binding peptide can be determined by any suitable method known per se, including, for example, surface plasmon resonance (SPR) assay, Scatchard analysis and/or competitive binding assays such as radioimmunoassay (RIA), enzyme immunoassay (EIA) and sandwich competition assay described herein, including different variants thereof that are known per se in the art. Preferably, the affinity of the peptide of the invention and the transferrin receptor may be less than 100 nM, preferably less than 50 nM, more preferably less than 20 nM, and even more preferably less than 10 nM KD, and, although it is not limited, it may be about 10⁻⁵ M to about 10⁻⁹ M, or in another manner, less than 10⁻⁷ M, for example, 10⁻⁷ M to 10⁻¹³ M, for example, 10⁻⁹ M to 10⁻¹³ M. Peptides are provided herein with binding capacities of various strengths, ranging from low to high binding capacities with the transferrin receptor in the above range.

### Peptide

It refers to a structure with multiple consecutive amino acids, and includes polypeptides and proteins. In this application, the term "amino acid" includes not only naturally occurring amino acids (natural amino acids) that are incorporated into peptide chains by translation of mRNA in cells, but also unnaturally occurring amino acids (unnatural amino acids) that can form part of a peptide chain by peptide bonding. Amino acids may be artificially synthesized or naturally occurring.

In the present application, peptides in which cyclic portion is formed by cyclization after synthesis (also called cyclic peptides) and peptides obtained by further chemical modification of such peptides are also included in the peptides of the present invention.

In this specification, a cyclic peptide means a peptide that is cyclic in whole or in part by the bonding of two amino acids separated by one or more amino acid residues in the amino acid sequence between them. Although there is no restriction on the bonding type between the two amino acids, amide bond between the carboxyl group of one amino acid and the amino group of the other amino acid, thioether bond between the carboxyl group of one amino acid and the thiol group of the other amino acid, thiol bond between the thiol group of one amino acid and the thiol group of the other amino acid, those with a cyclic structure formed by lactam ring formation or macrocyclization reaction, and those with lasso-peptide-like structures are also included in the cyclic peptide. However, when the two amino acids are bonded by the amide bond, the amide bond is not limited to those formed by the bonding of the carboxyl group of one amino acid with the amino group of the other amino acid, but only if the amide bond is formed as a result of a synthetic reaction. The same is true for other bond types.

That is, in the present application, a cyclic peptide may have a linear portion, as long as a portion of the cyclic peptide forms a cyclic structure. It may also take a complex cyclic structure, such as a bicyclic structure in which two amino acids in a cyclic peptide forming a single cyclic structure are further joined together.

In this specification, a part of amino acid may be modified for cyclization of peptides. Such partially modified amino acid is also included in the amino acid of this application. For example, a chloroacetyl group is added to the amino acid located at the N-terminal end, which is combined with a cysteine residue in the peptide to form a ring, and various (natural/unnatural) amino acids to which a chloroacetyl group is added are also included in the amino acid of the present application.

Non-natural amino acids are compounds other than natural amino acids that have the characteristics of amino acids. Examples include, but not limited to, amino acids that do not constitute proteins *in vivo,* such as β-amino acid, γ-amino acid, L-amino acid, D-amino acid (also called D-type amino acid), amino acid mutant, chemically modified amino acid such as amino acid derivative, norleucine, β-alanine, ornithine, etc. Also, Examples include N-methylamino acid, N-ethylamino acid, D-amino acid, histidine-like amino acid, amino acids having structures such as extra methylene or aromatic ring in the side chain, and amino acid derivatives having structures in which the carboxylic acid functional group in the side chain is replaced by a sulfonic acid group.

Examples of unnatural amino acids and their abbreviations in this specification are given below. CAS reference numbers or sources of purchase are given in parentheses, and synthetic example numbers are given for newly synthesized compounds. Specialty amino acids are not limited to those listed above and, for example, those with a structure in which one or more of the hydrogen atoms in the molecule is replaced by an alkyl group are also specialty amino acids. When a hydrogen atom is replaced by an alkyl group, the alkyl group is preferably a methyl group or an ethyl group, more preferably a methyl group. In this specification, amino acids with Me or N-Me in front of the amino acid name indicate N-methylamino acids unless otherwise noted. For example, N-methylated amino acid of alanine (Ala or A) is indicated as MeAla, N-MeAla, MeA or N-MeA. In addition, amino acids with a single letter notation and with the letter d in front of it are indicated as D-amino acids. For example, the D-amino acid of alanine (Ala or A) is indicated as da. The following amino acids can be used in peptide synthesis by Fmoc-protecting the alpha-amino group by known methods.

W7N: L-7-Azatryptophan (CAS No. 49758-35-2)
KCOpipzaa: N6-(4-(Carboxymethyl) piperazine-1-carbonyl)-L-lysine (Kishida Chemical Co., Ltd.)
4Py: L-4-Pyridylalanine (CAS No. 37535-49-2)
PeG: N-Phenethylglycine (CAS No. 7738-38-7)
3Py: 3-(3-Pyridyl)-L-alanine (CAS No. 64090-98-8)
3Py6NH2: (S)-2-Amino-3-(6-aminopyridin-3-yl) propanoic acid (CAS No. 1269968-61-7)
A4paa: (S)-2-Amino-3-(1-(carboxymethyl) piperidin-4-yl) propanoic acid (Kishida Chemical Co., Ltd.)
dkCOmeglumine: N6-(Methyl((2S,3R,4R,5R)-2,3,4,5,6-pentahydroxyhexyl) carbamoyl)-D-lysine (Synthesis Example 1-7)
F3COO: (S)-3-(2-Amino-2-carboxyethyl) benzoic acid (CAS No. 13861-02-4)
F3COO (allyl): (S)-3-(3-((Allyloxy) carbonyl) phenyl)-2-aminopropanoic acid (Synthesis

### Example 1-1)

F4aao: (S)-2-Amino-3-(4-(carboxymethoxy) phenyl) propanoic acid (CAS No. 24558-63) -2)
F4COO (allyl): (S)-3-(4-((Allyloxy) carbonyl) phenyl)-2-aminopropanoic acid (Synthesis

### Example 1-2)

F4OMe: (S)-2-Amino-3-(4-methoxyphenyl) propanoic acid (CAS No. 6230-11-1)
KdMe: N6,N6-Dimethyl-L-lysine (CAS No. 2259-86-1)
Kmor: (S)-2-Amino-6-morpholinohexanoic acid (CAS No. 960135-14-2) (Synthesis Example 1-10)
KN3: N6-Diazo-L-lysine (CAS No. 159610-92-1)
Me3Py: (S)-2-(Methylamino)-3-(pyridin-3-yl) propanoic acid (CAS No. 2651172-69-7)
Me4Py: (S)-2-(Methylamino)-3-(pyridin-4-yl) propanoic acid
MeA: Methyl-L-alanine (CAS No. 3913-67-5)
MeF: Methyl-L-phenylalanine (CAS No. 2566-30-5)
MeF3C: (S)-3-(3-Chlorophenyl)-2-(methylamino) propanoic acid (CAS No. 2255324-91-3)MeF3COO: (S)-3-(2-Carboxy-2-(methylamino) ethyl) benzoic acid (CAS No. 1499826) -56-0)
MeF3COO (allyl): (S)-3-(3-((Allyloxy) carbonyl) phenyl)-2-(methylamino) propanoic acid

### (Synthesis Example 1-3)

MeF4F: (S)-3-(4-Fluorophenyl)-2-(methylamino) propanoic acid (CAS No. 347851-71-2)
MeR: Methyl-L-arginine (CAS No. 2480-28-6)
MeW: Methyl-L-tryptophan (CAS No. 526-31-8)
MeY: Methyl-L-tyrosine (CAS No. 537-49-5)
QhEt: N5-(2-Hydroxyethyl)-L-glutamine (CAS No. 2650-74-0) (Synthesis Example 1-4)
Qpipzaa: (S)-2-Amino-5-(4-(carboxymethyl) piperazin-1-yl)-5-oxopentanoic acid (Synthesis

### Example 1-5)

W1aa: 1-(Carboxymethyl)-L-tryptophan (CAS No. 773823-50-0)
W1aa (allyl): 1-(2-(Allyloxy)-2-oxoethyl)-L-tryptophan (Synthesis Example 1-6)
W5C: (S)-2-Amino-3-(5-chloro-1H-indol-3-yl) propanoic acid (CAS No. 52448-15-4)
Ndm: N4,N4-Dimethyl-L-asparagine (CAS No. 62937-43-3)
F4C: N-α-Chloroacetyl-4-chloro-L-phenylalanine (CAS No. 14173-39-8)
dr: D-Arginine
dk: D-lysine
Aib: α-Methylalanine (CAS No. 62-57-7)
Ahp/Alahp: (S)-2-Aminoheptanoic acid (CAS No. 1115-90-8)
MeF3COO (PEG4Me): (S)-3-(3-((2,5,8,11-Tetraoxatridecan-13-yl) carbamoyl) phenyl)-2-(methylamino) propanoic acid (Synthesis Example 1-11)
MeF4COO (PEG4Me): (S)-3-(4-((2,5,8,11-Tetraoxatridecan-13-yl) (carbamoyl)phenyl)-2-(methylamino)propanoic acid (Synthesis Example 1-8)
F3COO (PEG4Me): (S)-3-(3-((2,5,8,11-Tetraoxatridecan-13-yl) carbamoyl) phenyl)-2-aminopropanoic acid (Synthesis Example 1-12)
K (Mecar): N6-(Methoxycarbonyl)-L-lysine (CAS No. 74761-45-8)
K (MePEG4c): (S)-20-Amino-14-oxo-2,5,8,11-tetraoxa-15-azahenicosane-21-oic acid (CAS No. 1188295-19-3)
E (PEG1Me): N5-(2-Methoxyethyl)-L-glutamine (CAS No. 132432-96-3)
E (NHdPEG1Me): N5-(1,3-Dimethoxypropan-2-yl)-L-glutamine
E (PEG4Me): (S)-18-Amino-15-oxo-2,5,8,11-tetraoxa-14-azanonadecane-19-oic acid (CAS No. 2234872-12-7)
E (PEG8Me): (S)-30-Amino-27-oxo-2,5,8,11,14,17,20,23-octaoxa-26-azahentriacontane-31-oic acid
E (Glucamine): N5-((2S,3R,4R,5R)-2,3,4,5,6-Pentahydroxyhexyl)-L-glutamine (CAS No. 1956384-48-7)
MeF4COO (PEG8Me): (S)-3-(4-((2,5,8,11,14,17,20,23-octaoxapentacosan-25-yl) carbamoyl) phenyl)-2-(methylamino) propanoic acid (Synthesis Example 1-13)
F3COO (PEG8Me): (S)-3-(3-((2,5,8,11,14,17,20,23-Octaoxapentacosan-25-yl) carbamoyl) phenyl)-2-aminopropanoic acid (Synthesis Example 1-9)
F4aao (PEG8Me): (S)-2-Amino-3-(4-((27-oxo-2,5,8,11,14,17,20,23-octaoxa-26-azaoctacosan-28-yl) oxy) phenyl) propanoic acid
all: L-Alloisoleucine (CAS No. 1509-34-8)
da: D-Alanine
de: D-Glutamic acid
ds: D-Serine
K (Mecar): N6-(Methoxycarbonyl)-L-lysine (CAS No. 74761-45-8)
PEG4c or PEG3: 1-Amino-3,6,9,12-tetraoxapentadecan-15-oic acid (CAS No. 663921-15-1) PEG8c: 1-Amino-3,6,9,12,15,18,21,24-octaoxaheptacosane-27-oic acid (CAS No. 756526-04-2)
PEG8Me: 2,5,8,11,14,17,20,23-Octaoxapentacosane-25-amine (CAS No. 869718-81-0) PEG12c or PEG11 or PEG12: 1-Amino-3,6,9,12,15,18,21,24,27,30,33,36-dodecaoxanonatriacontan-39-oic acid (CAS No. 1415408-69-3)
MePEG4c: 2,5,8,11-Tetraoxatetradecane-14-oic acid (CAS No. 67319-28-2)
PEG1Me: 2-Methoxyethane-1-amine (CAS No. 109-85-3)
PEG4Me: 2,5,8,11-Tetraoxotridecan-13-amine (CAS No. 85030-56-4)
NHdPEG1Me: 1,3-Dimethoxypropan-2-amine (CAS No. 78531-29-0)
BCNOCO: ((1R,8S,9s)-Bicyclo[6,1,0]non-4-yn-9-yl) methyl (2,5-dioxopyrrolidin-1-yl) carbonate (CAS No. 1426827-79-3)
K (BCNOCO): N6-(Methyl ((2S,3R,4R,5R)-2,3,4,5,6-pentahydroxyhexyl) carbamoyl ((1R,8S,9s)-bicyclo[6,1,0]non-4-yn-9-yl) methoxy)carbonyl)-L-lysine (CAS No. (N/A) 1493802-96-2)
C (AcNMe): S-(2-(Methylamino)-2-oxoethyl)-L-cysteine
Cit: L-Citrulline (CAS No. 372-75-8)
K (Ac): N6-Acetyl-L-lysine (CAS No. 692-04-6)
K (C5Mal): N6-(6-(2,5-Dioxo-2,5-dihydro-1H-pyrrol-1-yl) hexanoyl)-L-lysine
PEG24c: 1-Amino-3,6,9,12,15,18,21,24,27,30,33,36,39,42,45,48,51,54,57,60,63,66,69,72-tetracosaoxapentaheptacontane-75-oic acid (CAS No. 2563873-76-5)
K (Maleimide): N6-(4-((2,5-Dioxo-2,5-dihydro-1H-pyrrole-1-yl) methyl) cyclohexane-1-carbonyl)-L-lysine

The newly synthesized amino acids are useful in the production of various peptide derivatives because they may add new functions to various peptides.

The peptide of the present invention comprises an amino acid sequence described in Ala-Val-MePhe-Val-Trp-Asn-Tyr-Tyr-Ile-Ile-K(MePEG4c)-Arg-Phe-MeTyr-Cys (SEQ ID NO: 1), or the peptide comprises an amino acid sequence with one or more substitutions selected from:
(I) substitution of the 1st alanine residue in the SEQ ID NO: 1 for a MeA residue;
(II) substitution of the 3rd N-methylphenylalanine residue in the SEQ ID NO: 1 for an amino acid which contain an aromatic ring, which may have a substituent, in a side chain, or an N-methylamino acid;
(III) substitution of the 5th tryptophan residue in the SEQ ID NO: 1 for tryptophan which has a substituent or N-methyltryptophan;
(IV) substitution of the 6th asparagine residue of the SEQ ID NO: 1 for alanine or a positively charged amino acid;
(V) substitution of the 7th tyrosine residue of the SEQ ID NO: 1 for an amino acid having an aromatic ring, which may have a substituent, in a side chain, or an alkyl group of C₄ or more;
(VI) substitution of the 8th tyrosine residue of the SEQ ID NO: 1 for an amino acid having an aromatic ring, which may have a substituent which may have a linker, in a side chain, or N-methylamino acid;
(VII) substitution of the 9th isoleucine residue of the SEQ ID NO: 1 for an aliphatic amino acid;
(VIII) substitution of the 10th isoleucine residue of the SEQ ID NO: 1 for an amino acid having a butyl group, which may have a substituent, in a side chain;
(IX) substitution of the 11th K(MePEG4c) residue of the SEQ ID NO: 1 for Aib, or a hydrophilic amino acid which may have a linker or N-methylamino acid;
(X) substitution of the 12th arginine residue of the SEQ ID NO: 1 for a hydrophilic amino acid (including D-body) which may have a linker, or N-methylamino acid;
(XI) substitution of the 13th phenylalanine of the SEQ ID NO: 1 for an amino acid having an aromatic ring, which may have a linker, in a side chain; or
(XII) substitution of the 14th N-methyltyrosine residue of the SEQ ID NO: 1 for N-methylamino acid having an aromatic ring, which may have a linker, in the side chain.

Each of the above options (I) through (XII) may be selected in any combination.

### Conservative Amino Acid Substitution

A "conservative amino acid substitution" means a substitution with a functionally equivalent or similar amino acid. In general, a substitution within a group can be considered conservative with respect to structure and function. However, as should be obvious to one skilled in the art, the role played by a particular amino acid residue can be determined by its implications in the three-dimensional structure of the molecule containing that amino acid. For example, a cysteine residue can take a less polar, oxidized (disulfide) form compared to the reduced (thiol) form. The long aliphatic portion of the arginine side chain can constitute a structurally and functionally important feature. Also, side chains containing an aromatic ring (tryptophan, tyrosine, phenylalanine) can contribute to ion-aromatic or cation-pi interactions. In such cases, substitution of amino acids with these side chains for amino acids belonging to acidic or nonpolar groups can be structurally and functionally conservative. Residues such as proline, glycine and cysteine (disulfide form) can have a direct effect on the conformation of the main chain and often cannot be replaced without structural distortion.

Conservative amino acid substitution, as shown below, includes specific substitution based on the similarity of side chains (Lehninger, Biochemistry, Revised 2nd Edition, published in 1975, pp. 73 to 75: L. Lehninger, Biochemistry, 2nd edition, pp. 73 to 75, Worth Publisher, New York (1975)) and typical substitution.

Further to conservative amino acid substitution, for example, in the group obtained by dividing natural amino acids such as the following based on the properties of their common side chains, substitution for an amino acid belonging to the same group as the group to which a certain amino acid belongs is preferable.
(1) Hydrophobic (also called non-polar) amino acids: Amino acids that exhibit hydrophobic (also called non-polar) and include alanine ("Ala" or simply "A"), glycine ("Gly" or simply "G"), valine ("Va!" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), methionine ("Met" or simply "M").

The hydrophobic amino acids can be further divided into the following groups.

Aliphatic amino acids: Amino acids with fatty acids or hydrogen in the side chain, including Ala, Gly, Val, Ile and Leu.

Aliphatic and branched-chain amino acids: Amino acids with branched fatty acids in the side chain, including Val, Ile and Leu.

Aromatic amino acids: Amino acids having aromatic rings in the side chain, including Trp, Tyr and Phe.

(2) Hydrophilic (also called polar) amino acids: Amino acids that exhibit hydrophilicity (polarity) and include serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), lysine ("Lys" or simply "K"), arginine ("Arg" or simply "R"), and histidine ("His" or simply "H").

The hydrophilic amino acids can be further divided into the following groups.

Acidic amino acids: Amino acids whose side chains are acidic, including Asp and Glu.

Basic amino acids: Amino acids whose side chains are basic, including Lys, Arg and His.

Neutral amino acids: Amino acids whose side chains indicate neutrality, including Ser, Thr, Asn, Gln and Cys.

Gly and Pro can be divided into "amino acids affecting the direction of the main chain", and amino acids containing a sulfur molecule in the side chain, Cys and Met, can be divided into "sulfur-containing amino acids".

The group with aromatics in the side chain includes Trp, Tyr and Phe.

In the present specification, "amino acid" includes not only natural amino acids but also unnatural amino acids. Unnatural amino acids include, for example, N-alkyl amino acids in which a natural amino acid described above is N-alkylated; and those modified with lower alkyl groups (for example, of C1 to C5, preferably C1 to C3, and more preferably C1) in which the nitrogen forming a peptide bond is branched or not branched. Among the N-alkyl amino acids, N-ethyl amino acid, N-butyl amino acid, or N-methyl amino acid is preferable, and N-methyl amino acid is more preferable. C₄ or more alkyl group means a linear or branched alkyl group having 4 or more carbons. Examples of C₄ or more alkyl groups are C₄₋₁₀ alkyl groups, which may be C₄₋₆ alkyl groups or C₆₋₁₀ alkyl groups. Furthermore, unnatural amino acids also include D-type amino acids (also referred to as D-amino acids), chemically modified amino acids such as β-amino acids, γ-amino acids, amino acid variants, amino acid derivatives, or the like; amino acids that are not constituent materials for proteins *in vivo,* such as norleucine, ornithine, or the like; or the like. Also included are amino acids to which a functional group is further added to the side chain of a natural amino acid or substituted for another functional group (for example, an amino acid having a substitution or an addition in a part such as an arylene group, an alkylene group, or the like of the side chain; an amino acid wherein the arylene group, alkylene group or the alkyl group of the side chain has an increased C-number; an amino acid having a substitution in the aromatic ring of the side chain; an amino acid derivative with the structure in which the carboxylic acid functional group in the side chain is replaced by the sulfonic acid group; a heterocyclic or condensed cyclic amino acid; or the like).

Note that by adding or substituting a structure such as a functional group in the side chain of a natural amino acid, a property different from that of the natural amino acid may be imparted. Namely, the group described above, obtained by dividing natural amino acids based on the properties of their common side chains, may include unnatural amino acids having the same side chain property. For example, N-methylalanine (MeA), which is an amino acid in which the nitrogen atom in the main chain of alanine belonging to an aliphatic amino acid is methylated, is an unnatural amino acid, but it may be classified as a hydrophobic amino acid because it exhibits hydrophobic (nonpolar) property. Thus, an unnatural amino acid exhibiting the same side chain property as that of a certain amino acid may also be included as the subject of a conservative amino acid substitution.

In the present specification, "in a non-limiting manner" and "in one aspect" may be used interchangeably.

Non-limiting examples of conservative amino acid substitutions of unnatural amino acids include the following.
MeA: N-methylated amino acid, hydrophobic amino acid group and aliphatic amino acid group
MeF; N-methylated amino acid, hydrophobic amino acid group and aromatic amino acid group
MeW: N-methylated amino acid, hydrophobic amino acid group and aromatic amino acid group
MeY: N-methylated amino acid, hydrophobic amino acid group and aromatic amino acid group
MeR: N-methylated amino acid, hydrophilic amino acid group and basic amino acid group
da: D-type amino acid, hydrophobic amino acid group and aliphatic amino acid group
de: D-type amino acids, hydrophilic amino acid group and acidic amino acid group
dr: D-type amino acid, hydrophilic amino acid group and basic amino acid group
ds: D-type amino acid, hydrophilic amino acid group and neutral amino acid group
4Py: Basic amino acid group and aromatic amino acid group
3Py: Basic amino acid group and aromatic amino acid group
F4C: Hydrophobic amino acid group and aromatic amino acid group
F4OMe: Hydrophobic amino acid group and aromatic amino acid group
F4aao: Hydrophobic amino acid group and aromatic amino acid group
F3COO: Hydrophobic amino acid group and aromatic amino acid group
MeF3C: N-methylated amino acid, hydrophobic amino acid group and aromatic amino acid group
MeF4F: N-methylated amino acid, hydrophobic amino acid group and aromatic amino acid group
Me3Py: N-methylated amino acid, hydrophobic amino acid group and aromatic amino acid group
Me4Py: N-methylated amino acid, hydrophobic amino acid group and aromatic amino acid group
MeF3COO: N-methylated amino acid, hydrophobic amino acid group and aromatic amino acid group
MeF4COO: N-methylated amino acid, hydrophobic amino acid group and aromatic amino acid group
W5C: Hydrophobic amino acid group and aromatic amino acid group
W1aa: Hydrophobic amino acid group and aromatic amino acid group
W7N: Basic amino acid group and aromatic amino acid group
W5C: Hydrophobic amino acid group and aromatic amino acid group
3Py6NH2: Basic amino acid group and aromatic amino acid group
all: Hydrophobic amino acid group and aliphatic amino acid group
Aib: Hydrophobic amino acid group and aliphatic amino acid group
Ahp: Hydrophobic amino acid group and aliphatic amino acid group
A4paa: Hydrophobic amino acid group and aliphatic amino acid group
Kmor: Hydrophilic amino acid group
Kdme: Hydrophilic amino acid group
KCOpipzaa: Hydrophilic amino acid group
QhEt: Hydrophilic amino acid group
D-amino acids may be classified as D-amino acids, but they may also be classified according to the properties of their side chains, and N-methyl amino acids may be classified as N-alkyl amino acids and may also be classified according to the property of the side chain of the original amino acid that has not undergone N-methylation.

In this specification, a "positively charged amino acid" may be any natural or unnatural amino acid, and examples of natural positively charged amino acids are lysine, arginine and histidine. The "positively charged amino acid" may be any amino acid having a positive charge (e.g. NH₃⁺) and may have various substituents.

An Example of preferred peptides in this specification is a peptide that can bind to the human transferrin receptor (hTfR), especially TfR1, as well as the peptides described above.

One aspect of the peptides of the present invention may be peptide that satisfies at least one of the following (I) to (III).
(I) The 1st amino acid of SEQ ID NO: 1 is alanine.
(II) The 6th amino acid of SEQ ID NO: 1 is asparagine.
(III) The 9th amino acid of SEQ ID NO: 1 is isoleucine.

Here, alanine in (I) may be its derivative, asparagine in (II) may be its derivative, and isoleucine in (III) may be its derivative.

Each of the above options (I) through (III) may be selected in any combination, but the peptide may satisfy all of (I) through (III).

According to Example 7, (I) through (III) are considered to be amino acid residues that significantly affect binding to TfR1. Therefore, if the amino acid residues of SEQ ID NO: 1 satisfy at least one, preferably all, of the above (I) to (III), strong binding to TfR1 can be obtained. The affinity of these peptides to the transferrin receptor may be, but is not limited to, less than 50 nM KD, more preferably less than 20 nM KD, and even more preferably less than 10 nM KD.

The peptides of the invention provided herein have valine at 4th position of SEQ ID NO: 1, which is also an important residue for strong binding to TfR1, as supported by the examples.

In the peptide having the amino acid sequence described in SEQ ID NO: 1, the peptide may also have an amino acid sequence in which any one of the amino acid residues at 1st, 3rd, 5th, 6th, 7th, 8th, 9th, 10th, 11th, 12th, 13th and 14th positions of SEQ ID NO: 1 are replaced.

The term "amino acid residue is substituted" means that a specific amino acid residue is replaced by another amino acid residue that may be modified. Herein, substitution of an amino acid residue is preferably a conservative amino acid substitution.

The term "may be modified" means that known amino acid modifications or alterations may be made. Examples of modifications are N-methylation, amino acid modifications with abbreviations as described below, modification (conversion) to D-type, and conversion to known derivatives of the amino acid.

The term "may have a linker" means that a linker may be attached to the amino acid residue. Examples of amino acids with linkers are K(MePEG4c), in which MePEG4c is attached to the amino group of the side chain of a lysine residue; E(PEG1 Me), in which PEG1Me is attached to the carboxy group of the side chain of glutamic acid; F3COO(PEG4Me), in which PEG4Me is attached to the carboxy group of the side chain of F3COO, MeF3COO(PEG4Me), in which PEG4Me is attached to the carboxy group of the side chain of MeF3COO.

In one aspect of the present invention, the peptide may be a peptide having the following amino acid sequence:
(I) substitution of the 3rd N-methylphenylalanine residue of SEQ ID NO: 1 for one amino acid selected from the group consisting of F, MeF3C, MeF4F, Me3Py, Me4Py and MeF3COO;
(II) substitution of the 5th tryptophan residue of SEQ ID NO: 1 for one amino acid selected from the group consisting of W5C, MeW, W1aa and W7N;
(III) substitution of the 6th asparagine residue of SEQ ID NO: 1 for alanine or Ndm;
(IV) substitution of the 7th tyrosine residue of SEQ ID NO: 1 for 3Py6NH2;
(V) substitution of the 8th tyrosine residue of SEQ ID NO: 1 for one amino acid selected from the group consisting of 4Py, F4OMe, F4aao, 3Py, W and F4aao which may have a linker;
(VI) substitution of the 9th isoleucine residue of SEQ ID NO: 1 for alanine;
(VII) substitution of the 10th isoleucine residue in SEQ ID NO: 1 for E;
(VIII) substitution of the 11th K(MePEG4c) residue of SEQ ID NO: 1 for one amino acid selected from the group consisting of Aib, RMeR, K(Mecar), Kmor, K which may have a linker, E(Glucamine), QhEt, Qpipzaa, Q which may have a linker, and E which may have a linker;
(IX) substitution of the 12th arginine residue of SEQ ID NO: 1 with one amino acid selected from the group consisting of da, dr, A4paa, QhEt, Qpipzaa, E(Glucamine), K(Mecar), Kmor, KdMe, KCOpipzaa, Q which may have a linker, E which may have a linker and K to which a linker is attached,
(X) substitution of the 13th phenylalanine of SEQ ID NO: 1 for one amino acid selected from the group consisting of Y, 4Py, F3COO which may have a linker and E which may have a linker; and
(XI) substitution of the 14th N-methyltyrosine residue of SEQ ID NO: 1 for one amino acid selected from the group consisting of Me3Py, MeF4COO which may have a linker and MeF3COO which may have a linker; and
wherein the peptide may be said peptide containing an amino acid sequence with one or more substitutions selected from the above.

Each of the above options (I) through (XI) may be selected in any combination.

A further aspect of the invention is a peptide having the following amino acid sequence. The peptide may be a peptide in which:
(I) the 1st amino acid residue of SEQ ID NO: 1 is MeA or A;
(II) the 3rd amino acid residue of SEQ ID NO: 1 is MeF3C, MeF, F, MeF4F, Me3Py, Me4Py or MeF3COO;
(III) the 5th amino acid residue of SEQ ID NO: 1 is W, W5C, MeW, W1aa or W7N;
(IV) the 6th amino acid residue of SEQ ID NO: 1 is A, N or Ndm;
(V) the 7th amino acid residue of SEQ ID NO: 1 is Y or 3Py6NH2;
(VI) the 8th amino acid residue of SEQ ID NO: 1 is 4Py, F4OMe, Y, F4aao, 3Py, W or F4aao (PEG8Me);
(VII) the 9th amino acid residue of SEQ ID NO: 1 is A or I;
(VIII) the 10th amino acid residue of SEQ ID NO: 1 is E or I;
(IX) the 11th amino acid residue of SEQ ID NO: 1 is Aib, K, K(Mecar), K(MePEG4c), Kmor, R, MeR, Q, QhEt, Qpipzaa, E(PEG1 Me), E(PEG4Me), E(PEG8Me), E(Glucamine) or E(NHdPEG1 Me);
(X) the 12th amino acid residue of SEQ ID NO: 1 is da, R, dr, Q, QhEt, Qpipzaa, E(PEG8Me), E(PEG1 Me), E(Glucamine), K, K(Mecar), Kmor, KdMe, K(MePEG4c), KCOpipzaa or A4paa;
(XI) the 13th amino acid residue of SEQ ID NO: 1 is F, F3COO, F3COO(PEG4Me), 4Py, E(PEG4Me) or Y; and
(XII) the 14th amino acid residue of SEQ ID NO: 1 is MeY, Me3Py, MeF4COO(PEG4Me) or MeF3COO(PEG4Me).

However, it is preferable that the 11th and 12th amino acid residues in SEQ ID NO: 1 should not both be E(PEG8Me) or both should not be Qpipzaa.

Each of the above options (I) through (XII) may be selected in any combination.

Preferred examples of peptides of the invention are peptides containing the 1-15th amino acid sequence of the amino acid sequence described in any one of the SEQ ID NOs: 1 to 241 or conjugate of the amino acid sequence described in any one of the sequences 1 to 241 with a linker, wherein the amino acid sequence moiety has a cyclic structure.

A preferred example of this peptide is one of the above-mentioned peptides, which is a cyclic peptide.

A preferred example of this peptide is a peptide having a cyclic structure at the above 1-15th amino acid sequence moiety of any one of the above-mentioned peptides.

A preferred example of this peptide is any one of the above peptides, which consists of an amino acid sequence in which the N-terminal, namely 1st, amino acid is chloroacetylated and the acetyl group and the C-terminal amino acid, cysteine, are cyclized.

### About Cyclic Peptide

"Cyclic peptide" refers to a peptide in which two amino acids are bound and the entirety or a part thereof are cyclic. In the present invention, this peptide also includes an amino acid in the peptide forming a cross-linked structure; forming a cyclic structure by lactam ring formation or a macrocyclization reaction; having a lasso peptide-like structure; and the like. That is, a part of the cyclic peptide may form a cyclic structure or it may have a straight-chain part.

In some instances, some peptides exhibit poor metabolic stability *in vivo,* and some peptides are large in size, making it difficult for them to penetrate cell membranes. A method for cyclizing a peptide has been adopted in light of such problems. It has been suggested that when a peptide is cyclized, protease resistance is improved, metabolic stability is improved, and restrictions are also added to conformational change, so that rigidity is increased and membrane permeability and affinity for the target protein is improved.

### Cyclization Method

Cyclization of the peptide may be carried out according to a known method.

In a non-limiting manner, by designing the peptide to comprise two or more cysteine residues, for example, a cyclic structure may be formed by a disulfide bond after translation. Furthermore, according to the method of Goto et al. (Y. Goto, et al. ACS Chem. Biol. 3 120-129 (2008)), a peptide having a chloroacetyl group at its N-terminal may be synthesized by genetic code reprogramming technology and may also be circularized by disposing a cysteine residue containing a sulfur molecule in the peptide. Thus, a mercapto group spontaneously performs a nucleophilic attack on the chloroacetyl group after translation, and the peptide is circularized by thioether binding. Other amino acid combinations that bind to form a ring may be disposed within the peptide and circularized by genetic code reprogramming technology. The peptide can also be cyclized by synthesizing a peptide with a cycloamide at the N-terminal and placing Hgl residue in the peptide. In this manner, a known circularization method may be used without any particular limitation.

Said peptide has a cyclic structure in which the N-terminal amino acid (the 1st amino acid residue) and a cysteine residue in said peptide are bonded. In an aspect, said peptide has a cyclic structure in which the N-terminal amino acid (the 1st amino acid residue) is bonded to the 15th cysteine residue in said peptide. In one aspect, said peptide has a cyclic structure in which the chloroacetylated N-terminal amino acid (the 1st amino acid residue) is bonded to the 15th cysteine residue in said peptide. "Chloroacetylation" can also be "halogen acetylation" with other halogens. "Acetylation" can also be "acylation" with an acyl group other than an acetyl group.

In this specification, some amino acids may be modified for cyclization of peptides. Amino acids with such partial modifications are also included. For example, as mentioned above, a chloroacetyl group may be added to the amino acid located at the N-terminal, which is combined with a cysteine residue in the peptide for cyclization, and various (natural/unnatural) amino acids to which such a chloroacetyl group is added are also included in the amino acids in this application.

A preferred example of this peptide is any one of the above peptides, consisting of 15 amino acid residues.

A preferred example of this peptide is any one of the above-mentioned peptides, wherein a linker is attached to the 8th, 11th, 12th, 13th, 14th or 15th amino acid residue of the amino acid sequence. Even more preferably, said peptides are those described in any one of SEQ ID NOs: 10-241.

### Peptide Length

The number of amide bonds (the number and length of amino acids) in the peptide or peptide moiety is not particularly limited, but the total amino acid residues (if the substance bound to the peptide or the linker that binds said substance and peptide contains amino acids, those amino acids are not included) should not exceed 20 residues. Preferably, the number of amino acids is 10 or more, 11 or more, 12 or more, 13 or more, 14 or more, and preferably the number of amino acids is 19 or less, 18 or less, 17 or less, 16 or less.

Another embodiment disclosed in this specification relates to a complex (conjugate). This complex is a complex containing any one of the peptides described above, a linker bound to the peptide, and a substance bound to the linker. It is preferred that the complex is capable of binding at least to TfR.

The above complex may be a complex made by bonding the C-terminal of a cysteine (Cys) possessed by any one of the above peptides to said substance, or a complex made by bonding a linker attached to said C-terminal to said substance. Further the complex may be made by bonding a functional group possessed by a side chain end contained in an amino acid residue of any one of the above peptides to said substance, or a complex made by bonding a linker attached to said functional group to said substance.

An example of the linker is that its amino acid length is between 1 and 15 and contains one or more glycine (Gly) or serine (Ser).

Preferred example of this linker is cysteine (Cys), which may be modified at the N-terminal, or lysine (Lys), which may be modified.

Another example of a linker is one that has an amino acid length between 1 and 5 and contains either or both D-body glutamic acid (de) and methylated glycine (MeG).

Preferred example of this linker is cysteine (Cys), which may be modified at the N-terminal, or lysine (Lys), which may be modified.

Another example of a linker is a PEG linker containing polyethylene glycol (PEG) or a derivative of polyethylene glycol. Derivatives of polyethylene glycol include all those known as PEG linkers. Some may be partially substituted for a functional group or functionalized by a functional group. Examples include, but are not limited to, methyl groups, amino groups, azide groups, etc.

Preferably, the PEG linker is PEG4c, PEG12c, PEG8Me, MePEG4c, PEG1Me, PEG4Me or NHdPEG1 Me.

In addition, the linker may also contain reactive functional groups to bind the desired substance. Examples of reactive functional groups are maleimides and hydrazides.

Another example of a linker is a linker having the sequence indicated by any one of SEQ ID NOs: 242 to 253. The linker may also be the sequence of the Linker portion of SEQ ID NOs: 263 to 394 represented in TABLE 5. For example, in SEQ ID NO. 263, the [G-G-G-G-S-S-KN3] sequence starting from the 16th amino acid residue, glycine, is the linker.

Preferred examples of the peptide or complex to which this linker is attached are those in which the linker is:
a G linker which is a peptide linker consisting of polyethylene glycol (PEG), Gly or MeG; a GS linker which is a peptide linker consisting of Gly or MeG and Ser;
or a linker having the amino acid sequence shown in any one of SEQ ID NOs: 242 to 253. The linker may be that of a linker having the amino acid sequence shown in any one of SEQ ID NOs: 263 to 394.

Linker (also called crosslinker) herein refers to a structure that is additionally attached to an amino acid residue in the cyclic amino acid sequence of a peptide that binds to the transferrin receptor, or an intermolecular linkage between said peptide and the substance to be delivered to the TfR, and also it may be any linker known or described herein. In certain embodiments, said linker is, for example, a chemical linker, a fatty acid linker, a peptide linker (polypeptide linker). It may also be a complex of, for example, a chemical linker and a peptide linker. For example, it may be a linker structure having both PEG and amino acid residues or peptide moieties, as shown in SEQ ID NO. 245, etc.

Linkers may, for example, be such that they diverge or separate depending on the environment and condition, or they may maintain a stable structure.

Chemical linker: In some embodiments, the linker may be a chemical linker. Chemical linkers include, but are not limited to, substituted or unsubstituted alkylene, substituted or unsubstituted heteroalkylene, substituted or unsubstituted cycloalkylene, substituted or unsubstituted heterocycloalkylene, substituted or unsubstituted arylene and/or substituted or unsubstituted heteroarylene. The peptide and linker can also be conjugated via sulfhydryl group, amino group (amine), and/or carbohydrate or any suitable reaction group. Homobifunctional and heterobifunctional crosslinkers (conjugating agents) are available from many commercial sources. Crosslinkers may contain flexible arms, e.g., 2, 3, 4, 5, 6, 7, 8, 9, 10, 11, 12, 13, 14 or 15 carbon atoms. Examples of crosslinkers include BSS3 ([bis(sulfosuccinimidyl)suberate], NHSS/EDC (N-hydroxysuccinimide and N-ethyl-(dimethylaminopropyl)carbodiimide), sulfo-EMCSS ([N-e-maleimidocaproic acid] hydrazide, hydrazide, and SSATA (N-succinimidyl-SS-acetylthioacetic acid), and others.

Preferred examples of the chemical linker include a PEG (polyethylene glycol) linker. For example, the PEG linker may consist of 1 to 24 ethylene glycol units.

Fatty acid linker: The linker may be a fatty acid linker containing a divalent chemical moiety derived from a fatty acid. For example, the fatty acid linker may be a linker with 12-aminododecanoic acid.

Peptide linker: A peptide linker contains at least one amino acid (e.g., a peptide of at least 2, 3, 4, 5, 6, 7, 10, 15, 20, 25, 40 or 50 amino acids). In a certain embodiment, the linker is one amino acid (e.g., any natural amino acid such as Cys). In another embodiment, a glycine-rich peptide such as a peptide having a sequence [Gly-Gly-Gly-Gly-Ser]n (in the formula, n is 1, 2, 3, 4, 5, or 6) such as that according to US Patent No. 7,271,149. In another embodiment, a serine-rich peptide linker according to US Patent No. 5,525,491 may be used. As the serin-rich peptide linker, the linker which has the formula [X-X-X-X-Gly]y (wherein up to two of the Xs are Thr, the remaining Xs are Ser, and y is from 1 to 5) (e.g. Ser-Ser-Ser-Ser-Gly (where y is 2 or more)) can be mentioned. In some cases, the linker is a single amino acid (e.g., any amino acid such as Gly or Ala).

Another embodiment disclosed in this specification includes the peptide to which the linker described above is attached or the complex described above, and the substance described above is the active ingredient.

The substance may be any substance desired by a person having ordinary skill in the art as long as it is a substance the skilled person desires to be delivered to the TfR. Examples of the substance are not limited, but include the following:
Compound: Not only low molecular weight compounds and middle molecular weight compounds, but also any compounds that can be introduced by the cytolytic mechanism of the cell. Examples include known low molecular weight drugs.
Peptide: "Peptide" may be a peptide that binds to a target in the body and exhibits some kind of effect, for example, a cyclic peptide.
Rl: "RI" may be any compound that can be labelled by a radioisotope, such as a low molecular weight or middle molecular weight compound or an antibody labelled by a radioisotope. Examples include compounds for PET scanning.
Protein: "Protein" may be any protein that exhibits a useful function in the body, such as an antibody or an enzyme. Examples include enzymes used in enzyme replacement therapy.
Nucleic acid: Any substance having a base sequence, such as DNA and RNA. Examples include nucleic acid medicines.
DDS: "DDS" may be a known DDS molecule, such as a liposome or a micelle. The DDS molecule may further comprise a compound therein such as a pharmaceutical.
Moreover, the above substances may be a complex in which several of the examples given above are combined.

Another embodiment disclosed in this specification relates to a method for producing a pharmaceutical or diagnostic composition. This method is a method for producing a prophylactic, therapeutic or diagnostic agent, including the process of obtaining the above-described complex.

A preferred example of this method is the linker which has a polyethylene glycol (PEG), G linker or GS linker, or is the linker which has the amino acid sequence shown in any one of SEQ ID NOs: 242-253. Alternatively, the linker may be the sequence of the Linker description portion of SEQ ID NOs: 263 to 394, represented in TABLE 5.

Another embodiment disclosed in this specification relates to compositions for medicinal or diagnostic use containing a complex containing a substance further bound to the peptide or peptide and linker conjugate described above. Preferably, the composition is for medicinal or diagnostic use in TfR-related diseases.

The peptide of the present invention may be produced by, for example, any known method for producing a peptide, such as a chemical synthesis method such as a liquid phase method, a solid phase method, a hybrid method combining a liquid phase method and a solid phase method or the like, and a genetic recombination method; or the like.

In the solid phase method, for example, a hydroxy group of a resin having a hydroxy group and a carboxy group of a 1st amino acid (normally a C-terminal amino acid of a target peptide) in which an α-amino group is protected by a protecting group are subjected to an esterification reaction. For the esterification catalyst, a known dehydrating and condensing agent such as 1-mesitylenesulfonyl-3-nitro-1, 2, 4-triazole (MSNT), dicyclohexylcarbodiimide (DCC), and diisopropylcarbodiimide (DIPCDI) may be used.

Next, the protecting group of the α-amino group of the 1st amino acid is removed, a second amino acid in which all functional groups except the carboxy group of the main chain are protected is added, and the carboxy group is activated, binding the 1st and 2nd amino acids. Furthermore, the α-amino group of the 2nd amino acid is deprotected, a 3rd amino acid in which all functional groups except the carboxy group of the main chain are protected is added, the carboxy group is activated, binding the 2nd and 3rd amino acids. This is repeated, and after a peptide having a target length is synthesized, all of the functional groups are deprotected.

Examples of the resin for solid-phase synthesis include Merrifield resin, MBHA resin, C!-Trt resin, SASRIN resin, Wang resin, Rink amide resin, HMFS resin, Amino-PEGA resin (Merck KGaA), HMPA-PEGA resin (Merck KGaA), and the like. These resins may be used after being washed using a solvent (dimethylformamide (DMF), 2-propanol, methylene chloride, and the like).

Examples of the protecting group of the α-amino group include the benzyloxycarbonyl (Cbz or Z) group, *tert*-butoxycarbonyl (Boc) group, fluorenylmethoxycarbonyl (Fmoc) group, benzyl group, allyl group, allyloxycarbonyl (Alloc) group, and the like. The Cbz group may be deprotected by a treatment using hydrofluoric acid, hydrogenation, or the like, the Boc group may be deprotected by a treatment using trifluoroacetic acid (TFA), and the Fmoc group may be deprotected by a treatment using piperidine.

Examples such as methyl ester, ethyl ester, benzyl ester, *tert*-butyl ester, cyclohexyl ester, and the like may be used to protect the α-carboxy group.

As other functional groups of amino acids, the hydroxy groups of serine and threonine can be protected by benzyl or *tert*-butyl groups, and the hydroxy group of tyrosine can be protected by 2-bromobenzyloxycarbonyl group or *tert*-butyl group. The amino group of the lysine side chain and the carboxy group of glutamic acid and aspartic acid can be protected as well as the α-amino and α-carboxy groups.

Activation of the carboxy group may be performed using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazole-1-yloxy)tris(dimethylamino)phosphonium hexafluorophosphate (BOP), 1-[bis(dimethylamino)methyl]-1H-benzotriazolium-3-oxide hexafluorophosphate (HBTU), and the like.

Cleavage of the peptide chain from the resin may be performed by treating the peptide chain using an acid such as TFA, hydrogen fluoride (HF), or the like.

Production of a peptide by a gene recombination method (translation/synthesis system) may be performed using a nucleic acid encoding the peptide. The nucleic acid encoding the peptide may be DNA or RNA.

The nucleic acid encoding the peptide of the present invention may be prepared by a known method or a method equivalent thereto. For example, the nucleic acid may be synthesized by an automated synthesizer. A restriction enzyme recognition site may be added to insert the obtained DNA into a vector, or a base sequence encoding an amino acid sequence for splicing a formed peptide chain using an enzyme or the like may be incorporated.

As described above, when the peptide of the present invention is fused to a cell-penetrating peptide or the like, the nucleic acid also includes a nucleic acid encoding the cell-penetrating peptide.

A chimeric protein expression method for expressing the target peptide as a chimeric peptide of another peptide may also be used to suppress degradation by a host-derived protease. In this case, a nucleic acid encoding the target peptide and the peptide bound thereto may be used as the nucleic acid.

Subsequently, an expression vector is prepared using the nucleic acid encoding the peptide of the present invention. The nucleic acid may be inserted downstream of a promoter of the expression vector, either as is or after digestion with restriction enzymes or the addition of a linker. Examples of the vector include an *Escherichia coli*-derived plasmids (pBR322, pBR325, pUC12, pUC13, pUC18, pUC19, pUC118, pBluescript II, and the like), a *Bacillus subtilis-derived* plasmids (pUB110, pTP5, pC1912, pTP4, pE194, pC194, and the like), a yeast-derived plasmids (pSH19, pSH15, YEp, YRp, Yip, YAC, and the like), bacteriophages (e phage, M13 phage, and the like), viruses (retrovirus, vaccinia virus, adenovirus, adeno-associated virus (AAV), cauliflower mosaic virus, tobacco mosaic virus, baculovirus, and the like), cosmids, and the like.

The promoter may be selected appropriately according to the type of host. When the host is an animal cell, for example, a promoter derived from SV40 (simian virus 40) or a promoter derived from CMV (cytomegalovirus) may be used. When the host is *Escherichia coli,* a trp promoter, a T7 promoter, a lac promoter, or the like may be used.

The expression vector may incorporate, for example, a DNA replication starting point (ori), a selective marker (antibiotic resistance, auxotrophy, or the like), an enhancer, a splicing signal, a poly-A addition signal, a nucleic acid encoding a tag (FLAG, HA, GST, GFP, or the like), or the like.

Next, an appropriate host cell is transformed by the expression vector. The host may be appropriately selected in relation to the vector. Examples such as *Escherichia coli, Bacillus subtilis* (*Bacillus*)*,* yeast, insects or insect cells, animal cells, or the like may be used as the host. As the animal cells, for example, HEK293T cells, CHO cells, COS cells, myeloma cells, HeLa cells, and Vero cells may be used. Transformation may be carried out according to a known method, such as a lipofection method, a calcium phosphate method, an electroporation method, a microinjection method, a gene gun method, or the like depending on the type of host. The target peptide is expressed by culturing a transformant according to a conventional method.

As for purification of the peptide from the transformant culture, cultured cells are recovered and then suspended in an appropriate buffer solution, followed by disruption of cells by a method such as sonication, freeze-thawing, or the like, and then a crude extract is obtained by centrifugation or filtration. When the peptide is secreted into the culture solution, a supernatant is recovered.

Purification of the crude extract or the culture supernatant may also be performed by a known method or a method equivalent thereto (for example, salting-out, dialysis, an ultrafiltration method, gel filtration method, SDS-PAGE method, ion exchange chromatography, affinity chromatography, reversed-phase high-performance liquid chromatography, and the like).

The obtained peptide may be converted from a free body to a salt or from a salt to a free body by a known method or a method equivalent thereto.

The translation/synthesis system may be a cell-free translation system. The cell-free translation system includes, for example, a ribosome protein, an aminoacyl-tRNA synthase (ARS), a ribosome RNA, an amino acid, rRNA, GTP, ATP, a translation initiation factor (IF), an elongation factor (EF), a release factor (RF), and a ribosome regeneration factor (RRF), or another factor required for translation. An *Escherichia coli* extract or a wheat embryo extract may be added to increase expression efficiency. In addition, a rabbit red blood cell extract or an insect cell extract may be added.

By continuously supplying energy to a system including these using dialysis, a protein of several hundred µg to several mg/mL may be produced. The system may include an RNA polymerase to concurrently perform transcription of genomic DNA. Examples of commercially available cell-free translation systems that may be used include RTS-100 (registered trademark) by Roche Diagnostics K.K., PURE System by GeneFrontier Corporation, PURExpress In Vitro Protein Synthesis Kit by New England Biolabs Inc., and the like for a system derived from *Escherichia coli,* and a system by ZOIGENE, CellFree Sciences Co., Ltd., or the like for a system using wheat embryo extract.

According to the cell-free translation systems, the expression product can be obtained in a highly pure form without purification.

In the cell-free translation system, artificial aminoacyl-tRNA in which a desired amino acid or hydroxy acid may be linked (acylated) to a tRNA may be used in place of an aminoacyl-tRNA synthesized by a natural aminoacyl-tRNA synthetase. The aminoacyl-tRNA may be synthesized using an artificial ribozyme.

An example of the ribozyme includes a flexizyme (H. Murakami, H. Saito, and H. Suga, (2003), Chemistry & Biology, Vol. 10, 655-662; H. Murakami, D. Kourouklis, and H. Suga, (2003), Chemistry & Biology, Vol. 10, 1077-1084; H. Murakami, A. Ohta, H. Ashigai, and H. Suga (2006) Nature Methods 3, 357-359 "The flexizyme system: a highly flexible tRNA aminoacylation tool for the synthesis of nonnatural peptides"; N. Niwa, Y. Yamagishi, H. Murakami, H. Suga (2009) Bioorganic & Medicinal Chemistry Letters 19, 3892-3894 "A flexizyme that selectively charges amino acids activated by a water-friendly leaving group"; and WO 2007/066627 and the like). Flexizymes are also known under the names of prototype flexizyme (Fx), and dinitrobenzyl flexizyme (dFx), enhanced flexizyme (eFx), aminoflexizyme (aFx) and the like which are modified from it.

A desired codon may be translated in association with the desired amino acid or hydroxy acid by using the tRNA produced by flexizyme and to which the desired amino acid or hydroxy acid is linked. A specialty amino acid may be used as the desired amino acid. For example, an unnatural amino acid required for the above circularization may also be introduced into the binding peptide by this method.

Various methods commonly used in the technical field may be used for chemical synthesis of the macrocyclic peptides of the present invention and their analogs, including, for example, stepwise solid-phase synthesis, semisynthesis of peptide fragments undergoing conformationally supported religation, and chemical ligation. Synthesis of the peptides and their analogs described herein is chemical synthesis using various solid phase technologies described in, for example, K. J. Jensen, P. T. Shelton, S. L. Pedersen, Peptide Synthesis and Applications, 2nd Edition, Springer, 2013, and the like. A preferable strategy is based on a combination of an Fmoc group capable of temporarily protecting the α-amino group and being selectively removed using a base, and a protecting group that temporarily protects a side chain functional group and is stable under Fmoc deprotection conditions. Selection of this kind of general peptide side chain is known according to the aforementioned Peptide Synthesis and Applications, 2nd Edition; G. B. Fields, R. L. Noble, Solid Phase Peptide Synthesis Utilizing 9-Fluorenylmethoxycarbonyl Amino Acids, Int. J. Peptide Protein Res. 35, 1990, 161-214, and the like; however, preferable peptide side chain protecting groups include, for example, a Boc group and Mtt group for the amino group of the lysine side chain; a *tert-*butyl group for carboxyl groups, such as glutamic acid and aspartic acid; or a Trt group and a Mmt group for the thiol group of cysteine.

The peptides and their analogs described herein may be synthesized by a stepwise method on the solid-phase resin described above. The C-terminal amino acid used and all amino acids and peptides used in the synthesis must have the α-amino protecting group selectively removed in the synthetic process. Preferably, the solid-phase resin described above is used, and once a C-terminal carboxyl group of a peptide having its N-terminal properly protected by Fmoc or the like or a C-terminal carboxyl group of an amino acid having its N-terminal protected by Fmoc is made into an activated ester by an appropriate reagent, this is then added to the amino group on the solid-phase resin to start. Subsequent elongation of the peptide chain may be achieved by removing the N-terminal protecting group (Fmoc group) then successively repeating condensation of the protected amino acid derivative according to the amino acid sequence of the target peptide. Note that these may release the target peptide in a final stage. Examples of releasing conditions are given in Teixeira, W. E. Benckhuijsen, P. E. de Koning, A. R. P. M. Valentijn, J. W. Drijfhout, Protein Pept. Lett., 2002, 9, 379-385, and the like, and the peptide may be released in a TFA solution containing water/silyl hydride/thiol as a scavenger in TFA. Typical examples include TFA/Water/TIS/DODT (volume ratio 92.5:2.5:2.5:2.5).

Synthesis of the peptide analogs described in the present specification may be carried out using a single or multi-channel peptide synthesizer, for example, a Liberty Blue synthesizer from CEM Corporation, a Syro I synthesizer or a successor machine thereof from Biotage Japan, Ltd., or the like.

Activation of the carboxy group may be performed using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazole-1-yloxy)tris(dimethylamino)phosphonium hexafluorophosphate (BOP), 1-[bis(dimethylamino)methyl]-1H-benzotriazolium-3-oxide hexafluorophosphate (HBTU), and the like.

Another embodiment disclosed herein relates to pharmaceutical composition. This pharmaceutical composition contains the above-mentioned peptides, pharmaceutically acceptable salts or solvates thereof (for simplicity, they are hereinafter also referred to simply as peptides). The pharmaceutical composition preferably contains an effective amount of the above peptide as an active ingredient.

In the present specification, the form of administration of the pharmaceutical composition is not particularly limited and may be oral or parenteral. Examples of parenteral administration include injection, such as intramuscular injection, intravenous injection, or subcutaneous injection; transdermal administration; transmucosal administration (transnasal, transoral, transocular, transpulmonary, transvaginal, or transrectal); or the like.

The pharmaceutical composition may be modified in various ways, considering a property where a polypeptide is easily metabolized and excreted. For example, polyethylene glycol (PEG) or a sugar chain may be added to the polypeptide to extend its retention time in the blood to reduce antigenicity. Furthermore, bio-degradable polymeric compounds such as polylactic acid and glycol (PLGA), porous hydroxyapatite, liposomes, surface-modified liposomes, emulsions prepared with unsaturated fatty acids, nanoparticles, or nanospheres may be used as sustained release base, and the polypeptide can be encapsulated in them. In the case of transdermal administration, a weak current is allowed to pass through the skin surface and penetrate the stratum corneum (iontophoresis).

The above pharmaceutical compositions may be prepared by using the active ingredients as they are or formulated by adding pharmaceutically acceptable carriers, excipients, additives, or the like. Examples of the dosage form include a liquid agent (for example, an injection), a dispersant, a suspension, a tablet, a pill, a powder, a suppository, a powdered drug, a fine granule, a granule, a capsule, a syrup, a lozenge, an inhalant, an ointment, an eye drop, a nasal drop, an ear drop, a patch, or the like.

The formulation may be carried out by a common method using, for example, an excipient, a binder, a disintegrant, a lubricant, a dissolving agent, a solubilizing agent, a colorant, a flavoring agent, a stabilizer, an emulsifier, an absorption promoter, a surfactant, a pH regulator, a preservative, an antioxidant, or the like as appropriate.

Examples of ingredients used for formulation include but are not limited to purified water, saline, phosphate buffer solution, dextrose, glycerol, a pharmaceutically acceptable organic solvent such as ethanol, animal and vegetable oil, lactose, mannitol, glucose, sorbitol, crystalline cellulose, hydroxypropyl cellulose, starch, corn starch, anhydrous silicic acid, magnesium aluminum silicate, collagen, polyvinyl alcohol, polyvinyl pyrrolidone, carboxyvinyl polymer, sodium carboxymethylcellulose, sodium polyacrylate, sodium alginate, watersoluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, human serum albumin, or the like.

The pharmaceutical composition may comprise an absorption promoter for improving absorption of a poorly absorbable drug, in consideration of the fact that it is generally difficult for peptides to be absorbed through mucous membranes. The following may be used as the absorption promoter: a surfactant such as polyoxyethylene lauryl ether, sodium lauryl sulphate, and saponin; a bile salt such as glycocholic acid, deoxycholic acid, and taurocholic acid; a chelating agent such as EDTA and salicylic acid; a fatty acid such as caproic acid, capric acid, lauric acid, oleic acid, linoleic acid, a mixed micelle; an enamine derivative, an N-acyl collagen peptide, an N-acyl amino acid, a cyclodextrin, chitosan, a nitric oxide donor, or the like.

When the pharmaceutical composition is a pill or tablet, it may be coated using a sugar coating, or a gastric-soluble or enteric-coated substance.

When the pharmaceutical composition is an injection, it may comprise distilled water for injection, physiological saline, propylene glycol, polyethylene glycol, vegetable oil, alcohol, or the like. Additionally, a humectant, an emulsifier, a dispersant, a stabilizer, a dissolving agent, a solubilizing agent, a preservative, or the like may be added.

The dosage in the case of administering to a mammal (e.g., human, mouse, rat, guinea pig, rabbit, dog, horse, monkey, pig and the like), especially human, changes depending on symptoms, age of the patient, sex, weight, sensitivity difference, administration method, administration interval, type of active ingredient, and type of formulation, and it may be administered in a non-limiting manner: for example, by administering between 30 µg to 100 g, between 100 µg to 500 mg, or between 100 µg to 100 mg once or divided into several doses. In the case of injection, between 1 µg/kg and 3,000 µg/kg, or between 3 µg/kg and 1,000 µg/kg may be administered once or divided into several doses, according to the bodyweight of the patient.

The term "TfR-related diseases" refers to diseases in which TfR is a therapeutic target protein and diseases caused in cells or tissues expressing TfR. In addition, since binding to TfR allows it to cross the blood-brain barrier, the term "TfR-related diseases" includes brain diseases (diseases caused by some abnormality in the brain, such as central nervous system (CNS) diseases) and neuromuscular diseases (diseases in which muscle weakness is caused by lesions of nerves or muscles, such as the brain, spinal cord, and peripheral nerves), because TfR is expressed in large numbers in muscle tissue.

### Detection Reagent for TfR-related Diseases and Test Kit

The present invention also encompasses TfR-related disease detection agents containing peptides of the present invention. When used as a detection agent, the peptides of the invention may be detectably labelled. As the labels of peptides, for example; enzymes such as peroxidase and alkaline phosphatase; radioactive materials such as 125I, 131I, 35S, 3H; fluorescent substances such as fluorescein isothiocyanate, rhodamine, dansyl chloride, phycoerythrin, tetramethylrhodamine isothiocyanate, and near-infrared fluorescent materials; and antibodies labelled with luminescent substances such as luciferase, luciferin, and equoline are used. In addition, antibodies labelled with nanoparticles such as gold colloids and quantum dots can be used for detection. For example, TfR-related diseases can be detected by creating a complex with an antibody that binds to a specific target related to TfR-related diseases and a peptide of the invention, creating a complex with said labelled antibody or labelled peptide of the present invention, and then administering and detecting it.

In immunoassays, the peptides of the present invention can also be detected by labelling them with biotin and binding avidin or streptavidin labelled with enzymes and the like.

Among immunoassays, ELISA using enzyme-labelling is preferred method because it can measure antigens simply and rapidly. For example, an antibody is immobilized on a solid-phase carrier, a sample is added and allowed to react, then the labelled peptide of the present invention is added and allowed to react. After washing, the antibody is reacted with the enzyme substrate, and the absorbance is measured to detect TfR-related diseases. After the reaction of the sample with the antibody immobilized on the solid-phase carrier, the unlabelled peptide of the present invention is added, and the enzymatically labelled antibody to the peptide of the present invention may be further added.

Enzyme substrates such as 3,3'-diaminobenzidine (DAB), 3,3',5,5'-tetramethylbenzidine (TMB), o-phenylenediamine (OPD) can be used if the enzyme is a peroxidase, and tetramethylbenzidine (TMB), o-phenylenediamine (OPD), etc. can be used if the enzyme is a peroxidase, and p-nitrophenyl phosphate (NPP) and the like can be used if the enzyme is an alkaline phosphatase.

In this specification, "solid-phase carrier" is not limited to any carrier only if it can immobilize antibodies, and includes microtiter plates, substrates, beads, nitrocellulose membranes, nylon membranes, PVDF membranes and the like made of glass, metal, resin and the like, and the target substances can be immobilized on these solid-phase carriers according to known methods.

The test kit of the present invention includes reagents and instruments (including but not limited to peptides of the invention, antibodies, solid phase carriers, buffer solutions, enzyme reaction stopping solutions, microplate readers, etc.) necessary for the above detection.

Another embodiment disclosed in this specification may also be considered for use as a test kit containing the above-mentioned TfR-related disease detection agent or as a tool for elucidating TfR-related diseases and various cellular functions and life phenomena associated with them.

This specification also provides the use of the peptides for producing pharmaceutical or diagnostic compositions. In this case, the peptide can be any one of the above.

This specification also provides methods of preventing or treating TfR-related diseases, comprising the step of administering an effective amount of a peptide, a pharmaceutically acceptable salt thereof, or a solvate or complex thereof to a subject who is a human, non-human mammal, or bird. The peptides can be any one of the peptides described above. Examples of the non-human mammal are non-human primates, pigs, cattle, dogs, cats, horses, sheep, rats and mice.

This specification also provides methods of preventing or treating TfR-related diseases, comprising the step of administering an effective amount of a peptide, a pharmaceutically acceptable salt thereof, or a solvate or complex thereof to a subject who is a human, non-human mammal, or bird.

This specification provides a test method for peptide wherein the peptide is tested at least one of:
a) solubility in a solvent;
b) binding ability to hTfR;
c) toxicity to a cell and/or a tissue; and
d) toxicity to experimental animal,
wherein the peptide is a peptide having an amino acid sequence in which one to three amino acid residues are deleted, substituted, inserted and/or added in the amino acid sequence possessed by the peptide of the present invention.

The peptides of the above invention may be any one of the peptides described herein, but one preferred aspect of the peptides is the peptide satisfies, in the amino acid sequence of SEQ ID NO: 1, at least one of or all of the following:
(I) the 1st amino acid of SEQ ID NO:1 is alanine;
(II) the 6th amino acid of SEQ ID NO: 1 is asparagine; or
(III) the 9th amino acid of SEQ ID NO: 1 is isoleucine.

In the above, alanine of (I) may be a derivative thereof, asparagine of (II) may be a derivative thereof, and isoleucine of (III) may be a derivative thereof.

In one aspect of the peptide of the present invention comprises or consists of, but not limited to, an amino acid sequence described in any one of SEQ ID NOs: 1 to 241. Said peptide is, in an aspect, a cyclic peptide containing or consisting of an amino acid sequence described in any one of SEQ ID NOs: 1 to 241. One skilled in the art may select, but not limited to, one to three amino acids in the amino acid sequence described in any one of SEQ ID NOs: 1 to 241 as appropriate and create a sequence with deletion, substitution, insertion and/or addition. In the above method, the number of amino acids to be deleted, substituted, inserted and/or added is from 1 to 3, preferably from 1 to 2, more preferably 1.

Deletion, substitution, insertion and/or addition of amino acid residues is not limited, but substitution is preferred, and conservative amino acid substitution is more preferred.

The peptide may contain or consist of an amino acid sequence having at least 80% or more, preferably 85% or more, 86% or more, 87% or more, 88% or more, 90% or more, 91% or more, 92% or more, 93% or more, 94% or more amino acid identity to the amino acid sequence of the peptide of the present invention.

The percent identity of two amino acid sequences may be determined by visual inspection and mathematical calculation. Alternatively, the percent identity of two protein sequences may be determined by comparing sequence information based on the algorithm of Needleman, S. B. and Wunsch, C. D. (J. Mol. Biol., 48: 443-453, 1970) and using the GAP computer program available from the University of Wisconsin Genetics Computer Group (UWGCG). Preferred default parameters of the GAP program include: (1) the scoring matrix, blosum 62, as described in Henikoff, S. and Henikoff, J. G. (Proc. Natl. Acad. Sci. USA, 89: 10915-10919, 1992); (2) 12 gap weights; (3) 4 gap length weights; and (4) no penalty for the terminal gap.

Other programs for sequence comparison, used by those skilled in the art, may also be used. Percent identity can be determined by comparison with sequence information using, for example, the BLAST program described in Altschul et al. (Nucl. Acids. Res., 25, p. 3389-3402, 1997). The program is available on the Internet at the websites of the National Center for Biotechnology Information (NCBI) or the DNA Data Bank of Japan (DDBJ). Various conditions (parameters) for identity searches by using the BLAST program are described in detail on the above-mentioned website, and although some settings can be changed as necessary, searches are usually performed using default values. Alternatively, the % identity of two amino acid sequences may be determined using a program such as the genetic information processing software GENETYX Ver. 7 (GENETYX CORPORATION), or the FASTA algorithm. In such cases, default values may be used for the search.

Based on the amino acid sequence prepared in this way, a person skilled in the art can prepare peptides to be used in the test method of the present invention by known peptide production methods, such as chemical synthesis methods, e.g. the above-mentioned liquid phase method, solid phase method, and hybrid method combining liquid phase method and solid phase method, and genetic recombination method and the like.

The peptide having amino acid sequence with deletion, substitution, insertion and/or addition of one to three amino acid residues (i.e., the peptide to be tested as described in the aforementioned test methods) is, but not limited to, the peptide satisfies, in the amino acid sequence of SEQ ID NO: 1, at least one of or all of the following:
(I) the 1st amino acid of SEQ ID NO:1 is alanine;
(II) the 6th amino acid of SEQ ID NO: 1 is asparagine; or
(III) the 9th amino acid of SEQ ID NO: 1 is isoleucine.

Such peptides are considered to have high binding ability to hTfR.

In the above, alanine of (I) may be a derivative thereof, asparagine of (II) may be a derivative thereof, and isoleucine of (III) may be a derivative thereof.

Regarding the test method, the solubility of a peptide in a solvent may be tested by measuring the solubility of the peptide in the solvent. In the measurement of solubility, the solvent is not limited, and may be freely selected according to the purpose. For the method of solubility measurement, a known method can be appropriately selected according to the type of solvent.

The test for binding ability to hTfR may be a measurement of binding ability to hTfR and is not limited to, but known methods such as the surface plasmon resonance (SPR) assay described above, Scatchard analysis and/or radioimmunoassay (RIA), and competitive-binding assay such as enzyme immunoassay (EIA) and sandwich competition assay and the like can be preferably used.

The test of toxicity to cells and/or tissues may be any known toxicity evaluation test using cells and/or tissues, e.g., in-vitro. Cells and tissues may be, but are not limited to, cells and/or tissues for which toxicity evaluation studies are usually performed for pharmaceutical products.

The test method for toxicity in experimental animals may be any known toxicity evaluation test using experimental animals. Experimental animals are not limited to those normally used, but examples include mouse, rat, guinea pig, gerbil, hamster, ferret, rabbit, dog, cat, pig, goat, horse, cow, bird (e.g., chicken, quail, etc.), monkey, primate other than human (e.g., capuchin monkey, marmoset, rhesus monkey, etc.), and the like.

The above toxicity assessment test can be, but are not limited to, safety test that is usually conducted in non-clinical test of pharmaceuticals, including, for example, general toxicity test (single-dose toxicity test/repeated-dose toxicity test), genotoxicity test (Ames test/chromosome aberration test/in-vitro micronucleus test), oncogenicity test, reproductive toxicity test (ICH-I, II, III), local irritation test (eye irritation test, skin irritation test, etc.), other toxicity test (skin sensitization test, phototoxicity test, antigenicity test), chemical analysis and bioanalysis (TK/PK) and the like.

The abbreviations used in this specification, especially in the following representative examples, are well known to those skilled in the art. Some of the abbreviations used are as follows:
AF647 as Alexa Fluor (registered trademark) 647;
Å as angstrom (unit);
CIAc as chloroacetyl;
Cy5SAlk as Sulfo-Cy5-alkyne;
DCM as dichloromethane;
TIPS as triisopropylsilyl;
tBu as *tert*-butyl;
DTT as dithiothreitol;
DMSO as dimethyl sulfoxide;
Trt as trityl;
Boc as *tert*-butoxycarbonyl;
DMF as N,N-dimethylformamide;
DIEA or DIPEA as N,N-diisopropylethylamine;
DIPCI as N,N'-diisopropylcarbodiimide;
Oxyma pure as ethyl cyano(hydroxyimino)acetate;
DODT as 3,6-dioxer-1,8-octane-dithiol;
Fmoc as 9-fluorenylmethyloxycarbonyl;
g as gram (unit);
HATU as O-(7-azabenzotriazol-1-yl)-N,N,N',N'-tetramethyluronium hexafluorophosphate;
HOSu as N-hydroxysuccinimide;
HPLC as high performance liquid chromatography;
LC-MS or LC/MS as liquid chromatography mass spectrometer;
mL as millilitre (unit);
M as molar (unit);
*µ*L as microliter (unit);
mM as millimolar (unit);
*µ*M as micromolar (unit);
mmol as millimole (unit);
mg as milligram (unit);
MeCN or CH₃CN as acetonitrile;
min as minute (unit);
mm as millimeter (unit);
*µ*m as micrometer (unit);
nm as nanometer (unit);
nM as nanomolar (units);
OSu as succinimide;
PEG as polyethylene glycol;
rpm as revolutions per minute (unit);
tBu as *tert*-butyl;
TFA as trifluoroacetic acid;
TIS as triisopropylsilane;
Trt or Tr as trityl group;
H-PEG4Me as 2,5,8,11-tetraoxatridecane-13-amine;
H-PEG8Me as 2,5,8,11,14,17,20,23-octaoxapentacosane-25-amine;
AA as amino acid;
PyAOP as 7-((azabenzotriazol-1-yloxy)tripyrrolidinophosphonium hexafluorophosphate; CSA as 10-camphorsulfonic acid;
Fmoc-OSu as N-(9-fluorenylmethoxycarbonyloxy)succinimide;
THF as tetrahydrofuran;
CIAcOSu as N-(chloroacetoxy)succinimide;
Pd₂(dba)₃ as Tris(dibenzylideneacetone)dipalladium (0);
SPhos as 2-Dicyclohexylphosphino-2',6'-dimethoxybiphenyl;
EDCI · HCl as 1-(3-Dimethylaminopropyl)-3-ethylcarbodiimide hydrochloride; and
Pd(PPh₃)₄ as Tetrakis(triphenylphosphine)palladium (0).

### Example 1

### Chemical Synthesis

Synthesis may be completed using commercially available products as is for all raw materials, building blocks, reagents, acids, bases, solid-phase resins, and solvents used in chemical synthesis in the following examples, or by a person having ordinary skill in the art using organic chemistry techniques. Note that commercial products were used for amino acids containing protecting groups unless otherwise specified.

Elongation of the peptide chain in a solid-phase resin is performed by using the resin described in each example as a starting material and using a standard peptide coupling reaction condition and Fmoc removal reaction condition. Reactions were carried out using Liberty Blue, an automated peptide synthesizer manufactured by CEM, in accordance with the manufacturer's manual. As an example, some of the common amino acids used are listed below, and side chain protecting groups are shown in parentheses.

Fmoc-Trp(Boc)-OH; Fmoc-Thr(tBu)-OH; Fmoc-N-Me-Gly-OH; Fmoc-Asp(OtBu)-OH; Fmoc-N-Me-Phe-OH; Fmoc-Ala-OH; Fmoc-N-Me-Ala-OH; Fmoc-His(Trt)-OH; Fmoc-Tyr(tBu)-OH; Fmoc-Val-OH; Fmoc-HydPro(tBu)-OH; Fmoc-Cys(Trt)-OH; Fmoc-Lys(Mtt)-OH; Fmoc-Ser(tBu)-OH; and Fmoc-N-Me-Ser(tBu)-OH.

Introduction of the chloroacetyl group was performed by removing the Fmoc group of the α-amino group from the solid-phase resin holding the Fmoc-protected peptide obtained in the previous step by the method described above, and then adding chloroacetic acid (3 equivalents) to a DMF solution of 3 equivalents of N,N'-diropropylcarbodiimide (0.5 M) and a DMF solution of 3 equivalents of HOAt (0.5 M) and shaking at room temperature for 40 min.

For deprotection of side chains and cut-out from the solid-phase resin, the resin obtained after the chloroacetyl group introduction step was first washed five times each with DMF and methylene chloride, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin and shaken at room temperature for 150 min. The reaction solution was collected by filtration through a frit. The solid-phase resin remaining in the reaction vessel was shaken again with the cut-out cocktail, and the solution component was collected from the frit and mixed with the aforementioned filtrate. When this filtrate was added to excess diethyl ether cooled to 0°C, a cloudy precipitate was formed. The mixture was centrifuged (9000 rpm, 3 min) and the solution was decanted. The resulting solid was washed again with a small amount of diethyl ether cooled to 0°C, and the resulting solid was used for the next cyclization reaction.

The peptide cyclization reaction was performed by dissolving the peptide in DMSO so that the final concentration of peptide was 5 mM based on the number of moles of solid-phase resin, adding 6 equivalents of triethylamine, and stirring at room temperature for about 16 hours. The resulting reaction solution was acidified with acetic acid and concentrated under reduced pressure using Biotage (registered trademark) V-10 (manufactured by Biotage Japan).

Reversed-phase separation HPLC was carried out as the method for purifying the obtained crude purified peptide using an AutoPurification System-SQD2 single quadruple mass spectrometer, manufactured by Waters, and elution was performed while monitoring m/z ions derived from the target product. It was confirmed that the mass spectrum obtained in ESI-positive scanning mode and the mass spectrum containing polyvalent ions calculated by the molecular formula of the target product matched within the error range of the mass spectrometer used. Note that the purification conditions including the columns used are shown in the respective examples.

As for the structure determination of chemically synthesized peptides, the molecular weight calculated in consideration of the amino acid used according to the target sequence and the building block used as necessary was confirmed by ESI-MS(+) in the mass spectrum analysis method. Note that "ESI-MS(+)" indicates an electrospray ionization mass spectrometry method performed in positive ion mode. The detected mass is reported in "m/z" units. Note that compounds having a molecular weight greater than approximately 1,000 are frequently detected as bivalent ions or trivalent ions. The analysis method are shown in the respective examples.

### Example 2

### Chemical Synthesis of Special Cyclic Peptides That Bind to hTfR

Peptides with binding activity to hTfR and hTfR-binding peptides with linkers were chemically synthesized. The sequences of the synthesized peptides are shown in TABLE 1, TABLE 2 and TABLE 5. The synthesized peptides were analyzed under the analytical conditions described in each example, and the structure was confirmed by ESI-MS(+) in mass spectrometry. The obtained ESI-MS(+) observation and the value of X when expressed as the number of proton additions (M+XH)X+ in such cases are shown in TABLE 1, TABLE 2 and TABLE 5. The sequence of the linker portion is shown in TABLE 3. TABLE 1 and TABLE 2 show the sequence and a linker portion and their observed values for a single sequence by combination of these two table portions due to their size. Note that ND in TABLE 2 indicates No Data and low signal indicates no activity. The C-terminal of only the peptide with the linker described in SEQ ID NO: 358 in TABLE 5 is -OH, while all other peptides are -NH₂. ND in TABLE 5 indicates No Data.

**[TABLE 1-1-1]**

| SEQ ID No. | Name | SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 1 | 894_0263 | A | V | MeF | V | W | N | Y | Y | I | I | K(MePEG4c) | R | F | MeY | C |
| 2 | 894_0264 | A | V | MeF | V | W | N | Y | Y | I | I | R | K(MePEG4c) | F | MeY | C |
| 3 | 894_0267 | A | V | MeF4F | V | W5C | N | Y | Y | I | I | MeR | K(MePEG4c) | F | MeY | C |
| 4 | 894_0268 | A | V | MeF4F | V | W5C | N | Y | F4OMe | I | I | MeR | K(MePEG4c) | F | MeY | C |
| 5 | 894_0269 | A | V | MeF4F | V | MeW | N | Y | Y | I | I | K(MePEG4c) | dr | F | MeY | C |
| 6 | 894_0270 | A | V | MeF4F | V | MeW | N | Y | F4OMe | ! | I | K(MePEG4c) | dr | F | MeY | C |
| 7 | 894_0271 | A | V | MeF4F | V | MeW | N | Y | Y | I | I | MeR | K(MePEG4c) | F | MeY | C |
| 8 | 894_0272 | A | V | MeF4F | V | MeW | N | Y | F40Me | I | I | MeR | K(MePEG4c) | F | MeY | C |
| 9 | 894_3152 | A | V | MeF | V | W | Ndm | Y | Y | I | I | R | R | Y | MeY | C |
| 10 | 894_0287 | MeA | V | MeF3C | V | MeW | N | Y | F40Me | I | I | R | da | F | MeY | C |
| 11 | 894_3149 | A | V | MeF3C | V | W | N | Y | F4OMe | I | I | R | R | F | MeY | C |
| 12 | 894_3150 | A | V | MeF3C | V | W | N | Y | F4OMe | I | I | K | R | F | MeY | C |
| 13 | 894_3151 | A | V | MeF | V | W | N | Y | Y | I | I | R | R | Y | MeY | C |
| 14 | 894_0282 | A | V | MeF | V | W | N | Y | Y | I | I | R | dr | F | MeY | C |
| 15 | 894_0283 | A | V | MeF | V | W | N | Y | F4OMe | I | I | R | dr | F | MeY | C |
| 16 | 894_0284 | A | V | MeF | V | W | N | Y | Y | I | I | MeR | R | F | MeY | C |
| 17 | 894_0285 | A | V | MeF | V | W | N | Y | F40Me | I | I | MeR | R | F | MeY | C |
| 18 | 894_0250 | A | V | MeF | V | W5C | N | Y | Y | I | I | R | R | F | MeY | C |
| 19 | 894_0251 | A | V | MeF | V | W5C | N | Y | F4OMe | I | I | R | R | F | MeY | C |
| 20 | 894_0252 | A | V | MeF | V | W5C | N | Y | Y | I | I | R | dr | F | MeY | C |
| 21 | 894_0253 | A | V | MeF | V | W5C | N | Y | F40Me | I | I | R | dr | F | MeY | C |
| 22 | 894_0254 | A | V | MeF4F | V | W5C | N | Y | Y | I | I | R | R | F | MeY | C |
| 23 | 894_0255 | A | V | MeF4F | V | W5C | N | Y | F4OMe | I | I | R | R | F | MeY | C |
| 24 | 894_0256 | A | V | MeF4F | V | W5C | N | Y | Y | I | I | R | dr | F | MeY | C |
| 25 | 894_0257 | A | V | MeF4F | V | W5C | N | Y | Y | I | I | MeR | dr | F | MeY | C |

**[Table 1-1-2]**

| Linker | | | m/z | [M+XH]X+ | SPR |
|---|---|---|---|---|---|
| | | | 1136.17 | 2 | B |
| | | | 1136.23 | 2 | B |
| | | | 1169.42 | 2 | C |
| | | | 1176.45 | 2 | B |
| | | | 1152.21 | 2 | B |
| | | | 1159.21 | 2 | B |
| | | | 1159.19 | 2 | B |
| | | | 1166.21 | 2 | B |
| | | | 1062.78 | 2 | C |
| PEG4c | | | 1160.49 | 2 | B |
| G | KN3 | | 1170.92 | 2 | B |
| G | KN3 | | 1156.94 | 2 | B |
| G | KN3 | | 1154.72 | 2 | A |
| PEG4c | | | 1164.76 | 2 | B |
| PEG4c | | | 1171.74 | 2 | B |
| PEG4c | | | 1171.74 | 2 | B |
| PEG4c | | | 1178.76 | 2 | B |
| PEG4c | | | 1181.91 | 2 | A |
| PEG4c | | | 1188.93 | 2 | A |
| PEG4c | | | 1181.94 | 2 | B |
| PEG4c | | | 1188.95 | 2 | B |
| PEG4c | | | 1190.93 | 2 | A |
| PEG4c | | | 1198.00 | 2 | A |
| PEG4c | | | 1190.95 | 2 | B |
| PEG4c | | | 1197.96 | 2 | C |

**[Table1-2-1]**

| | | | | | | | | | | | | | | | | |
|---|---|---|---|---|---|---|---|---|---|---|---|---|---|---|---|---|
| 26 | 894_0258 | A | V | MeF4F | V | W5C | N | Y | F4OMe | I | I | R | dr | F | MeY | C |
| 27 | 894_0259 | A | V | MeF4F | V | W5C | N | Y | F4OMe | I | I | MeR | dr | F | MeY | C |
| 28 | 894_0260 | A | V | MeF4F | V | MeW | N | Y | Y | I | I | R | dr | F | MeY | C |
| 29 | 894_0261 | A | V | MeF4F | V | MeW | N | Y | F40Me | I | I | R | dr | F | MeY | C |
| 30 | 894_0262 | A | V | MeF4F | V | MeW | N | Y | F4OMe | I | I | MeR | dr | F | MeY | C |
| 31 | 894_0280 | A | V | MeF3C | V | W | N | Y | F4OMe | I | I | R | R | F | MeY | C |
| 32 | 894_0281 | A | V | MeF3C | V | W | N | Y | F4OMe | I | I | K | R | F | MeY | C |
| 33 | 894_0282 | A | V | MeF | V | W | N | Y | Y | I | I | R | dr | F | MeY | C |
| 34 | 894_0283 | A | V | MeF | V | W | N | Y | F4OMe | I | I | R | dr | F | MeY | C |
| 35 | 894_0284 | A | V | MeF | V | W | N | Y | Y | I | I | MeR | R | F | MeY | C |
| 36 | 894_0285 | A | V | MeF | V | W | N | Y | F4OMe | I | I | MeR | R | F | MeY | C |
| 37 | 894_0289 | A | V | MeF | V | W5C | N | Y | F4OMe | I | I | R | R | Y | MeY | C |
| 38 | 894_0291 | A | V | MeF3C | V | W | N | Y | F4OMe | I | I | R | R | Y | MeY | C |
| 39 | 894_3167 | A | V | MeF3C | V | W1aa | N | Y | F4OMe | I | E | R | R | F | MeY | C |
| 40 | 894_3168 | A | V | MeF3C | V | W1aa | N | Y | F4aao | I | I | R | R | F | MeY | C |
| 41 | 894_3169 | A | V | MeF | V | W | N | Y | F4aao | I | I | R | R | Y | MeY | C |
| 42 | 894_3170 | A | V | MeF | V | W1aa | N | Y | F4aao | I | I | R | R | Y | MeY | C |
| 43 | 894_0294 | A | V | MeF3C | V | W1aa | N | Y | F4OMe | I | E | R | R | F | MeY | C |
| 44 | 894_0295 | A | V | MeF3C | V | W1aa | N | Y | F4aao | I | I | R | R | F | MeY | C |
| 45 | 894_0296 | A | V | MeF | V | W | N | Y | F4aao | I | I | R | R | Y | MeY | C |
| 46 | 894_0297 | A | V | MeF | V | W1aa | N | Y | F4aao | I | I | R | R | Y | MeY | C |
| 47 | 894_3182 | A | V | MeF3C | V | W1aa | N | Y | F4aao | I | I | R | R | F | MeY | C |
| 48 | 894_0306 | A | V | MeF3C | V | W1aa | N | Y | 3Py | I | I | R | R | 4Py | MeY | C |
| 49 | 894_0307 | A | V | MeF3C | V | W1aa | N | 3Py6NH2 | 3Py | I | I | R | R | 4Py | MeY | C |
| 50 | 894_0308 | A | V | MeF3C | V | W1aa | N | 3Py6NH2 | F4OMe | I | I | R | R | 4Py | MeY | C |

**[Table 1-3-1]**

| | | | | | | | | | |
|---|---|---|---|---|---|---|---|---|---|
| PEG4c | | | | | | | 1198.01 | 2 | B |
| PEG4c | | | | | | | 1204.94 | 2 | B |
| PEG4c | | | | | | | 1180.75 | 2 | B |
| PEG4c | | | | | | | 1187.73 | 2 | B |
| PEG4c | | | | | | | 1194.77 | 2 | B |
| PEG4c | | | | | | | 1188.91 | 2 | A |
| PEG4c | | | | | | | 1174.95 | 2 | A |
| PEG4c | | | | | | | 1164.76 | 2 | B |
| PEG4c | | | | | | | 1171.74 | 2 | B |
| PEG4c | | | | | | | 1171.74 | 2 | B |
| PEG4c | | | | | | | 1178.76 | 2 | B |
| PEG4c | | | | | | | 1196.91 | 2 | A |
| PEG4c | | | | | | | 1197.11 | 2 | A |
| G | de | de | de | ds | ds | KN3 | 1488.62 | 2 | A |
| G | de | de | de | ds | ds | KN3 | 1002.06 | 3 | A |
| G | de | de | de | ds | ds | KN3 | 1464.4 | 2 | B |
| G | de | de | de | ds | ds | KN3 | 1493.42 | 2 | B |
| PEG4c | | | | | | | 1225.89 | 2 | B |
| PEG4c | | | | | | | 1239.9 | 2 | B |
| PEG4c | | | | | | | 1201.69 | 2 | B |
| PEG4c | | | | | | | 821.1 | 3 | B |
| G | PEG8c | KN3 | | | | | 956.06 | 3 | B |
| PEG4c | | | | | | | 1203.89 | 2 | B |
| PEG4c | | | | | | | 802.95 | 3 | B |
| PEG4c | | | | | | | 812.62 | 3 | B |

**[Table1-3-1]**

| | | | | | | | | | | | | | | | | |
|---|---|---|---|---|---|---|---|---|---|---|---|---|---|---|---|---|
| 51 | 894_0309 | A | V | MeF3C | V | W | N | Y | 3Py | I | I | R | R | 4Py | MeY | C |
| 52 | 894_0310 | A | V | MeF3C | V | W | N | 3Py6NH2 | 3Py | I | I | R | R | 4Py | MeY | C |
| 53 | 894_0311 | A | V | MeF3C | V | W | N | 3Py6NH2 | F4OMe | I | I | R | R | 4Py | MeY | C |
| 54 | 894_0312 | A | V | MeF3C | V | W | N | Y | 4Py | I | I | R | R | 4Py | MeY | C |
| 55 | 894_0313 | A | V | Me3Py | V | W | N | Y | F4OMe | I | I | R | R | F | MeY | C |
| 56 | 894_0314 | A | V | Me4Py | V | W | N | Y | F4OMe | I | I | R | R | F | MeY | C |
| 57 | 894_0315 | A | V | MeF3C | V | W | N | 3Py6NH2 | F4OMe | I | I | R | R | F | MeY | C |
| 58 | 894_0316 | A | V | Me3Py | V | W | N | Y | F4OMe | I | I | R | R | 4Py | MeY | C |
| 59 | 894_0317 | A | V | MeF3C | V | W | N | Y | F4OMe | I | I | R | R | 4Py | MeY | C |
| 60 | 894_0318 | A | V | MeF3C | V | W | N | Y | 3Py | I | I | R | R | F | MeY | C |
| 61 | 894_0319 | A | V | MeF3C | V | W | N | Y | 4Py | I | I | R | R | F | MeY | C |
| 62 | 894_0320 | A | V | MeF3C | V | W | N | 3Py6NH2 | 4Py | I | I | R | R | 4Py | MeY | C |
| 63 | 894_0322 | A | V | Me3Py | V | W | N | 3Py6NH2 | F4OMe | I | I | R | R | F | MeY | C |
| 64 | 894_0323 | A | V | Me4Py | V | W | N | 3Py6NH2 | F4OMe | I | I | R | R | F | MeY | C |
| 65 | 894_0324 | A | V | MeF3C | V | W7N | N | Y | F4OMe | I | I | R | R | 4Py | MeY | C |
| 66 | 894_0325 | A | V | MeF3C | V | W | N | Y | F4OMe | I | I | R | R | 4Py | Me3Py | C |
| 67 | 894_0327 | A | V | MeF3C | V | W | N | Y | 3Py | I | I | Q | Q | 4Py | MeY | C |
| 68 | 894_3192 | A | V | MeF3C | V | W | N | Y | 3Py | I | I | R | R | 4Py | MeY | C |
| 69 | 894_3193 | A | V | MeF3C | V | W | N | Y | 3Py | I | I | R | R | 4Py | MeY | C |
| 70 | 894_0334 | A | V | MeF3C | V | W1aa | N | Y | F4OMe | I | I | R | E(PEG8Me) | F | MeY | C |
| 71 | 894_0337 | A | V | MeF3C | V | W1aa | N | 3Py6NH2 | F4aao | I | I | R | R | F | MeY | C |
| 72 | 894_0339 | A | V | MeF3C | V | W1aa | N | 3Py6NH2 | F4OMe | I | I | R | R | F3COO | MeY | C |
| 73 | 894_0340 | A | V | MeF3C | V | W1aa | N | Y | 3Py | I | I | R | E(PEG8Me) | F | MeY | C |
| 74 | 894_0341 | A | V | MeF3C | V | W1aa | N | Y | 3Py | I | I | R | R | F3COO | MeY | C |
| 75 | 894_0342 | A | V | MeF3C | V | W1aa | N | Y | F4aao | I | I | R | R | F3COO | MeY | C |

**[Table1-3-2]**

| | | | | | | | | | |
|---|---|---|---|---|---|---|---|---|---|
| PEG4c | | | | | | | 1174.9 | 2 | B |
| PEG4c | | | | | | | 783.62 | 3 | B |
| PEG4c | | | | | | | 793.3 | 3 | B |
| PEG4c | | | | | | | 783.58 | 3 | B |
| PEG4c | | | | | | | 1172.22 | 2 | B |
| PEG4c | | | | | | | 1172.16 | 2 | B |
| PEG4c | | | | | | | 1188.92 | 2 | B |
| PEG4c | | | | | | | 782.15 | 3 | B |
| PEG4c | | | | | | | 1189.4 | 2 | A |
| PEG4c | | | | | | | 1174.34 | 2 | B |
| PEG4c | | | | | | | 1174.39 | 2 | B |
| PEG4c | | | | | | | 783.89 | 3 | B |
| PEG4c | | | | | | | 1172.51 | 2 | B |
| PEG4c | | | | | | | 782.06 | 3 | B |
| PEG4c | | | | | | | 793.86 | 3 | B |
| PEG4c | | | | | | | 1181.94 | 2 | B |
| PEG4c | | | | | | | 1146.86 | 2 | B |
| G | dkCOmeglumine | dkCOmeglumine | dkCOmeglumine | ds | ds | KN3 | 1178.96 | 3 | B |
| G | de | de | de | ds | ds | KN3 | 1437.61 | 2 | B |
| PEG4c | | | | | | | 1387.15 | 2 | B |
| PEG4c | | | | | | | 826.98 | 3 | B |
| PEG4c | | | | | | | 1239.95 | 2 | B |
| PEG4c | | | | | | | 1372.63 | 2 | B |
| PEG4c | | | | | | | 1225.39 | 2 | B |
| PEG4c | | | | | | | 1261.9 | 2 | B |

**[TABLE 1-4-1]**

| | | | | | | | | | | | | | | | | |
|---|---|---|---|---|---|---|---|---|---|---|---|---|---|---|---|---|
| 76 | 894_0343 | A | V | MeF3C | V | W1aa | N | Y | F4aao | I | I | R | R | 4Py | MeY | C |
| 77 | 894_0344 | A | V | MeF3C | V | W1aa | N | Y | F4OMe | I | I | R | E(PEG8Me) | F3COO | MeY | C |
| 78 | 894_0345 | A | V | MeF3C | V | W1aa | N | Y | F4OMe | I | I | R | E(PEG8Me) | 4Py | MeY | C |
| 79 | 894_0347 | A | V | MeF3C | V | W1aa | N | 3Py6NH2 | 3Py | I | I | R | R | F3COO | MeY | C |
| 80 | 894_0348 | A | V | MeF3C | V | W1aa | N | 3Py6NH2 | F4aao | I | I | R | R | 4Py | MeY | C |
| 81 | 894_0349 | A | V | MeF3C | V | W1aa | N | 3Py6NH2 | F4aao | I | I | R | R | F3COO | MeY | C |
| 82 | 894_0350 | A | V | MeF3C | V | W1aa | N | Y | F4aao | I | I | R | E(PEG8Me) | 4Py | MeY | C |
| 83 | 894_0351 | A | V | MeF3C | V | W1aa | N | Y | 3Py | I | I | R | E(PEG8Me) | 4Py | MeY | C |
| 84 | 894_0352 | A | V | MeF3C | V | W1aa | N | Y | 3Py | I | I | R | E(PEG8Me) | F3COO | MeY | C |
| 85 | 894_0353 | A | V | MeF3C | V | W1aa | N | 3Py6NH2 | 3Py | I | I | R | E(PEG8Me) | F3COO | MeY | C |
| 86 | 894_0354 | A | V | MeF3C | V | W1aa | N | 3Py6NH2 | 3Py | I | I | R | E(PEG8Me) | 4Py | MeY | C |
| 87 | 894_0355 | A | V | MeF3C | V | W1aa | N | 3Py6NH2 | F4aao | I | I | R | E(PEG8Me) | F3COO | MeY | C |
| 88 | 894_0356 | A | V | MeF3C | V | W1aa | N | 3Py6NH2 | F4aao | I | I | R | E(PEG8Me) | 4Py | MeY | C |
| 89 | 894_0362 | A | V | MeF3C | V | W | N | Y | F4aao | I | I | R | E(PEG8Me) | F | MeY | C |
| 90 | 894_0365 | A | V | MeF3C | V | W | N | Y | F4OMe | I | I | R | E(PEG8Me) | F3COO | MeY | C |
| 91 | 894_0368 | A | V | MeF3C | V | W | N | 3Py6NH2 | F4aao | I | I | R | R | F3COO | MeY | C |
| 92 | 894_0369 | A | V | MeF3C | V | W | N | 3Py6NH2 | F4aao | I | I | R | R | 4Py | MeY | C |
| 93 | 894_0370 | A | V | MeF3C | V | W | N | Y | 3Py | I | I | R | E(PEG8Me) | F3COO | MeY | C |
| 94 | 894_0371 | A | V | MeF3C | V | W | N | Y | 3Py | I | I | R | E(PEG8Me) | 4Py | MeY | C |
| 95 | 894_0372 | A | V | MeF3C | V | W | N | Y | F4aao | I | I | R | E(PEG8Me) | F3COO | MeY | C |
| 96 | 894_0373 | A | V | MeF3C | V | W | N | Y | F4aao | I | I | R | E(PEG8Me) | 4Py | MeY | C |
| 97 | 894_0374 | A | V | MeF3C | V | W | N | 3Py6NH2 | 3Py | I | I | R | E(PEG8Me) | F3COO | MeY | C |
| 98 | 894_0375 | A | V | MeF3C | V | W | N | 3Py6NH2 | 3Py | I | I | R | E(PEG8Me) | 4Py | MeY | C |
| 99 | 894_0376 | A | V | MeF3C | V | W | N | 3Py6NH2 | F4aao | I | I | R | E(PEG8Me) | F3COO | MeY | C |
| 100 | 894_0377 | A | V | MeF3C | V | W | N | 3Py6NH2 | F4aao | I | I | R | E(PEG8Me) | 4Py | MeY | C |

**[TABLE 1-4-2]**

| | | | | |
|---|---|---|---|---|
| PEG4c | | 1240.39 | 2 | B |
| PEG4c | | 1409.13 | 2 | B |
| PEG4c | | 1387.66 | 2 | B |
| PEG4c | | 817.29 | 3 | B |
| PEG4c | | 1240.42 | 2 | B |
| PEG4c | | 1261.95 | 2 | B |
| PEG4c | | 1409.63 | 2 | B |
| PEG4c | | 1373.1 | 2 | B |
| PEG4c | | 1394.72 | 2 | B |
| PEG4c | | 1394.69 | 2 | C |
| PEG4c | | 915.78 | 3 | C |
| PEG4c | | 954.4 | 3 | C |
| PEG4c | | 940.08 | 3 | C |
| PEG4c | | 1380.14 | 2 | B |
| PEG4c | | 920.68 | 3 | B |
| PEG4c | | 1232.95 | 2 | B |
| PEG4c | | 807.94 | 3 | B |
| PEG4c | | 910.73 | 3 | B |
| PEG4c | | 896.38 | 3 | B |
| PEG4c | | 1402.07 | 2 | B |
| PEG4c | | 1380.62 | 2 | B |
| PEG4c | | 1365.59 | 2 | B |
| PEG4c | | 896.36 | 3 | B |
| PEG4c | | 935.05 | 3 | B |
| PEG4c | | 920.74 | 3 | B |

**[TABLE 1-5-1]**

| | | | | | | | | | | | | | | | | |
|---|---|---|---|---|---|---|---|---|---|---|---|---|---|---|---|---|
| 101 | 894_0298 | A | V | MeF3C | V | W1aa | N | Y | F4aao | I | I | E(PEG8Me) | R | F | MeY | C |
| 102 | 894_0299 | A | V | MeF3C | V | W1aa | N | Y | F4aao | I | I | R | E(PEG8Me) | F | MeY | C |
| 103 | 894_0301 | A | V | MeF3C | V | W1aa | N | Y | F4aao | I | I | E(PEG8Me) | R | F3COO | MeY | C |
| 104 | 894_0302 | A | V | MeF3C | V | W1aa | N | Y | F4aao | I | I | Q | R | F | MeY | C |
| 105 | 894_0303 | A | V | MeF3C | V | W1aa | N | Y | F4aao | I | I | R | Q | F | MeY | C |
| 106 | 894_0393 | A | V | MeF | V | W | N | Y | Y | I | I | R | R | Y | MeY | C |
| 107 | 894_0394 | A | V | MeF | V | W | N | Y | Y | I | I | R | R | Y | MeY | C |
| 108 | 894_0391 | A | V | MeF3C | V | W1aa | N | Y | F4OMe | I | I | R | R | F | MeY | C |
| 109 | 894_0392 | A | V | MeF3C | V | W | N | Y | F4aao | I | I | R | R | F | MeY | C |
| 110 | 894_0395 | A | V | MeF3C | V | W1aa | N | Y | F4aao | I | I | E(PEG4Me) | R | F | MeY | C |
| 111 | 894_0396 | A | V | MeF3C | V | W1aa | N | Y | F4aao | I | I | E(NHdPEG1Me) | R | F | MeY | C |
| 112 | 849_0397 | A | V | MeF3C | V | W | N | Y | F4aao | I | I | E(PEG8Me) | R | F | MeY | C |
| 113 | 849_0398 | A | V | MeF3C | V | W | N | Y | F4aao | I | I | E(PEG4Me) | R | F | MeY | C |
| 114 | 849_0399 | A | V | MeF3C | V | W | N | Y | F4aao | I | I | E(NHdPEG1Me) | R | F3COO | MeY | C |
| 115 | 849_0400 | A | V | MeF3C | V | W1aa | N | Y | F4aao | I | I | E(PEG4Me) | KCOpipzaa | F | MeY | C |
| 116 | 849_0401 | A | V | MeF3C | V | W | N | Y | F4aao | I | I | E(PEG4Me) | KCOpipzaa | F | MeY | C |
| 117 | 849_0402 | A | V | MeF3C | V | W1aa | N | 3Py6NH2 | F4aao | I | I | E(PEG4Me) | R | 4Py | MeY | C |
| 118 | 849_0403 | A | V | MeF3C | V | W | N | 3Py6NH2 | F4aao | I | I | E(PEG4Me) | KCOpipzaa | 4Py | MeY | C |
| 119 | 849_0404 | A | V | MeF3C | V | W1aa | N | Y | F4aao | I | I | E(PEG4Me) | Kmor | F | MeY | C |
| 120 | 849_0405 | A | V | MeF3C | V | W | N | Y | F4aao | I | I | E(PEG4Me) | Kmor | F | MeY | C |
| 121 | 849_0406 | A | V | MeF3C | V | W | N | Y | F4aao | I | I | E(PEG4Me) | K | F | MeY | C |
| 122 | 849_0407 | A | V | MeF3C | V | W | N | Y | F4aao | I | I | E(PEG4Me) | KdMe | F | MeY | C |
| 123 | 849_0408 | A | V | MeF3C | V | W | N | Y | F4aao | I | I | E(PEG4Me) | Q | F | MeY | C |
| 124 | 849_0409 | A | V | MeF3C | V | W | N | Y | F4aao | I | I | E(PEG4Me) | R | E(PEG4Me) | MeY | C |
| 125 | 849_0410 | A | V | MeF3C | V | W | N | Y | F4aao | I | I | E(PEG4Me) | R | E(PEG4Me) | MeY | C |

**[TABLE 1-5-2]**

| | | | | | | | | | |
|---|---|---|---|---|---|---|---|---|---|
| PEG4c | | | | | | | 939.99 | 3 | B |
| PEG4c | | | | | | | 939.92 | 3 | B |
| PEG4c | | | | | | | 1431.19 | 2 | B |
| PEG4c | | | | | | | 1225.93 | 2 | B |
| PEG4c | | | | | | | 1225.95 | 2 | B |
| G | de | de | de | S | S | C(AcNMe) | 1409.37 | 2 | B |
| G | de | de | de | de | de | C(AcNMe) | 992 | 3 | B |
| PEG4c | | | | | | | 1217.93 | 2 | A |
| PEG4c | | | | | | | 1210.97 | 2 | A |
| PEG4c | | | | | | | 1321.01 | 2 | B |
| PEG4c | | | | | | | 1276.91 | 2 | B |
| PEG4c | | | | | | | 1380.09 | 2 | A |
| PEG4c | | | | | | | 1291.96 | 2 | B |
| PEG4c | | | | | | | 1269.94 | 2 | A |
| PEG4c | | | | | | | 1392.05 | 2 | B |
| PEG4c | | | | | | | 1363.06 | 2 | B |
| PEG4c | | | | | | | 1321.52 | 2 | B |
| PEG4c | | | | | | | 1363.58 | 2 | B |
| PEG4c | | | | | | | 1342.07 | 2 | A |
| PEG4c | | | | | | | 1313 | 2 | B |
| PEG4c | | | | | | | 1277.93 | 2 | B |
| PEG4c | | | | | | | 1291.99 | 2 | B |
| PEG4c | | | | | | | 1277.9 | 2 | B |
| PEG4c | | | | | | | 918.79 | 3 | B |
| G | ds | ds | ds | ds | ds | | 1000.49 | 3 | B |

**[TABLE 1-6-1]**

| | | | | | | | | | | | | | | | | |
|---|---|---|---|---|---|---|---|---|---|---|---|---|---|---|---|---|
| 126 | 849_0411 | A | V | MeF3C | V | W | N | Y | F4aao | I | I | E(PEG4Me) | R | F3COO(PEG4Me) | MeY | C |
| 127 | 849_0412 | A | V | MeF3C | V | W | N | Y | F4aao | I | I | Q | R | F3COO(PEG4Me) | MeF4COO(PEG4Me) | C |
| 128 | 849_0413 | A | V | MeF3C | V | W | N | Y | F4aao | I | I | Q | R | F3COO(PEG4Me) | MeF3COO(PEG4Me) | C |
| 129 | 849_0414 | A | V | MeF3C | V | W | N | Y | F4aao | I | I | Q | R | F3COO(PEG4Me) | MeF3COO(PEG4Me) | C |
| 130 | 849_0415 | A | V | MeF3C | V | W7N | N | Y | F4aao | I | I | E(PEG4Me) | R | F3COO(PEG4Me) | MeY | C |
| 131 | 849_0416 | A | V | MeF3C | V | W7N | N | Y | F4aao | I | I | Q | R | F3COO(PEG4Me) | MeF4COO(PEG4Me) | C |
| 132 | 849_0417 | A | V | MeF3C | V | W7N | N | Y | F4aao | I | I | Q | R | F3COO(PEG4Me) | MeF3COO(PEG4Me) | C |
| 133 | 849_0418 | A | V | MeF3C | V | W7N | N | Y | F4aao | I | I | E(PEG4Me) | R | F | MeY | C |
| 134 | 894_0419 | A | V | MeF3C | V | W1aa | N | Y | F4aao | I | I | Q | R | 4Py | MeY | C |
| 135 | 894_0420 | A | V | MeF3C | V | W1aa | N | Y | F4aao | I | I | Q | Kmor | 4Py | MeY | C |
| 136 | 894_0421 | A | V | MeF3C | V | W1aa | N | Y | F4aao | I | I | Q | R | F3COO | MeY | C |
| 137 | 894_0424 | A | V | MeF3C | V | W7N | N | Y | F4aao | I | I | Q | R | 4Py | MeY | C |
| 138 | 894_0425 | A | V | MeF3C | V | W7N | N | Y | F4aao | I | I | Q | Kmor | 4Py | MeY | C |
| 139 | 894_0426 | A | V | MeF3C | V | W7N | N | Y | F4aao | I | I | E(PEG8Me) | Kmor | 4Py | MeY | C |
| 140 | 894_0428 | A | V | MeF3C | V | W7N | N | Y | F4aao | I | I | Q | Kmor | F3COO | MeY | C |
| 141 | 894_0455 | A | V | MeF3C | V | W | N | 3Py6NH2 | F4aao | I | I | E(PEG8Me) | R | 4Py | MeY | C |

**[TABLE 1-6-2]**

| | | | | | | | | |
|---|---|---|---|---|---|---|---|---|
| PEG4c | | | | | | 1408.64 | 2 | B |
| PEG4c | | | | | | 1422.13 | 2 | B |
| PEG4c | | | | | | 1422.14 | 2 | B |
| G | ds | ds | ds | ds | ds | 1030.14 | 3 | B |
| PEG4c | | | | | | 1409.14 | 2 | B |
| PEG4c | | | | | | 1422.62 | 2 | B |
| PEG4c | | | | | | 1422.63 | 2 | B |
| PEG4c | | | | | | 1292.48 | 2 | B |
| PEG4c | | | | | | 1226.44 | 2 | B |
| PEG4c | | | | | | 1247.58 | 2 | B |
| PEG4c | | | | | | 1247.94 | 2 | B |
| PEG4c | | | | | | 798.99 | 3 | B |
| PEG4c | | | | | | 813.06 | 3 | B |
| PEG4c | | | | | | 935.2 | 3 | B |
| PEG4c | | | | | | 827.44 | 3 | B |
| PEG4c | | | | | | 920.8 | 3 | C |

**[TABLE 2-1-1]**

| SEQ ID No. | Name | SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 142 | 894_3153 | A | V | MeF | V | W5C | N | Y | Y | I | I | R | R | F | MeY | C |
| 143 | 894_3154 | A | V | MeF | V | W5C | N | Y | F4OMe | I | I | R | R | F | MeY | C |
| 144 | 894_3155 | A | V | MeF | V | W5C | N | Y | F4OMe | I | I | R | dr | F | MeY | C |
| 145 | 894_3156 | A | V | MeF4F | V | W5C | N | Y | F4OMe | I | I | R | R | F | MeY | C |
| 146 | 894_3157 | A | V | MeF4F | V | W5C | N | Y | F4OMe | I | I | R | dr | F | MeY | C |
| 147 | 894_3158 | A | V | Me4Py | V | MeW | N | Y | F4OMe | I | I | R | dr | F | MeY | C |
| 148 | 894_3159 | A | V | MeF3COO | V | MeW | N | Y | F4OMe | I | I | R | dr | F | MeY | C |
| 149 | 894_3160 | A | V | MeF | V | W | A | Y | Y | I | I | R | R | Y | MeY | C |
| 150 | 894_3161 | A | V | MeF | V | W5C | N | Y | Y | I | I | R | R | Y | MeY | C |
| 151 | 894_3162 | A | V | MeF | V | W5C | N | Y | F4OMe | I | 1 | R | R | Y | MeY | C |
| 152 | 894_3163 | A | V | MeF4F | V | W5C | N | Y | F4OMe | I | I | R | R | Y | MeY | C |
| 153 | 894_3164 | A | V | MeF3C | V | W | N | Y | F4OMe | I | I | R | R | Y | MeY | C |
| 154 | 894_3165 | A | V | MeF3C | V | W | N | Y | F4OMe | I | I | K | R | Y | MeY | C |
| 155 | 894_3166 | A | V | MeF3C | V | MeW | N | Y | F4OMe | I | I | R | R | Y | MeY | C |
| 156 | 894_0288 | A | V | MeF | V | W5C | N | Y | Y | I | I | R | R | Y | MeY | C |
| 157 | 894_3171 | A | V | MeF3C | V | W1aa | N | Y | F4aao | I | I | R | R | F | MeY | C |
| 158 | 894_3172 | A | V | MeF3C | V | W1aa | N | Y | F4aao | I | I | E(PEG8Me) | R | F | MeY | C |
| 159 | 894_3173 | A | V | MeF3C | V | W1aa | N | Y | F4aao | I | I | R | E(PEG8Me) | F | MeY | C |
| 160 | 894_3174 | A | V | MeF3C | V | W1aa | N | Y | F4aao | I | I | E(PEG8Me) | E(PEG8Me) | F | MeY | C |
| 161 | 894_3175 | A | V | MeF3C | V | W1aa | N | Y | F4aao | I | I | E(PEG8Me) | R | F3COO | MeY | C |
| 162 | 894_3176 | A | V | MeF3C | V | W1aa | N | Y | F4aao | I | I | Q | R | F | MeY | C |
| 163 | 894_3177 | A | V | MeF3C | V | W1aa | N | Y | F4aao | I | I | R | Q | F | MeY | C |
| 164 | 894_3178 | A | V | MeF3C | V | W1aa | N | Y | F4aao | I | I | Q | Q | F | MeY | C |
| 165 | 894_3180 | A | V | MeF3C | V | W1aa | N | Y | F4OMe | I | E | E(PEG8Me) | E(PEG8Me) | F | MeY | C |

**[TABLE 2-1-2]**

| Linker | | | | | | | m/z | [M+XH]X+ | SPR |
|---|---|---|---|---|---|---|---|---|---|
| G | KN3 | | | | | | 1164.01 | 2 | B |
| G | KN3 | | | | | | 1171.05 | 2 | B |
| G | KN3 | | | | | | 1171.09 | 2 | B |
| G | KN3 | | | | | | 1180.1 | 2 | B |
| G | KN3 | | | | | | 1180.07 | 2 | C |
| G | KN3 | | | | | | 774.56 | 3 | B |
| G | KN3 | | | | | | 1182.91 | 2 | C |
| G | KN3 | | | | | | 1133.35 | 2 | C |
| G | KN3 | | | | | | 1172.1 | 2 | B |
| G | KN3 | | | | | | 1179.09 | 2 | B |
| G | KN3 | | | | | | 1188.13 | 2 | B |
| G | KN3 | | | | | | 1179.14 | 2 | B |
| G | KN3 | | | | | | 1165.1 | 2 | B |
| G | KN3 | | | | | | 1186.14 | 2 | B |
| PEG4c | | | | | | | 1189.95 | 2 | B |
| G | KN3 | | | | | | 1221.95 | 2 | B |
| G | KN3 | | | | | | 1391.19 | 2 | B |
| G | KN3 | | | | | | 1391.15 | 2 | B |
| G | KN3 | | | | | | 1040.55 | 3 | Low signal |
| G | KN3 | | | | | | 942.39 | 3 | Low signal |
| G | KN3 | | | | | | 1207.92 | 2 | B |
| G | KN3 | | | | | | 1207.91 | 2 | B |
| G | KN3 | | | | | | 1193.88 | 2 | B |
| G | de | de | de | ds | ds | KN3 | 1218.65 | 3 | Low signal |

**[TABLE 2-2-1]**

| SEQ ID No. | Name | SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 166 | 894_3181 | A | V | MeF3C | V | W1aa | N | Y | F4aao | I | I | E(PEG8Me) | E(PEG8Me) | F | MeY | C |
| 167 | 894_3183 | A | V | MeF3C | V | W1aa | N | Y | F4aao | I | I | E(PEG8Me) | E(PEG8Me) | F | MeY | C |
| 168 | 894_3184 | A | V | MeF3C | V | W1aa | N | Y | F4aao | I | I | E(PEG8Me) | E(PEG8Me) | F3COO | MeY | C |
| 169 | 894_0321 | A | V | Me3Py | V | W | N | 3Py6NH2 | 3Py | I | I | R | R | 4Py | MeY | C |
| 170 | 894_0290 | A | V | MeF4F | V | W5C | N | Y | F4OMe | I | I | R | R | Y | MeY | C |
| 171 | 894_0292 | A | V | MeF3C | V | W | N | Y | F4OMe | I | I | K | R | Y | MeY | C |
| 172 | 894_0293 | A | V | MeF3C | V | MeW | N | Y | F4OMe | I | I | R | R | Y | MeY | C |
| 173 | 894_0326 | A | V | MeF3C | V | \N | N | Y | 3Py | I | I | E(PEG1Me) | E(PEG1Me) | 4Py | MeY | C |
| 174 | 894_0328 | A | V | MeF3C | V | W | N | Y | 3Py | I | I | E(Glucamine) | E(Glucamine) | 4Py | MeY | C |
| 175 | 894_0329 | A | V | MeF3C | V | W1aa | N | Y | F4aao | I | I | E(PEG1Me) | E(PEG1Me) | F3COO | MeY | C |
| 176 | 894_0330 | A | V | MeF3C | V | W | N | Y | F4OMe | I | I | E(PEG1Me) | E(PEG1Me) | F | MeY | C |
| 177 | 894_0331 | A | V | MeF3C | V | W | N | Y | F4OMe | I | I | Q | Q | F | MeY | C |
| 178 | 894_3185 | A | V | MeF3C | V | W1aa | N | Y | 3Py | I | I | R | R | 4Py | MeY | C |
| 179 | 894_3186 | A | V | MeF3C | V | W1aa | N | 3Py6NH2 | 3Py | I | I | R | R | 4Py | MeY | C |
| 180 | 894_3187 | A | V | MeF3C | V | W1aa | N | 3Py6NH2 | F4OMe | I | I | R | R | 4Py | MeY | C |
| 181 | 894_3188 | A | V | MeF3C | V | W | N | Y | 3Py | I | I | R | R | 4Py | MeY | C |
| 182 | 894_3189 | A | V | MeF3C | V | W | N | 3Py6NH2 | 3Py | I | I | R | R | 4Py | MeY | C |
| 183 | 894_3190 | A | V | MeF3C | V | W | N | 3Py6NH2 | F4OMe | I | I | R | R | 4Py | MeY | C |
| 184 | 894_3191 | A | V | MeF3C | V | W | N | Y | 4Py | I | I | R | R | 4Py | MeY | C |
| 185 | 894_0332 | A | V | MeF3C | V | W1aa | N | 3Py6NH2 | F4OMe | I | I | R | R | F | MeY | C |
| 186 | 894_0333 | A | V | MeF3C | V | W1aa | N | Y | 3Py | I | I | R | R | F | MeY | C |
| 187 | 894_0335 | A | V | MeF3C | V | W1aa | N | Y | F4OMe | I | I | R | R | 4Py | MeY | C |
| 188 | 894_0336 | A | V | MeF3C | V | W1aa | N | Y | F4OMe | I | I | R | R | F3COO | MeY | C |
| 189 | 894_0338 | A | V | MeF3C | V | W1aa | N | 3Py6NH2 | 3Py | I | I | R | R | F | MeY | C |
| 190 | 894_0357 | A | V | MeF3C | V | W | N | 3Py6NH2 | 3Py | I | I | R | R | F | MeY | C |

**[TABLE 2-2-2]**

| Linker | | | | | | | m/z | [M+XH]X+ | SPR |
|---|---|---|---|---|---|---|---|---|---|
| G | de | de | de | ds | ds | KN3 | 1227.7 | 2 | Low signal |
| G | PEG8c | KN3 | | | | | 1181.7 | 3 | Low signal |
| G | KN3 | | | | | | 1055.19 | 3 | Low signal |
| PEG4c | | | | | | | 772.49 | 3 | B |
| PEG4c | | | | | | | 1197.41 | 2 | B |
| PEG4c | | | | | | | 1183.06 | 2 | B |
| PEG4c | | | | | | | 1204.13 | 2 | B |
| PEG4c | | | | | | | 1204.92 | 2 | B |
| PEG4c | | | | | | | 1310.98 | 2 | B |
| PEG4c | | | | | | | 1291.96 | 2 | B |
| PEG4c | | | | | | | 1218.95 | 2 | B |
| PEG4c | | | | | | | 1160.9 | 2 | B |
| G | KN3 | | | | | | 790.94 | 3 | B |
| G | KN3 | | | | | | 1186.25 | 2 | B |
| G | KN3 | | | | | | 800.83 | 3 | B |
| G | KN3 | | | | | | 771.61 | 3 | B |
| G | KN3 | | | | | | 771.54 | 3 | B |
| G | KN3 | | | | | | 781.46 | 3 | B |
| G | KN3 | | | | | | 771.59 | 3 | ND |
| PEG4c | | | | | | | 812.3 | 3 | B |
| PEG4c | | | | | | | 1203.4 | 2 | B |
| PEG4c | | | | | | | 1218.4 | 2 | B |
| PEG4c | | | | | | | 1239.87 | 2 | B |
| PEG4c | | | | | | | 1203.41 | 2 | B |
| PEG4c | | | | | | | 783.43 | 3 | B |

**[TABLE 2-3-1]**

| SEQ ID No. | Name | SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 191 | 894_0358 | A | V | MeF3C | V | W | N | 3Py6NH2 | F4aao | I | I | R | R | F | MeY | C |
| 192 | 894_0359 | A | V | MeF3C | V | W | N | 3Py6NH2 | F4OMe | I | I | R | R | F3COO | MeY | C |
| 193 | 894_0360 | A | V | MeF3C | V | W | N | Y | 3Py | I | I | R | E(PEG8Me) | F | MeY | C |
| 194 | 894_0361 | A | V | MeF3C | V | W | N | Y | 3Py | I | I | R | R | F3COO | MeY | C |
| 195 | 894_0363 | A | V | MeF3C | V | W | N | Y | F4aao | I | I | R | R | F3COO | MeY | C |
| 196 | 894_0364 | A | V | MeF3C | V | W | N | Y | F4aao | I | I | R | R | 4Py | MeY | C |
| 197 | 894_0366 | A | V | MeF3C | V | W | N | Y | F4OMe | I | I | R | E(PEG8Me) | 4Py | MeY | C |
| 198 | 894_0367 | A | V | MeF3C | V | W | N | 3Py6NH2 | 3Py | I | I | R | R | F3COO | MeY | C |
| 199 | 894_0378 | A | V | MeF3C | V | W1aa | N | Y | F4aao | I | I | K(Mecar) | R | F | MeY | C |
| 200 | 894_0379 | A | V | MeF3C | V | W1aa | N | Y | F4aao | I | I | R | K(Mecar) | F | MeY | C |
| 201 | 894_0380 | A | V | MeF3C | V | W1aa | N | Y | F4aao | I | I | QhEt | R | F | MeY | C |
| 202 | 894_0381 | A | V | MeF3C | V | W1aa | N | Y | F4aao | I | I | R | QhEt | F | MeY | C |
| 203 | 894_0382 | A | V | MeF3C | V | W1aa | N | Y | F4aao | I | I | QhEt | QhEt | F | MeY | C |
| 204 | 894_0383 | A | V | MeF3C | V | W1aa | N | Y | F4aao | I | I | Qpipzaa | R | F | MeY | C |
| 205 | 894_0384 | A | V | MeF3C | V | W1aa | N | Y | F4aao | I | I | R | Qpipzaa | F | MeY | C |
| 206 | 894_0385 | A | V | MeF3C | V | W1aa | N | Y | F4aao | I | I | Qpipzaa | Qpipzaa | F | MeY | C |
| 207 | 894_0386 | A | V | MeF3C | V | W1aa | N | Y | F4aao | I | I | A4paa | R | F | MeY | C |
| 208 | 894_0387 | A | V | MeF3C | V | W1aa | N | Y | F4aao | I | I | R | A4paa | F | MeY | C |
| 209 | 894_0388 | A | V | MeF3C | V | W1aa | N | Y | F4aao | I | I | Kmor | R | F | MeY | C |
| 210 | 894_0389 | A | V | MeF3C | V | W1aa | N | Y | F4aao | I | I | R | Kmor | F | MeY | C |
| 211 | 894_0390 | A | V | MeF3C | V | W1aa | N | Y | F4aao | I | I | Kmor | Kmor | F | MeY | C |
| 212 | 894_3197 | A | V | MeF3C | V | W1aa | N | Y | F4aao | I | I | R | R | F | MeY | C |
| 213 | 894_3198 | A | V | MeF3C | V | W1aa | N | Y | F4aao | I | I | R | R | F | MeY | C |
| 214 | 894_3199 | A | V | MeF3C | V | W1aa | N | Y | F4aao | I | I | E(PEG8Me) | R | F | MeY | C |
| 215 | 894_3200 | A | V | MeF | V | MeW | N | Y | Y | I | I | K | R | Y | MeY | C |

**[TABLE 2-3-2]**

| Linker | | | | | | | m/z | [M+XH]X+ | SPR |
|---|---|---|---|---|---|---|---|---|---|
| PEG4c | | | | | | | 1210.95 | 2 | B |
| PEG4c | | | | | | | 1210.92 | 2 | B |
| PEG4c | | | | | | | 1343.52 | 2 | B |
| PEG4c | | | | | | | 1196.41 | 2 | B |
| PEG4c | | | | | | | 1232.94 | 2 | B |
| PEG4c | | | | | | | 1211.43 | 2 | B |
| PEG4c | | | | | | | 1358.69 | 2 | B |
| PEG4c | | | | | | | 1196.42 | 2 | B |
| PEG4c | | | | | | | 1254.96 | 2 | A |
| PEG4c | | | | | | | 1254.97 | 2 | B |
| PEG4c | | | | | | | 1247.94 | 2 | B |
| PEG4c | | | | | | | 1247.96 | 2 | B |
| PEG4c | | | | | | | 1255.88 | 2 | A |
| PEG4c | | | | | | | 1289.48 | 2 | B |
| PEG4c | | | | | | | 1289.47 | 2 | B |
| PEG4c | | | | | | | 893.02 | 3 | Low signal |
| PEG4c | | | | | | | 1267.99 | 2 | A |
| PEG4c | | | | | | | 1267.92 | 2 | B |
| PEG4c | | | | | | | 1260.94 | 2 | A |
| PEG4c | | | | | | | 1260.96 | 2 | A |
| PEG4c | | | | | | | 1281.99 | 2 | A |
| G | de | de | de | ds | ds | K(BCNOCO) | 1052.2 | 3 | A |
| G | ds | ds | ds | ds | ds | K(BCNOCO) | 1010.17 | 3 | A |
| G | K(BCNOCO) | | | | | | 978.11 | 3 | A |
| G | ds | ds | ds | ds | ds | KN3 | 1365.7 | 2 | A |

**[TABLE 2-4-1]**

| SEQ ID No. | Name | SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 216 | 894_0422 | A | V | MeF3C | V | W1aa | N | Y | F4aao | I | I | Q | Kmor | F3COO | MeY | C |
| 217 | 894_0423 | A | V | MeF3C | V | W1aa | N | Y | F4aao | I | I | E(PEG8Me) | Kmor | F3COO | MeY | C |
| 218 | 894_0444 | A | V | MeF3C | V | W7N | N | Y | F4aao | I | I | E(PEG8Me) | R | 4Py | MeY | C |
| 219 | 894_0446 | A | V | MeF3C | V | W1aa | N | Y | F4aao | I | I | E(PEG8Me) | Kmor | 4Py | MeY | C |
| 220 | 894_0447 | A | V | MeF3C | V | W7N | N | Y | F4aao | I | I | E(PEG8Me) | Kmor | F3COO | MeY | C |
| 221 | 894_0450 | A | V | MeF3C | V | W7N | N | Y | F4aao | I | I | E(PEG8Me) | R | E(PEG8Me) | MeY | C |
| 222 | 894_0457 | A | V | MeF3C | V | W1aa | N | 3Py6NH2 | F4aao | I | I | E(PEG8Me) | R | 4Py | MeY | C |
| 223 | 894_0458 | A | V | MeF3C | V | W1aa | N | 3Py6NH2 | F4aao | I | I | E(PEG8Me) | R | F3COO | MeY | C |
| 224 | 894_0459 | A | V | MeF3C | V | W7N | N | 3Py6NH2 | F4aao | I | I | E(PEG8Me) | R | 4Py | MeY | C |
| 225 | 894_0427 | A | V | MeF3C | V | W7N | N | Y | F4aao | I | I | Q | R | F3COO | MeY | C |
| 226 | 894_0439 | A | V | MeF3C | V | W7N | N | Y | F4aao(PEG8Me) | I | I | Q | Q | F3COO(PEG8Me) | MeF4COO(PEG8Me) | C |
| 227 | 894_0440 | A | V | MeF3C | V | W7N | N | Y | F4aao(PEG8Me) | I | I | Q | Q | 4Py | MeF4COO(PEG8Me) | C |
| 228 | 894_0441 | A | V | MeF3C | V | W7N | N | Y | F4OMe | I | I | Q | Q | F3COO(PEG8Me) | MeF4COO(PEG8Me) | C |
| 229 | 894_0442 | A | V | MeF3C | V | W7N | N | Y | F4aao(PEG8Me) | I | I | Q | Q | F3COO(PEG8Me) | MeY | C |
| 230 | 894_0443 | A | V | MeF3C | V | W7N | N | Y | F4aao | I | I | E(PEG8Me) | R | F3COO | MeY | C |
| 231 | 894_0445 | A | V | MeF3C | V | W1aa | N | Y | F4aao | I | I | E(PEG8Me) | R | 4Py | MeY | C |
| 232 | 894_0448 | A | V | MeF3C | V | W7N | N | 3Py6NH2 | F4aao | I | I | E(PEG8Me) | R | E(PEG8Me) | MeY | C |
| 233 | 894_0449 | A | V | MeF3C | V | W7N | N | 3Py6NH2 | F4aao | I | I | E(PEG8Me) | Kmor | F3COO | MeY | C |
| 234 | 894_0451 | A | V | MeF3C | V | W7N | N | Y | F4aao | I | I | E(PEG8Me) | Kmor | E(PEG8Me) | MeY | C |
| 235 | 894_0452 | A | V | MeF3C | V | W7N | N | Y | F4OMe | I | I | E(PEG8Me) | Kmor | E(PEG8Me) | MeY | C |
| 236 | 894_0453 | A | V | MeF3C | V | W7N | N | 3Py6NH2 | F4OMe | I | I | E(PEG8Me) | Kmor | 4Py | MeY | C |
| 237 | 894_0454 | A | V | MeF3C | V | W7N | N | 3Py6NH2 | F4OMe | I | I | E(PEG8Me) | KCOpipzaa | 4Py | MeY | C |
| 238 | 894_0456 | A | V | MeF3C | V | W | N | 3Py6NH2 | F4aao | I | I | E(PEG8Me) | R | F3COO | MeY | C |
| 239 | 894_0460 | A | V | MeF3C | V | W7N | N | 3Py6NH2 | F4aao | I | I | E(PEG8Me) | R | F3COO | MeY | C |
| 240 | 894_0461 | A | V | MeF3C | V | W1aa | N | Y | F4aao | I | I | Kmor | Kmor | F3COO | MeY | C |
| 241 | 894_3201 | A | V | MeF | V | MeW | N | Y | Y | I | I | K | R | Y | MeY | C |

**[TABLE 2-4-2]**

| Linker | | | | | | | m/z | [M+XH]X+ | SPR |
|---|---|---|---|---|---|---|---|---|---|
| PEG4c | | | | | | | 1268.99 | 2 | B |
| PEG4c | | | | | | | 968.5 | 3 | B |
| PEG4c | | | | | | | 921.23 | 3 | B |
| PEG4c | | | | | | | 954.2 | 3 | B |
| PEG4c | | | | | | | 949.49 | 3 | B |
| PEG4c | | | | | | | 1036.68 | 3 | B |
| PEG4c | | | | | | | 940.13 | 3 | B |
| PEG4c | | | | | | | 954.59 | 3 | B |
| PEG4c | | | | | | | 921.24 | 3 | B |
| PEG4c | | | | | | | 813.3 | 3 | A |
| PEG4c | | | | | | | 1178.72 | 3 | B |
| PEG4c | | | | | | | 1042.63 | 3 | B |
| PEG4c | | | | | | | 1042.27 | 3 | B |
| PEG4c | | | | | | | 1047.62 | 3 | B |
| PEG4c | | | | | | | 935.41 | 3 | B |
| PEG4c | | | | | | | 940.09 | 3 | B |
| PEG4c | | | | | | | 1036.58 | 3 | C |
| PEG4c | | | | | | | 949.46 | 3 | B |
| PEG4c | | | | | | | 1050.59 | 3 | C |
| PEG4c | | | | | | | 1035.94 | 3 | B |
| PEG4c | | | | | | | 920.44 | 3 | B |
| PEG4c | | | | | | | 953.81 | 3 | B |
| PEG4c | | | | | | | 935.06 | 3 | B |
| PEG4c | | | | | | | 935.38 | 3 | B |
| PEG4c | | | | | | | 1304.08 | 2 | B |
| G | de | de | de | ds | ds | KN3 | 1428.53 | 2 | B |

### [TABLE 3]

**[TABLE 3]**

| SEQ ID No. | Linker | | | | | | |
|---|---|---|---|---|---|---|---|
| 242 | G | KN3 | | | | | |
| 243 | G | de | de | de | ds | ds | KN3 |
| 244 | G | PEG8c | KN3 | | | | |
| 245 | G | dkCOmeglumine | dkCOmeglumine | dkCOmeglumine | ds | ds | KN3 |
| 246 | G | de | de | de | S | S | C |
| 247 | G | de | de | de | de | de | C |
| 248 | G | ds | ds | ds | ds | ds | |
| 249 | G | de | de | de | ds | ds | KN3 |
| 250 | G | de | de | de | ds | ds | KN3 |
| 251 | G | ds | ds | ds | ds | ds | K(BCNOCO) |
| 252 | G | K(BCNOCO) | | | | | |
| 253 | G | de | de | de | ds | ds | KN3 |

### Example 3

### Evaluation Test of Molecular Interaction Between Transferrin Receptor (hTfR) and Peptide Using Surface Plasmon Resonance (SPR)

The intermolecular interactions of various synthesized peptides with the transferrin receptor (hTfR) by surface plasmon resonance (SPR) of the peptides were tested by the methods described below. Specific test are shown below.

### [SPR Measurement]

NTA sensor chip (Global Life Sciences Technologies Japan K.K.) was inserted into BiacoreT200 (Global Life Sciences Technologies Japan K.K.), then the priming operation was performed three times by using running buffer containing: 10 mM HEPES pH 8.0 (Nacalai Tesque, Inc.), 150 mM NaCl (Nacalai Tesque, Inc.), 0.05% Tween 20 (Nacalai Tesque, Inc.), 0.1% BSA (SIGMA-ALDRICH) and 1.0% DMSO (FUJIFILM Wako Pure Chemical Corporation), and equilibrated at a flow rate of 30 µL/min. 350 mM EDTA solution was reacted at a flow rate of 10 µL/min for 60 seconds, 0.5 mM NiCl₂ solution (Kishida Chemical Co., Ltd.) was reacted at a flow rate of 10 µL/min for 60 seconds, and then the NTA sensor chip was washed with 3 mM EDTA solution (Nacalai Tesque, Inc.) at a flow rate of 10 µL/min for 60 seconds. After mixing 50 µL each of 60 mM EDC solution (Global Life Science Technologies Japan K.K.) and 650 mM NHS solution (Global Life Science Technologies Japan K.K.), the reaction was carried out at a flow rate of 10 µL/min for 420 seconds. 150 µL of 0.5 µM hTfR dimer solution was prepared by dilution with running buffer and reacted at a flow rate of 10 µL/min for 300 seconds to immobilize hTfR on the NTA sensor chip. For the human transferrin receptor (hTfR), recombinant hTfR described in Example 2 of WO2018/124121 was used. After immobilization, capping was performed by reacting with 1.0 M ethanolamine solution (Global Life Sciences Technologies Japan K.K.) at a flow rate of 10 µL/min for 420 seconds. Peptide lysate prepared in DMSO solution to 10 mM was diluted with running buffer to be a final concentration of 10 µM peptide lysate, then peptide solutions of 100 nM, 50 nM, 25 nM, 10 nM, and 5 nM were prepared. Using the above samples, the kinetics of the peptide against hTfR was obtained by SPR measurement.

The kinetic evaluation model was single-cycle kinetics, and curve fitting was performed using Biacore T200 Evaluation Software Version 3.0 (Global Life Science Technologies Japan K.K.). The binding of the peptide to hTfR was evaluated by performing curve fitting on the obtained sensorgram by least squares method and obtaining the KD value. The KD values thus obtained in this way are shown in TABLE 1, denoted A for KD values less than 1 nM, B for KD values between 1 nM and 100 nM, C for KD values between 100 nM and 1 µM, and D for KD values greater than 1 µM. As a result, the synthesized special cyclic peptides were shown to have remarkable binding ability to hTfR.

### Example 4

The following peptides or peptides with linker were synthesized.

### [Example 4-1]

### Synthesis of 894_0263 (SEQ ID NO: 1)

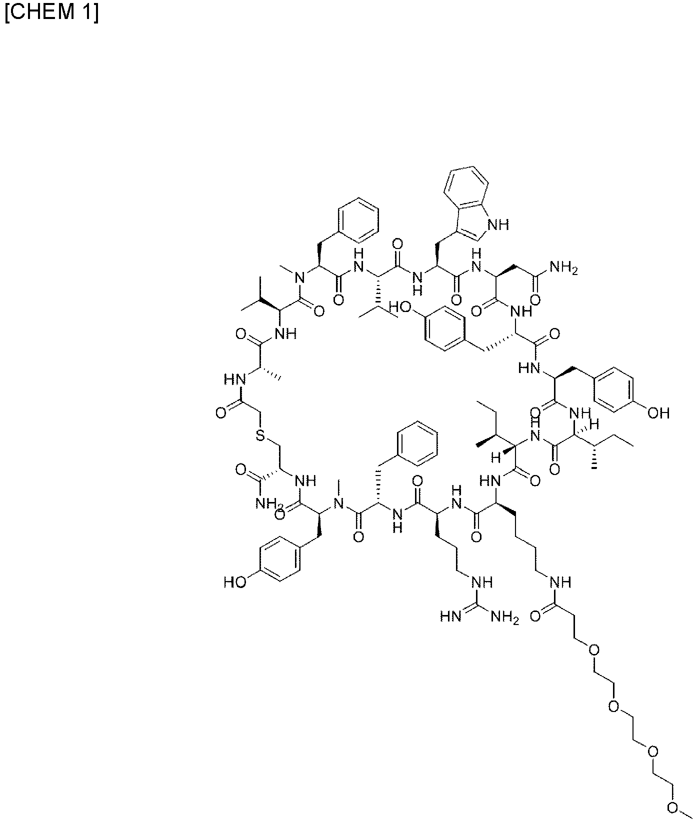

The target peptides were synthesized using Sieber Amide Resin (Watanabe Chemical Industries, Ltd., 0.48 mmol/g, 0.26 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once for 3 min at 90°C in DMF. However, the 1st, 3rd, 4th, 9th, and 10th residues were reacted once at 75°C for 20 min. The 2nd residue was reacted twice at 75°C for 30 min. 11th and 13th residues were reacted twice at 90°C for 3 min. The 12th residue was reacted twice at 50°C for 15 min. The 15th residue was reacted once at 50°C for 15 min. Fmoc removal was performed by reacting with 20% piperidine in DMF at 75°C for 3 min. However, Fmoc removal of the 2nd and 13th residues was carried out by reaction at 25°C for 5 min followed by reaction for 5 min. For the introduction of the chloroacetyl group, the Fmoc group of the α-amino group was removed from the solid-phase resin holding the Fmoc-protected peptide obtained in the previous step by the method described above, and then chloroacetic acid (about 5 equivalents), HATU (about 5 equivalents), and DIEA (about 10 equivalents) were added and the reaction was carried out by shaking in DMF at 25°C for 30 min. For deprotection of the side chains and removal from the solid-phase resin, the resin obtained after the chloroacetyl group introduction step was first washed five times with DMF and three times with methylene chloride, followed by washing with diethyl ether and drying under reduced pressure. Then, to the reaction vessel containing the solid-phase resin, reactant cocktail-A (4 mL, mixture of TFA/H₂O/TIS/DODT in a volume ratio of 92.5/2.5/2.5/2.5) was added, and the vessel was shaken at room temperature for 60 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction, the peptide was dissolved in DMSO to a final concentration of 5 mM based on the mol number of the solid-phase resin, and then 10 equivalents of triethylamine was added and stirred at 25°C for 15 hours. The mixture was then concentrated under reduced pressure using a Savant Explorer SpeedVac. The residue was dissolved in DMSO to 25 mM based on the mol number of the solid-phase resin, and MePEG4c (1.2 equivalents), HATU (1.1 equivalents), and DIEA (3 equivalents) were added and shaken at 25°C for 1 hour.

The resulting reaction mixture was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 13-38% over 3 min, then 38-43% over 8 min, then 43-60% over 1 min; Flow Rate: 120 mL/min).

The purity of the target product was 92.3%, calculated from the area ratio of the LC/MS (UV wavelength 225 nm) chromatogram under analytical conditions.

Analytical Conditions: Retention Time = 5.28 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B conc): 20-60% over 7.15 min, then 60-95% over 0.3 min, then 95-95% over 1.55 min; Flow Rate: 0.5 mL/min.

ESI-MS (+) observed m/z = 1136.17 (M+2H)²⁺

### [Example 4-2]

### Synthesis of 894_0294 (SEQ ID NO: 43)

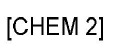

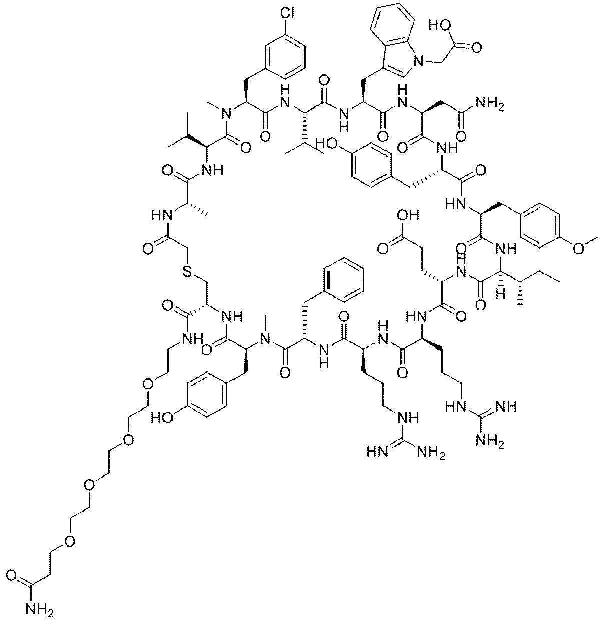

The target peptides were synthesized using Sieber Amide Resin (Watanabe Chemical Industries, Ltd., 0.48 mmol/g, 0.26 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 90°C for 10 min in DMF. However, the 2nd residue was reacted twice at 75°C for 30 min. The 5th, 6th, 7th, 14th, and 16th residues were reacted once at 90°C for 3 min. The 11th and 12th residues were reacted twice at 50°C for 15 min. The 13th residue was reacted twice at 90°C for 3 min. The 15th residue was reacted once at 50°C for 15 min. Fmoc removal was performed by reacting with 10% pyrrolidine in DMF at 90°C for 1 min. However, Fmoc removal of 2nd and 13th residues was carried out by reaction at 25°C for 1 min followed by reaction for 1 min. For the introduction of the chloroacetyl group, the Fmoc group of the α-amino group was removed from the solid-phase resin holding the Fmoc-protected peptide obtained in the previous step by the method described above, and then ClAcOSu (0.025 M), which was prepared by mixing chloroacetic acid (about 5 equivalents), DIPCI (about 5 equivalents), and HOSu (about 5 equivalents) and shaking in DCM for 60 min followed by adding the same volume of DMF as DCM, was added and shaked at 25°C for 60 min. For deprotection of the side chains and removal from the solid-phase resin, the resin obtained after the chloroacetyl group introduction step was first washed five times with DMF and three times with methylene chloride, followed by washing with diethyl ether and drying under reduced pressure. Then, to the reaction vessel containing the solid-phase resin, reactant cocktail-A (4 mL, mixture of TFA/H₂O/TIS/DODT in a volume ratio of 92.5/2.5/2.5/2.5) was added, and the vessel was shaken at room temperature for 60 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO/water (9/1) to a final concentration of 5 mM based on the mol number of the solid-phase resin, and then 10 equivalents of triethylamine was added and stirred at room temperature for 15 hours. The reaction solution was quenched with 15 equivalents of acetic acid, and concentrated under reduced pressure using Genevac HT-12.

The resulting crude product was purified using the following conditions (Column: Waters Xbridge (registered trademark) C18 5µm 50x250 mm; Mobile Phase: A = 0.1% TFA in H₂O, B = 0.1% TFA in MeCN; Temperature: 50°C; Gradient (%B): 7.3-3.3% over 0.1 min, then 3.3-3.3% over 4.9 min, then 3.3-7.3% over 2 min, then 7.3-32.7% over 3 min, then 32.7-37.8% over 15 min, then 37.8-60% over 3 min; Flow Rate: 118-18 mL/min over 0.1 min, then 18-18 mL/min over 4.9 min, then 18-118 mL/min over 2 min, then 118 mL/min).

The purity of the target product was 93.9%, calculated from the area ratio of the LC/MS (UV wavelength 225 nm) chromatogram under analytical conditions.

Analytical Conditions: Retention Time = 4.32 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1225.89 (M+2H)²⁺

### [Example 4-3]

### Synthesis of 894_0298 (SEQ ID NO: 101)

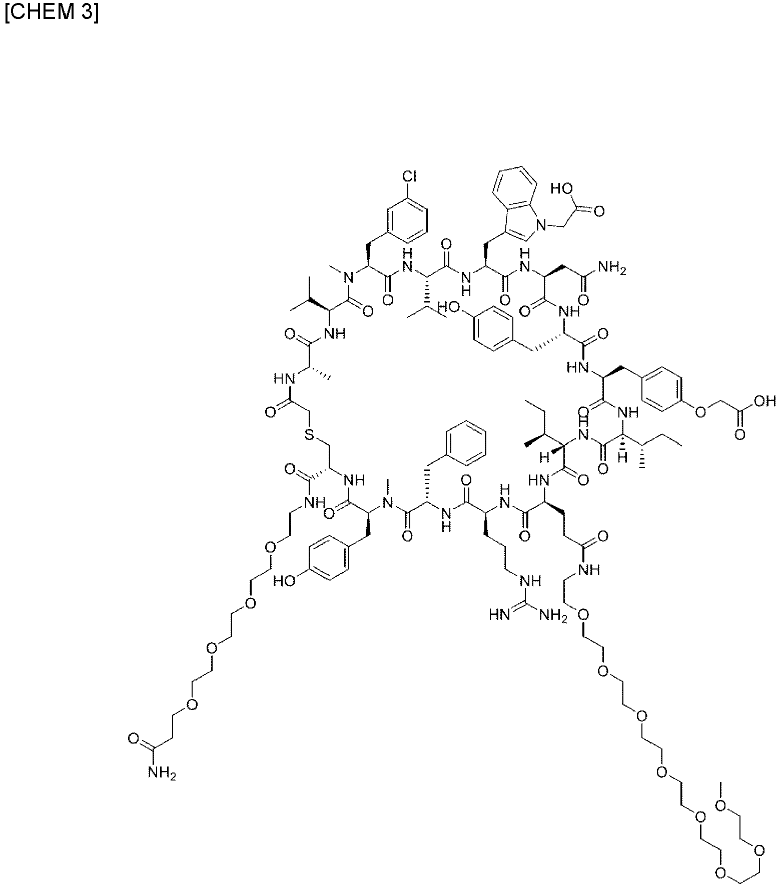

The target peptides were synthesized using Sieber Amide Resin (Watanabe Chemical Industries, Ltd., 0.48 mmol/g, 0.26 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 90°C for 10 min in DMF. However, the 2nd residue was reacted twice at 75°C for 30 min. The 12th residue was reacted twice at 50°C for 15 min. The 13th residue was reacted twice at 90°C for 3 min. The 14th and 16th residues were reacted once at 75°C for 20 min. The 15th residue was reacted once at 40°C for 30 min. Fmoc removal was performed by reacting with 10% pyrrolidine in DMF at 90°C for 1 min. However, Fmoc removal of 2nd, 11th, and 13th residues was carried out by reaction at 75°C for 1 min followed by reaction for 1 min. Fmoc removal of 15th and 16th residues was carried out by reaction at 25°C for 3 min. The solid-phase resin was then shaken with a DMF solution of 5 equivalents of Fmoc-OSu per 1 equivalent of solid-phase resin for 1 hour and washed with DMF. The solid-phase resin was suspended in DCM and 0.2 equivalents of tetrakis(triphenylphosphine)palladium(0) and 10 equivalents of phenylsilane were added to 1 equivalent of resin, shaken for 1 hour, and washed with DCM and DMF. The resin was suspended in DMF and reacted with H-PEG8Me/DIPCI/Oxyma pure (4 equivalents/8 equivalents/4 equivalents) to 1 equivalent of resin at 75°C for 30 min. The introduction of the chloroacetyl group was performed by reacting the solid-phase resin holding the Fmoc-protected peptide obtained in the previous step with a 20% piperidine solution in DMF at 25°C for 5 min twice to remove the Fmoc group of the α-amino group, followed by washing with DMF, then adding ClAcOSu (0.025 M), which was prepared by mixing chloroacetic acid (about 5 equivalents), DIPCI (about 5 equivalents), and HOSu (about 5 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed five times with DMF and three times with methylene chloride, followed by washing with diethyl ether and drying under reduced pressure. Then, to the reaction vessel containing the solid-phase resin, reactant cocktail-A (4 mL, mixture of TFA/H2O/TIS/DODT in a volume ratio of 92.5/2.5/2.5/2.5) was added, and the vessel was shaken at room temperature for 60 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO/water (9/1) to a final concentration of 5 mM based on the mol number of the solid-phase resin, and then 10 equivalents of triethylamine was added and stirred at room temperature for 15 hours. The reaction solution was quenched with acetic acid, and concentrated under reduced pressure using Genevac HT-12.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 11-36% over 3 min, then 36-41% over 8 min, then 41-60% over 1 min; Flow Rate: 120 mL/min).

The purity of the target product was 92.7%, calculated from the area ratio of the LC/MS (UV wavelength 225 nm) chromatogram under analytical conditions.

Analytical Conditions: Retention Time = 5.22 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B conc): 20-60% over 7.15 min, then 60-95% over 0.3 min, then 95-95% over 1.55 min; Flow Rate: 0.5 mL/min.

ESI-MS (+) observed m/z = 939.99 (M+3H)³⁺

### [Example 4-4]

### Synthesis of 894_0301 (SEQ ID NO: 103)

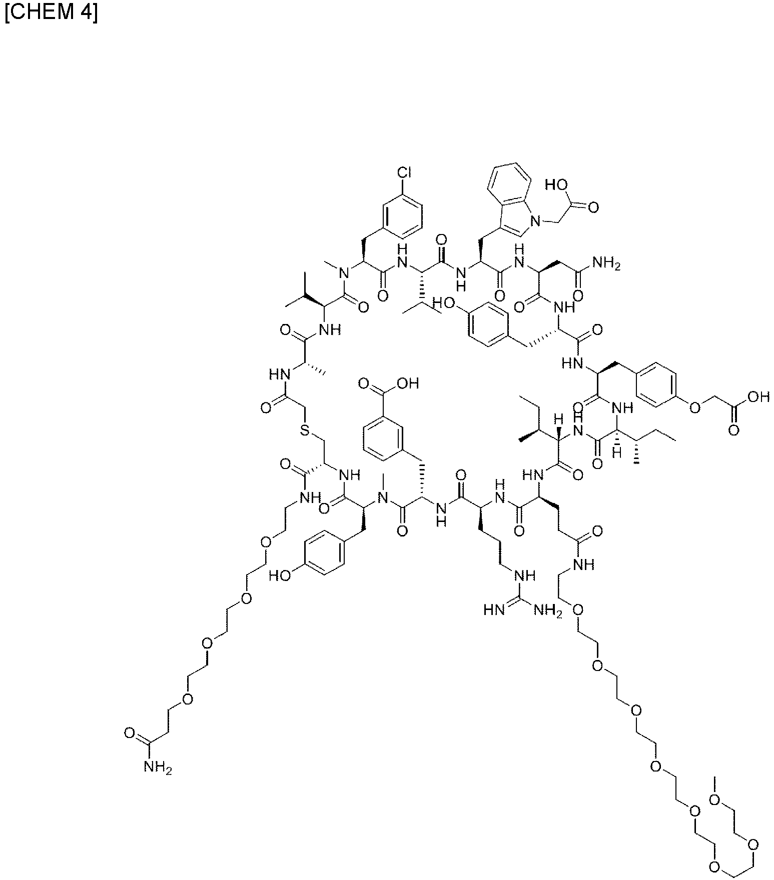

The target peptides were synthesized using Sieber Amide Resin (Watanabe Chemical Industries, Ltd., 0.48 mmol/g, 0.26 g), starting with the removal of the Fmoc group by the general method described above. Liberty Blue HT (CEM Inc.) was used as the solid-phase synthesizer, and the synthesis was performed according to the manufacturer's manual. The peptides were synthesized in the same manner as 894_0298. For the introduction of each residue, Fmoc-AA/DIPCI/Oxyma Pure (4.2 equivalents/8 equivalents/4 equivalents) was used per 1 equivalent of resin, and the reaction was carried out once at 90°C for 10 min in DMF. However, the 2nd residue was reacted twice at 75°C for 30 min. The 12th residue was reacted twice at 50°C for 15 min. The 13th residue was reacted twice at 90°C for 3 min. The 14th and 16th residues were reacted once at 75°C for 20 min. The 15th residue was reacted once at 40°C for 30 min. Fmoc removal was performed by reacting with 10% pyrrolidine in DMF at 90°C for 1 min. However, Fmoc removal of 2nd, 11th, and 13th residues was carried out by reaction at 75°C for 1 min followed by reaction for 1 min. Fmoc removal of 15th and 16th residues was carried out by reaction at 25°C for 3 min. The solid-phase resin was then shaken with a DMF solution of 5 equivalents of Fmoc-OSu per 1 equivalent of solid-phase resin for 1 hour and washed with DMF. The solid-phase resin was suspended in DCM and 0.2 equivalents of tetrakis(triphenylphosphine)palladium(0) and 10 equivalents of phenylsilane were added to 1 equivalent of resin, shaken for 1 hour, and washed with DCM and DMF. The resin was suspended in DMF and reacted with H-PEG8Me/DIPCI/Oxyma pure (4 equivalents/8 equivalents/4 equivalents) to 1 equivalent of resin at 75°C for 30 min. The introduction of the chloroacetyl group was performed by reacting the solid-phase resin holding the Fmoc-protected peptide obtained in the previous step with a 20% piperidine solution in DMF at 25°C for 5 min twice to remove the Fmoc group of the α-amino group, followed by washing with DMF, then adding ClAcOSu (0.025 M), which was prepared by mixing chloroacetic acid (about 5 equivalents), DIPCI (about 5 equivalents), and HOSu (about 5 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed five times with DMF and three times with methylene chloride, followed by washing with diethyl ether and drying under reduced pressure. Then, to the reaction vessel containing the solid-phase resin, reactant cocktail-A (4 mL, mixture of TFA/H2O/TIS/DODT in a volume ratio of 92.5/2.5/2.5/2.5) was added, and the vessel was shaken at room temperature for 60 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO/water (9/1) to a final concentration of 5 mM based on the mol number of the solid-phase resin, and then 10 equivalents of triethylamine was added and stirred at room temperature for 15 hours. The reaction solution was quenched with acetic acid, and concentrated under reduced pressure using Genevac HT-12.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 11-36% over 3 min, then 36-41% over 8 min, then 41-60% over 1 min; Flow Rate: 120 mL/min).

The purity of the target product was 88.1%, calculated from the area ratio of the LC/MS (UV wavelength 225 nm) chromatogram under analytical conditions.

Analytical Conditions: Retention Time = 5.28 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B conc): 20-60% over 7.15 min, then 60-95% over 0.3 min, then 95-95% over 1.55 min; Flow Rate: 0.5 mL/min.

ESI-MS (+) observed m/z = 1431.19 (M+2H)²⁺

### [Example 4-5]

### Synthesis of 894_0306 (SEQ ID NO: 48)

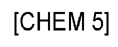

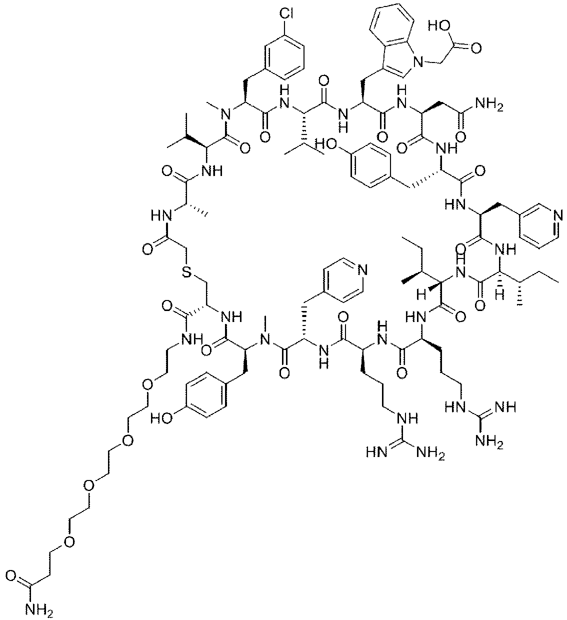

The target peptides were synthesized using Sieber Amide Resin (Watanabe Chemical Industries, Ltd., 0.48 mmol/g, 0.13 g), starting with the removal of the Fmoc group by the general method described above. Liberty Prime (CEM Inc.) was used as the solid-phase synthesizer, and the synthesis was performed according to the manufacturer's manual. For the introduction of each residue, Fmoc-AA/DIPCI/Oxyma Pure (4.2 equivalents/16 equivalents/6 equivalents) was used per 1 equivalent of resin, and the reaction was carried out once at 105°C for 1.5 min in DMF. However, the 2nd residue was reacted twice at 75°C for 30 min. The 11th and 12th residues were reacted twice at 50°C for 15 min. The 13th residue was reacted twice at 90°C for 10 min. The 15th residue was reacted once at 50°C for 15 min. Fmoc removal was performed by reacting with a DMF solution of 83 mM Oxyma pure containing 4% pyrrolidine at 110°C for 1 min. However, Fmoc removal of 2nd and 13th residues was carried out by reaction with a DMF solution of 10% pyrrolidine at 25°C for 1.5 min followed by reaction for 1.5 min. The introduction of the chloroacetyl group was performed by removing the Fmoc group of the α-amino group from the solid-phase resin holding the Fmoc-protected peptide obtained in the previous step, followed by adding chloroacetic acid (about 5 equivalents), HATU (about 5 equivalents), and DIEA (about 10 equivalents) and shaking in DMF at 25°C for 30 min. For deprotection of the side chains and removal from the solid-phase resin, the resin obtained after the chloroacetyl group introduction step was first washed with DMF and methylene chloride, followed by washing with diethyl ether and drying under reduced pressure. Then, to the reaction vessel containing the solid-phase resin, reactant cocktail-A (3 mL, mixture of TFA/H2O/TIS/DODT in a volume ratio of 92.5/2.5/2.5/2.5) was added, and the vessel was shaken at room temperature for 60 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO to a final concentration of 3 mM based on the mol number of the solid-phase resin, and then 10 equivalents of triethylamine was added and stirred at room temperature overnight, then the reaction solution was quenched with acetic acid. The reaction solution was then concentrated under reduced pressure using Genevac EZ-II elite.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 5-5% over 2 min, then 5-27% over 1 min, then 27-32% over 8 min, then 32-60% over 1 min; Flow Rate: 20 mL/min over 1 min, then 20mL/min-120mL/min over 1 min, then 120mL/min).

The purity of the target product was 86.7%, calculated from the area ratio of the LC/MS (UV wavelength 225 nm) chromatogram under analytical conditions.

Analytical Conditions: Retention Time = 3.08 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1203.89 (M+2H)²⁺

### [Example 4-6]

### Synthesis of 894_0392 (SEQ ID NO: 109)

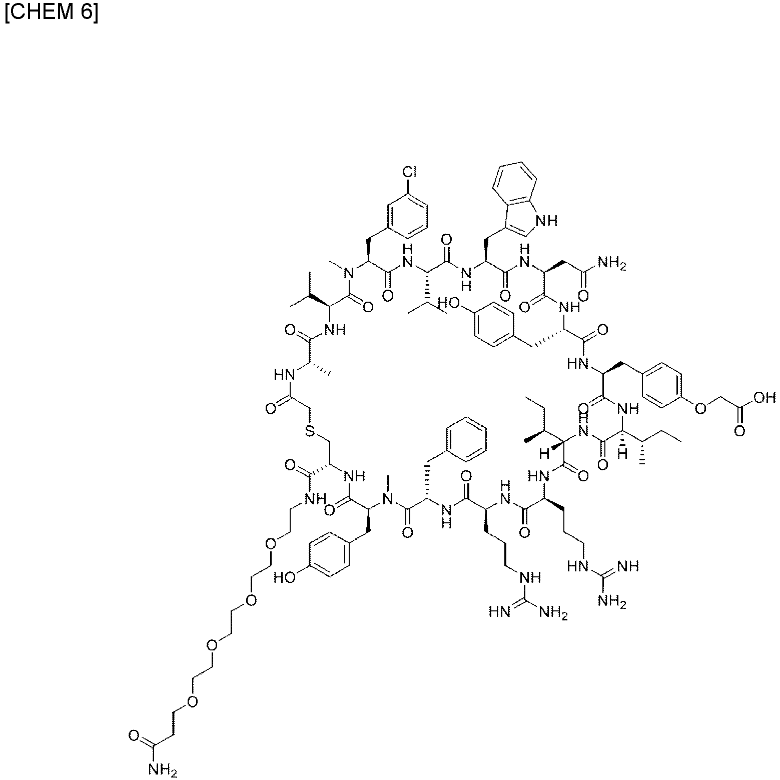

The target peptides were synthesized using Sieber Amide Resin (Watanabe Chemical Industries, Ltd., 0.48 mmol/g, 0.10 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (5.3 equivalents/15 equivalents/7.5 equivalents) was used per 1 equivalent of resin, and the reaction was carried out once at 90°C for 3 min in DMF. However, the 2nd residue was reacted twice at 75°C for 30 min. The 11th and 12th residues were reacted twice at 50°C for 15 min. The 13th residue was reacted twice at 90°C for 10 min. The 15th residue was reacted once at 50°C for 15 min. Fmoc removal was performed by reacting with a DMF solution containing 10% pyrrolidine at 90°C for 1 min. However, Fmoc removal of 2nd and 13th residues was carried out by reaction at 25°C for 1 min followed by reaction for 1 min. The introduction of the chloroacetyl group was performed by removing the Fmoc group of the α-amino group from the solid-phase resin holding the Fmoc-protected peptide obtained in the previous step, followed by adding chloroacetic acid (about 5 equivalents), HATU (about 5 equivalents), and DIEA (about 10 equivalents) and shaking in DMF at 25°C for 30 min. For deprotection of the side chains and removal from the solid-phase resin, the resin obtained after the chloroacetyl group introduction step was first washed with DMF and methylene chloride, followed by washing with diethyl ether and drying under reduced pressure. Then, to the reaction vessel containing the solid-phase resin, reactant cocktail-A (2.5 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO/water (9/1) to a final concentration of 5 mM based on the mol number of the solid-phase resin, and then 10 equivalents of triethylamine was added and stirred at 25°C for 15 hours, then the reaction solution was quenched with acetic acid. The reaction solution was then concentrated under reduced pressure using Genevac EZ-II elite.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 11-11% over2 min, then 11-36% over 1 min, then 36-41% over 8 min, then 41-60% over 1 min; Flow Rate: 20 mL/min over 1 min, then 20mL/min-120mL/min over 1 min, then 120mL/min).

The purity of the target product was 94.9%, calculated from the area ratio of the LC/MS (UV wavelength 225 nm) chromatogram under analytical conditions.

Analytical Conditions: Retention Time = 4.46 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1210.97 (M+2H)²⁺

### [Example 4-7]

### Synthesis of 894_0350 (SEQ ID NO: 82)

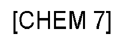

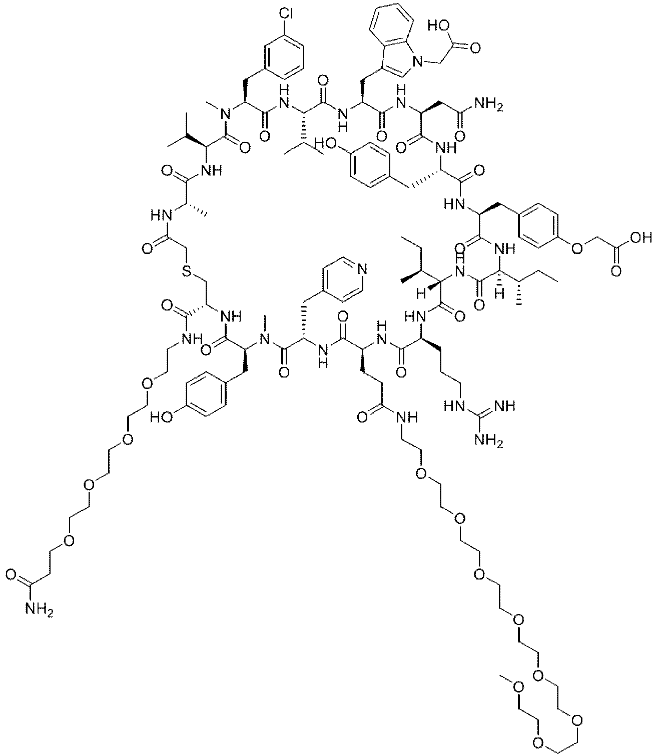

The target peptides were synthesized using Sieber Amide Resin (Watanabe Chemical Industries, Ltd., 0.48 mmol/g, 0.13 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (4.2 equivalents/12 equivalents/6 equivalents) was used per 1 equivalent of resin, and the reaction was carried out once at 90°C for 3 min in DMF. However, the 2nd residue was reacted twice at 75°C for 30 min. The 11th residue was reacted twice at 50°C for 15 min. The 13th residue was reacted twice at 90°C for 3 min. The 14th residue and 16th residue were reacted once at 90°C for 3 min. The 15th residue was reacted once at 50°C for 15 min. Fmoc removal was performed by reacting with a DMF solution containing 10% pyrrolidine at 25°C for 1 min, followed by reaction for 1 min. However, Fmoc removal of 13th, 14th, 15th, and 16th residues was carried out by reaction at 90°C for 1 min. The introduction of the chloroacetyl group was performed by removing the Fmoc group of the α-amino group from the solid-phase resin holding the Fmoc-protected peptide obtained in the previous step, then adding ClAcOSu (0.025 M), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (3 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in acetonitrile/water (1/1) to a final concentration of 5 mM based on the mol number of the solid-phase resin, and then 10 equivalents of triethylamine were added and stirred at room temperature for 2 hours. The reaction solution was quenched with acetic acid and concentrated under reduced pressure using Genevac HT-12. The resulting residue was dissolved in DMF so that the final concentration of peptide was 25 mM based on the mol number of the solid-phase resin, and then H-PEG8Me/HATU/DIEA (1.1 equivalents/1.2 equivalents/5 equivalents) was added, and shaken at room temperature for 1 hour, and then quenched with acetic acid. 0.1 equivalent of tetrakis(triphenylphosphine)palladium (0) and 10 equivalents of phenylsilane are added to the reaction mixture, and the reaction solution was shaken for 1 hour. Diisopropyl ether was added to the reaction solution, the precipitated solid was centrifuged (9000 rpm, 2 min), and the solution was decanted.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 7-7% over 2 min, then 7-32% over 1 min, then 32-37% over 8 min, then 37-60% over 1 min; Flow Rate: 20-20 mL/min over 1 min, then 20-120mL/min over 1 min, then 120mL/min).

The purity of the target product was 94.6%, calculated from the area ratio of the LC/MS (UV wavelength 225 nm) chromatogram under analytical conditions.

Analytical Conditions: Retention Time = 4.71 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B conc): 20-60% over 7.15 min, then 60-95% over 0.3 min, then 95-95% over 1.55 min; Flow Rate: 0.5 mL/min.

ESI-MS (+) observed m/z = 1409.63 (M+2H)²⁺

### [Example 4-8]

### Synthesis of 894_0356 (SEQ ID NO: 88)

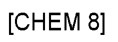

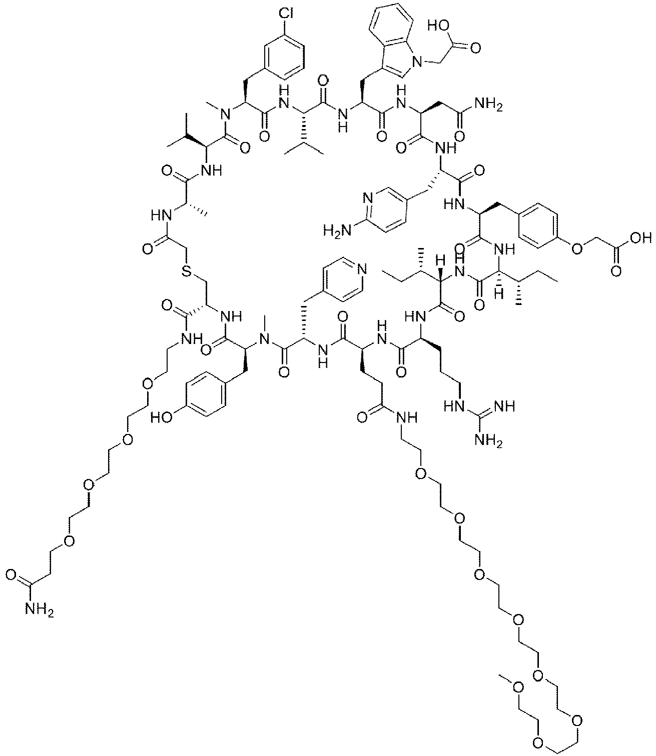

The target peptides were synthesized using Sieber Amide Resin (Watanabe Chemical Industries, Ltd., 0.48 mmol/g, 0.13 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (4.2 equivalents/12 equivalents/6 equivalents) was used per 1 equivalent of resin, and the reaction was carried out once at 90°C for 3 min in DMF. However, the 2nd residue was reacted twice at 75°C for 30 min. The 3rd residue and 9th residue were reacted once at 90°C for 10 min. The 11th residue was reacted twice at 50°C for 15 min. The 13th residue was reacted twice at 90°C for 3 min. The 15th residue was reacted once at 50°C for 15 min. Fmoc removal was performed by reacting with a DMF solution containing 10% pyrrolidine at 25°C for 1 min, followed by reaction for 1 min. However, Fmoc removal of 2nd, 12th, and 13th residues was carried out by reaction at 90°C for 1 min. The solid-phase resin was then shaken with a DCM solution of 5 equivalents of Fmoc-OSu per 1 equivalent of solid-phase resin for 1 hour and washed with DMF. The solid-phase resin was suspended in DCM and 0.2 equivalents of tetrakis(triphenylphosphine)palladium(0), 1 equivalent of triphenylphosphine, and 10 equivalents of triphenylsilane were added to 1 equivalent of resin, shaken for 1 hour, and washed with DCM and DMF. The resin was suspended in DMF and reacted with H-PEG8Me/DIPCI/Oxyma pure (4.2 equivalents/8 equivalents/4 equivalents) to 1 equivalent of resin at 75°C for 10 min. The introduction of the chloroacetyl group was performed by removing the Fmoc group of the α-amino group of the solid-phase resin holding the Fmoc-protected peptide obtained in the previous step by reacting with 10% pyrrolidine solution in DMF at 25°C for 10 min, followed by washing with DMF, then adding ClAcOSu (0.013 M), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (3 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO to a final concentration of 5 mM based on the mol number of the solid-phase resin, and then 10 equivalents of triethylamine were added and stirred at room temperature for 15 hours. The reaction solution was quenched with acetic acid and concentrated under reduced pressure using Genevac HT-12.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 5-29% over 3 min, then 29-34% over 8 min, then 34-60% over 1 min; Flow Rate: 120 mL/min).

The purity of the target product was 89.2%, calculated from the area ratio of the LC/MS (UV wavelength 225 nm) chromatogram under analytical conditions.

Analytical Conditions: Retention Time = 3.44 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B conc): 20-60% over 7.15 min, then 60-95% over 0.3 min, then 95-95% over 1.55 min; Flow Rate: 0.5 mL/min.

ESI-MS (+) observed m/z = 940.08 (M+3H)³⁺

### [Example 4-9]

### Synthesis of 894_0324 (SEQ ID NO: 65)

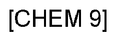

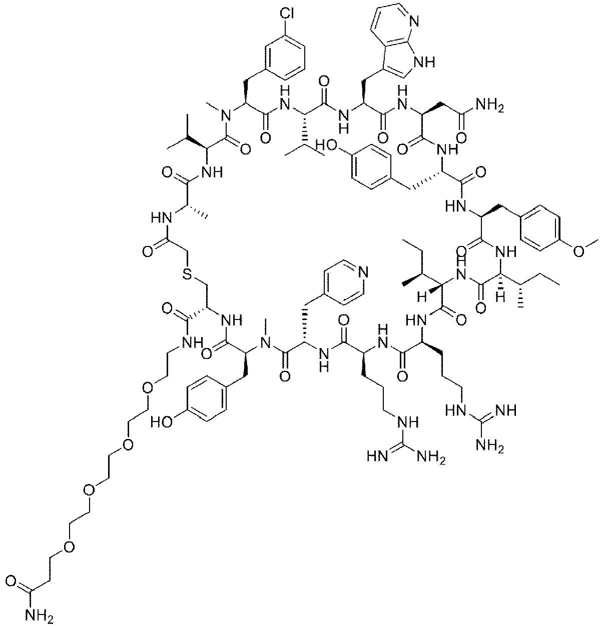

The target peptides were synthesized using Sieber Amide Resin (Watanabe Chemical Industries, Ltd., 0.48 mmol/g, 0.10 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (5.3 equivalents/15 equivalents/7.5 equivalents) was used per 1 equivalent of resin, and the reaction was carried out once at 90°C for 3 min in DMF. However, the 2nd residue was reacted twice at 75°C for 30 min. The 11th residue and 12th residue were reacted twice at 50°C for 15 min. The 13th residue was reacted twice at 90°C for 3 min. The 15th residue was reacted once at 50°C for 15 min. Fmoc removal was performed by reacting with a DMF solution containing 10% pyrrolidine at 90°C for 1 min. However, Fmoc removal of 2nd and 13th residues was carried out by reaction at 25°C for 1 min, followed by reaction for 1 min. The introduction of the chloroacetyl group was performed by removing the Fmoc group of the α-amino group of the solid-phase resin holding the Fmoc-protected peptide obtained in the previous step by the method described above, and then adding ClAcOSu (0.010 M), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed with DMF and methylene chloride, followed by washing with diethyl ether and drying under reduced pressure. Then, to the reaction vessel containing the solid-phase resin, reactant cocktail-A (3 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl isopropylether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO/water (9/1) to a final concentration of 5 mM based on the mol number of the solid-phase resin, 10 equivalents of triethylamine were added and stirred at 40°C for 4 hours, and then hydrazine monohydrate was added (to 2% of DMSO). The reaction solution was concentrated under reduced pressure using Genevac EZ-II elite.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 5-26% over 3 min, then 26-31% over 8 min, then 31-60% over 1 min; Flow Rate: 120 mL/min).

The purity of the target product was 84.5%, calculated from the area ratio of the LC/MS (UV wavelength 225 nm) chromatogram under analytical conditions.

Analytical Conditions: Retention Time = 5.67 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 5-45% over 7.15 min, then 45-95% over 0.3 min, then 95-95% over 1.55 min; Flow Rate: 0.5 mL/min.

ESI-MS (+) observed m/z = 793.86 (M+3H)³⁺

### [Example 4-10]

### Synthesis of 894_0416 (SEQ ID NO: 131)

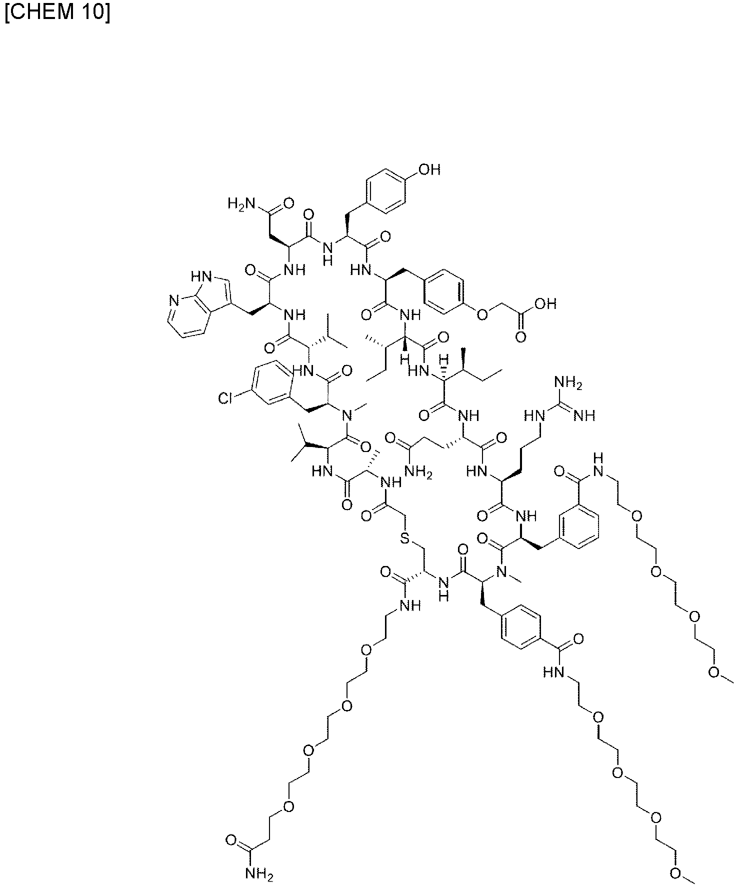

The target peptides were synthesized using Sieber Amide Resin (Watanabe Chemical Industries, Ltd., 0.48 mmol/g, 0.10 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (5.3 equivalents/15 equivalents/7.5 equivalents) was used per 1 equivalent of resin, and the reaction was carried out once at 90°C for 3 min in DMF. However, the 2nd residue was reacted twice at 75°C for 30 min. The 12th residue was reacted twice at 50°C for 15 min. The 13th residue was reacted twice at 90°C for 10 min. The 15th residue was reacted once at 50°C for 15 min. Fmoc removal was performed by reacting with a DMF solution containing 10% pyrrolidine at 90°C for 1 min. However, Fmoc removal of 2nd residue and 13th residue were carried out by reacting with a DMF solution containing 10% pyrrolidine at 25°C for 1 min, followed by reacting for 1 min. The introduction of the chloroacetyl group was performed by removing the Fmoc group of the α-amino group of the solid-phase resin holding the Fmoc-protected peptide obtained in the previous step by the method described above, and then adding ClAcOSu (0.013 M), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (3 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in 5% hydrous DMSO to a final concentration of 5 mM based on the mol number of the solid-phase resin, and then 5 equivalents of triethylamine was added and stirred at 25°C for 2 hours. The reaction solution was concentrated under reduced pressure using Genevac HT-12. The resulting residue was dissolved in DMF to 25 mM based on the mol number of solid-phase resin, then 4.2 equivalents of H-PEG4Me, 4 equivalents of HATU, and 8 equivalents of DIEA were added, and the reaction was carried out at 25°C for 60 min, followed by quenching with acetic acid. 0.1 equivalent of tetrakis(triphenylphosphine)palladium(0) and 10 equivalents of phenylsilane were added to the reaction mixture, and shaken for 1 hour. Diisopropyl ether was added to the reaction solution and the precipitated solid was centrifuged (9000 rpm, 2 min) and the solution was decanted.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 5-30% over 3 min, then 30-35% over 8 min, then 35-60% over 1 min; Flow Rate: 120 mL/min).

The purity of the target product was 85.7%, calculated from the area ratio of the LC/MS (UV wavelength 225 nm) chromatogram under analytical conditions.

Analytical Conditions: Retention Time = 4.26 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1422.62 (M+2H)²⁺

### [Example 4-11]

### Synthesis of 894_0367 (SEQ ID NO: 198)

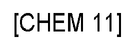

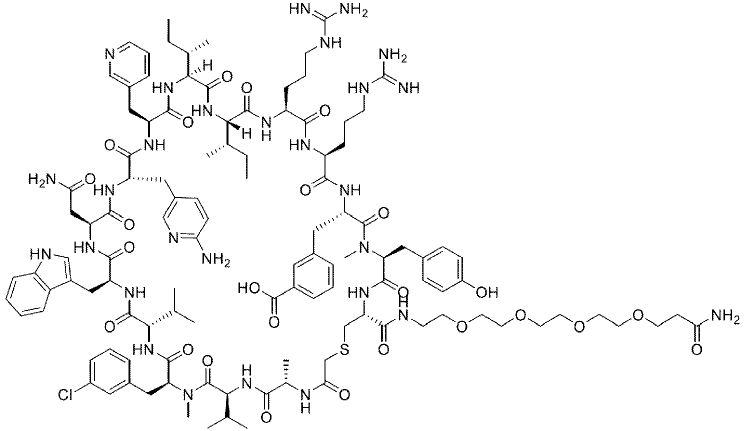

The target peptides were synthesized using Sieber Amide Resin (Watanabe Chemical Industries, Ltd., 0.48 mmol/g, 0.13 g), starting with the removal of the Fmoc group by the general method described above. Liberty Prime (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 105°C for 1.5 min in DMF. However, the 2nd residue was reacted twice at 75°C for 30 min. The 11th residue and 12th residue were reacted twice at 50°C for 15 min. The 13th residue was reacted twice at 90°C for 10 min. The 15th residue was reacted once at 50°C for 15 min. Fmoc removal was performed by reacting with a DMF solution of 83 mM Oxyma pure containing 4% piperidine at 110°C for 1 min. However, Fmoc removal of 2nd residue and 13th residue were carried out by reacting with a DMF solution containing 10% piperidine at 25°C for 1.5 min, followed by reacting for 1.5 min. The introduction of the chloroacetyl group was performed by removing the Fmoc group of the α-amino group of the solid-phase resin holding the Fmoc-protected peptide obtained in the previous step by the method described above, and then adding chloroacetic acid (about 5 equivalents), HATU (about 5 equivalents), and DIEA (about 10 equivalents) and shaking in DCM at 25°C for 30 min. For deprotection of the side chains and removal from the solid-phase resin, the resin obtained after the chloroacetyl group introduction step was first washed with DMF and methylene chloride, followed by washing with diethyl ether and drying under reduced pressure. Then, to the reaction vessel containing the solid-phase resin, reactant cocktail-A (4 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in MeCN/water (1/1) to a final concentration of 2.5 mM based on the mol number of the solid-phase resin, and then 10 equivalents of triethylamine was added and stirred at 25°C for 8 hours. The reaction solution was concentrated under reduced pressure using Genevac EZ-II elite.

The resulting crude product was purified using the following conditions (Column: Waters Xbridge (registered trademark) C18 5µm 50x150 mm; Mobile Phase: A = 0.1% TFA in H₂O, B = 0.1% TFA in MeCN; Temperature: 40°C; Gradient (%B): 5-26% over 3 min, then 26-31% over 8 min, then 31-60% over 1 min; Flow Rate: 120 mL/min).

The purity of the target product was 82.9%, calculated from the area ratio of the LC/MS (UV wavelength 225 nm) chromatogram under analytical conditions.

Analytical Conditions: Retention Time = 3.16 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B conc): 20-60% over 7.15 min, then 60-95% over 0.3 min, then 95-95% over 1.55 min; Flow Rate: 0.5 mL/min.

ESI-MS (+) observed m/z = 1196.42 (M+2H)²⁺

### [Example 4-12]

### Synthesis of 894_0442 (SEQ ID NO: 229)

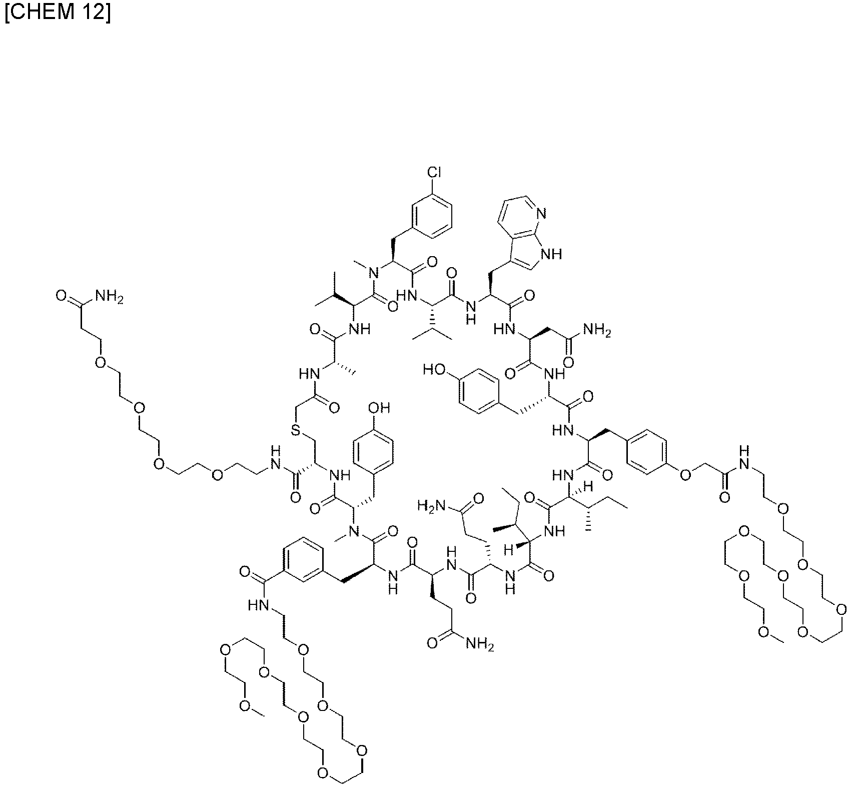

The target peptides were synthesized using Sieber Amide Resin (Watanabe Chemical Industries, Ltd., 0.48 mmol/g, 0.10 g), starting with the removal of the Fmoc group by the general method described above. Liberty PRIME (CEM Inc.) was used as the solid-phase synthesizer, and the synthesis was performed according to the manufacturer's manual. For the introduction of each residue, Fmoc-AA/DIPCI/Oxyma Pure (5.3 equivalents/20 equivalents/7.5 equivalents) was used per 1 equivalent of resin, and the reaction was carried out once at 105°C for 90 seconds in DMF. However, the 2nd residue was reacted twice at 75°C for 30 min. The 13th residue was reacted twice at 90°C for 10 min. The 15th residue was reacted once at 50°C for 15 min. Fmoc removal was performed by reacting with a DMF solution of 83 mM Oxyma pure containing 4% pyrrolidine at 110°C for 1 min. However, Fmoc removal of 2nd residue and 13th residue were carried out by reacting with a DMF solution containing 10% pyrrolidine at room temperature for 90 seconds, followed by reacting for 90 seconds. The introduction of the chloroacetyl group was performed by removing the Fmoc group of the α-amino group of the solid-phase resin holding the Fmoc-protected peptide obtained in the previous step by the method described above, and then adding ClAcOSu (0.013 M), which was prepared by mixing chloroacetic acid (about 5 equivalents), DIPCI (about 5 equivalents), and HOSu (about 5 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (2 mL, mixture of TFA/H2O/TIS/DODT in a volume ratio of 92.5/2.5/2.5/2.5) was added, and the vessel was shaken at room temperature for 10 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO to a final concentration of 5 mM based on the mol number of the solid-phase resin, and then 10 equivalents of triethylamine was added and stirred at room temperature for 15 hours. The reaction solution was concentrated under reduced pressure using Genevac EZ-II elite. The resulting residue was dissolved in DMF to 25 mM based on the mol number of solid-phase resin, then 3.2 equivalents of H-PEG8Me, 2 equivalents of PyAOP, and 6 equivalents of DIEA were added and the reaction was carried out at room temperature for 30 min.

The resulting reaction product was purified using the following conditions (Column: Waters Xbridge (registered trademark) C18 5µm 30x150 mm; Mobile Phase: A = 0.1% TFA in H₂O, B = 0.1% TFA in MeCN; Temperature: 50°C; Gradient (%B): 9-9% over 2 min, then 9-34% over 3 min, then 34-39% over 8 min, then 34-60% over 1 min; Flow Rate: 45 mL/min).

The purity of the target product was 95.9%, calculated from the area ratio of the LC/MS (UV wavelength 225 nm) chromatogram under analytical conditions.

Analytical Conditions: Retention Time = 5.02 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1047.62 (M+3H)³⁺

### [Example 4-13]

### Synthesis of 894_0461 (SEQ ID NO: 240)

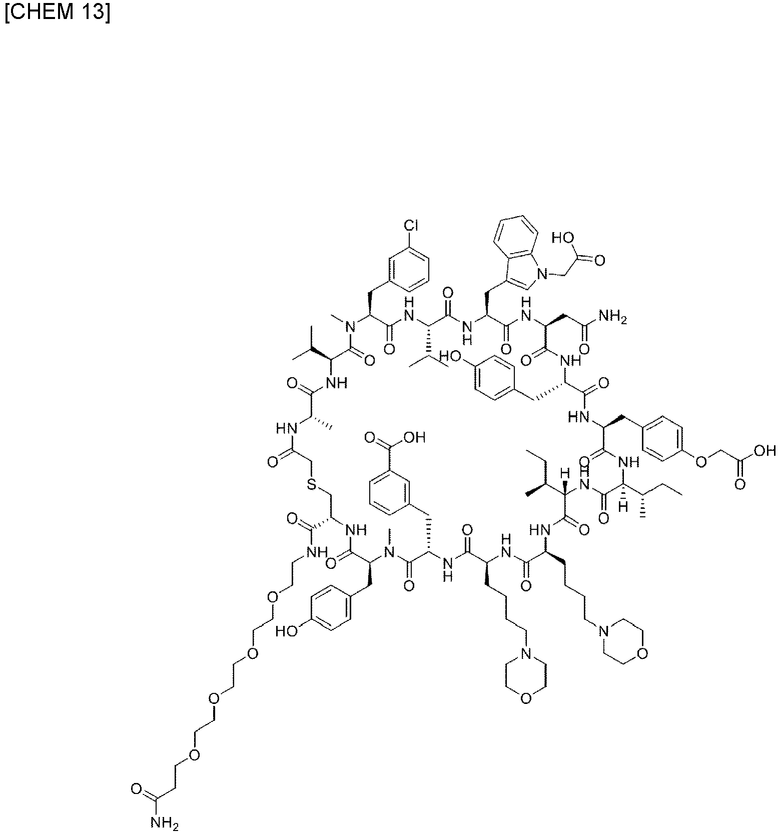

The target peptides were synthesized using Sieber Amide Resin (Watanabe Chemical Industries, Ltd., 0.48 mmol/g, 0.21 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/DIPCI/Oxyma Pure (5.3 equivalents/10 equivalents/5 equivalents) was used per 1 equivalent of resin, and the reaction was carried out once at 90°C for 10 min in DMF. However, the 2nd residue was reacted twice at 90°C for 10 min. The 11th residue and 12th residue were reacted once at 50°C for 15 min. The 13th residue was reacted twice at 90°C for 10 min. The 15th residue was reacted once at 50°C for 15 min. Fmoc removal was performed by reacting with a DMF solution containing 10% pyrrolidine at 75°C for 3 min. However, Fmoc removal of 2nd residue and 13th residue were carried out by reacting at 25°C for 5 min, followed by reacting for 5 min. The introduction of the chloroacetyl group was performed by removing the Fmoc group of the α-amino group of the solid-phase resin holding the Fmoc-protected peptide obtained in the previous step by the method described above, and then adding ClAcOSu (0.020 M), which was prepared by mixing chloroacetic acid (about 5 equivalents), DIPCI (about 5 equivalents), and HOSu (about 5 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed with DMF and methylene chloride, followed by washing with diethyl ether and drying under reduced pressure. Then, to the reaction vessel containing the solid-phase resin, reactant cocktail-A (4.0 mL, mixture of TFA/H2O/TIS/DODT in a volume ratio of 92.5/2.5/2.5/2.5) was added, and the vessel was shaken at room temperature for 60 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in DMSO/water (9/1) to a final concentration of 5 mM based on the mol number of the solid-phase resin, and then 10 equivalents of triethylamine was added and stirred at 25°C for 15 hours, and quenched with acetic acid. The reaction solution was concentrated under reduced pressure using Genevac HT-12.

The resulting crude product was purified using the following conditions (Column: Waters Xbridge (registered trademark) C18 5µm 30x150 mm; Mobile Phase: A = 0.1% TFA in H₂O, B = 0.1% TFA in MeCN; Temperature: 50°C; Gradient (%B): 11-11% over 2 min, then 11-36% over 3 min, then 36-41% over 8 min, then 41-60% over 1 min; Flow Rate: 45 mL/min).

The purity of the target product was 92.3%, calculated from the area ratio of the LC/MS (UV wavelength 225 nm) chromatogram under analytical conditions.

Analytical Conditions: Retention Time = 4.07 min; Column: Kinetex EVO C18 2.6 µm 2.1x150 mm, 100Å; Mobile Phase: A = 0.025% TFA in H₂O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1304.08 (M+2H)²⁺

### [Example 4-14]

### Synthesis of 894_3228 (SEQ ID NO: 362)

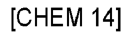

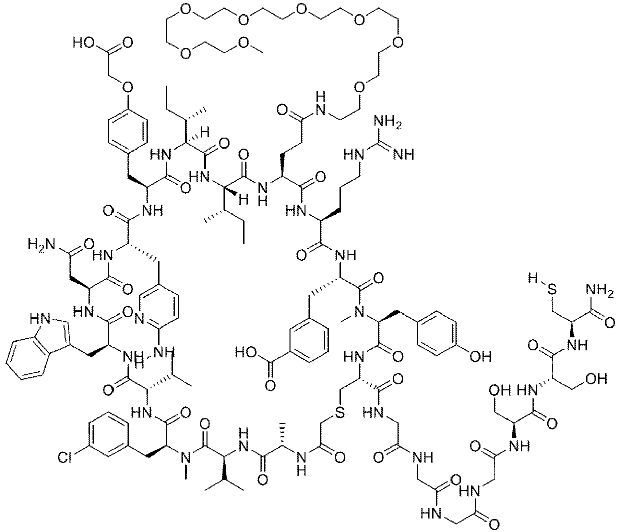

The target peptides were synthesized using Sieber Amide Resin (Watanabe Chemical Industries, Ltd., 0.58 mmol/g, 0.22 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 90°C for 3 min in DMF. However, the 2nd residue was reacted twice at 75°C for 30 min. The 7th residue, 16th residue and 17th residue were reacted once at 90°C for 10 min. The 12th residue was reacted twice at 50°C for 15 min. The 15th residue and 22th residue were reacted at 50°C for 15 min. Fmoc removal was performed by reacting with a DMF solution containing 10% pyrrolidine at 90°C for 1 min. However, Fmoc removal of 1st residue, 2nd residue, 3rd residue, 4th residue, 5th residue, 6th residue, 7th residue, 8th residue, 9th residue, 10th residue, 11th residue and 13th residue were carried out by reacting at 25°C for 1 min, followed by reacting for 1 min. The solid-phase resin was then shaken with a DCM solution of 5 equivalents of Fmoc-OSu per 1 equivalent of solid-phase resin for 1 hour and washed with DMF. The solid-phase resin was suspended in DCM and 0.2 equivalents of tetrakis(triphenylphosphine)palladium(0) and 10 equivalents of phenylsilane were added to 1 equivalent of resin, then shaken for 1 hour and washed with DCM, DMF and DCM. The resin was suspended in DMF and reacted with H-PEG8Me/DIPCI/Oxyma pure (4 equivalents/8 equivalents/4 equivalents) to 1 equivalent of resin at 75°C for 30 min. The introduction of the chloroacetyl group was performed by reacting the solid-phase resin containing the Fmoc-protected peptide obtained in the previous step with a 20% piperidine solution in DMF at 25°C for 10 min to remove the Fmoc group of the α-amino group, followed by washing with DMF and then adding ClAcOSu (0.025 M), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (12 mL, mixture of TFA/H2O/TIS/DODT in a volume ratio of 90/2.5/2.5/5.0) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in acetonitrile/water (1/1) to a final concentration of 5 mM based on the mol number of the solid-phase resin, and then 10 equivalents of triethylamine was added and stirred at room temperature for 1 hour. The reaction solution was quenched with acetic acid and concentrated under reduced pressure using Genevac HT-12. The resulting residue was dissolved in DMSO so that the final concentration of peptide becomes 25mM based on the mol number of the solid-phase resin, and then 3 equivalents of silver acetate was added and reaction was performed at room temperature for 5 hours, then the reaction solvent was quenched with 2M DTT solution. The mixture was centrifuged (9000 rpm, 2 min) and the solution was collected. The solid was washed with DMSO and the supernatant was mixed with the previously collected solution.

The resulting crude product was purified using the following conditions (Column: Waters Xbridge (registered trademark) C18 5µm 50x150 mm; Mobile Phase: A = 0.1% TFA in H2O, B = 0.1% TFA in MeCN; Temperature: 40°C; Gradient (%B): 30-30% over 3 min, then 30-35% over 8 min, then 35-60% over 1 min; Flow Rate: 20-20mL/min over 1 min, then 20-120mL/min over 2 min, then 120mL/min).

The purity of the target product was 93.1%, calculated from the area ratio of the LC/MS (UV wavelength 225 nm) chromatogram under analytical conditions.

Analytical Conditions: Retention Time = 3.82 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1021.25 (M+3H)³⁺

### [Example 4-15]

### Synthesis of 894_3326 (SEQ ID NO: 347)

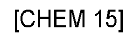

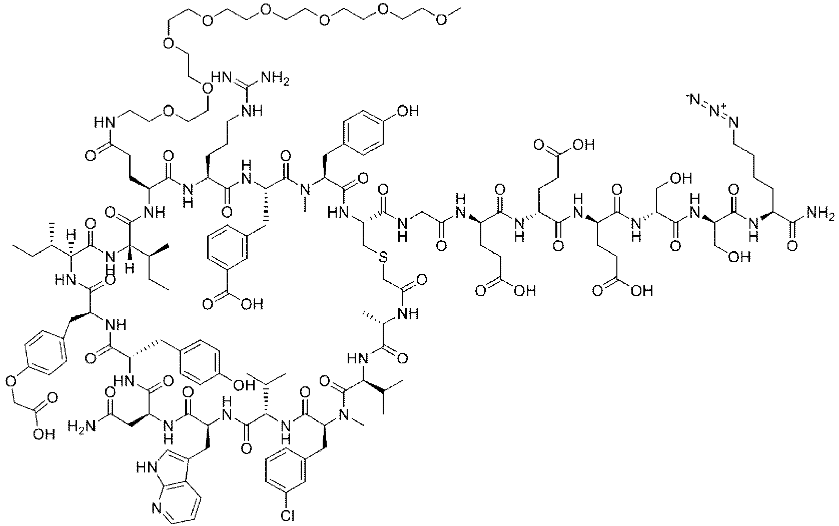

The target peptides were synthesized using Sieber Amide Resin (Watanabe Chemical Industries, Ltd., 0.57 mmol/g, 0.22 g), starting with the removal of the Fmoc group by the general method described above. Liberty Blue HT (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 90°C for 3 min in DMF. However, the 2nd residue was reacted twice at 75°C for 30 min. The 11th residue was reacted once at 75°C for 30 min. The 12th residue was reacted twice at 50°C for 15 min. The 15th residue was reacted once at 50°C for 15 min. Fmoc removal was performed by reacting with a DMF solution containing 10% pyrrolidine at 90°C for 1 min. However, Fmoc removal of 2nd residue and 13th residue were carried out by reacting at 25°C for 1 min, followed by reacting for 1 min. The introduction of the chloroacetyl group was performed by removing the Fmoc group of α-amino group from the solid-phase resin containing the Fmoc-protected peptide obtained in the previous step by the method described above, and after washing with DMF, then adding ClAcOSu (0.25 M), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 120 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (7 mL, mixture of TFA/H2O/TIS/DODT in a volume ratio of 90/2.5/2.5/5.0) was added, and the vessel was shaken at room temperature for 30 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diethyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in acetonitrile/water (1/1) to a final concentration of 5 mM based on the mol number of the solid-phase resin, and then 10 equivalents of triethylamine was added and stirred at room temperature for 1 hour, followed by adding 2% hydrazine and reacting for 2 hours. The reaction solution was quenched with acetic acid and concentrated under reduced pressure using Genevac HT-12.

The resulting crude product was purified using the following conditions (Column: Waters Xbridge (registered trademark) C18 5µm 50x150 mm; Mobile Phase: A = 0.1% TFA in H2O, B = 0.1% TFA in MeCN; Temperature: 40°C; Gradient (%B): 5-5% over 2 min, then 5-29% over 1 min, then 29-34% over 8 min, then 34-60% over 1 min; Flow Rate: 20-20mL/min over 1 min, then 20-120mL/min over 1 min, then 120mL/min). After lyophilization, the product was purified using the following conditions (Column: Waters XSelect (registered trademark) C18 5µm 19x150 mm; Mobile Phase: A = 0.1% TFA in H2O, B = 0.1% TFA in MeCN; Temperature: 60°C; Gradient (%B): 5-30% over 3 min, then 30-35% over 8 min, then 35-60% over 1 min; Flow Rate: 17mL/min).

The purity of the target product was 98.4%, calculated from the area ratio of the LC/MS (UV wavelength 225 nm) chromatogram under analytical conditions.

Analytical Conditions: Retention Time = 3.84 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1110.97 (M+3H)³⁺

### [Example 5]

The peptide conjugates (complexes of peptides and payloads bound via linkers) listed in TABLE 6 were synthesized. In this example, examples of synthesis are described below. For the fluorescent Alexa Fluor (registered trademark) 647-peptide conjugates represented by 894_0501 and 894_0502 in TABLE 6, the exact MS value of Alexa Fluor (registered trademark) 647 is unknown, so the X value of [M+XH]X+ is not listed.

### [Example 5-1]

### Synthesis of 894_0502 (TABLE 6)

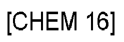

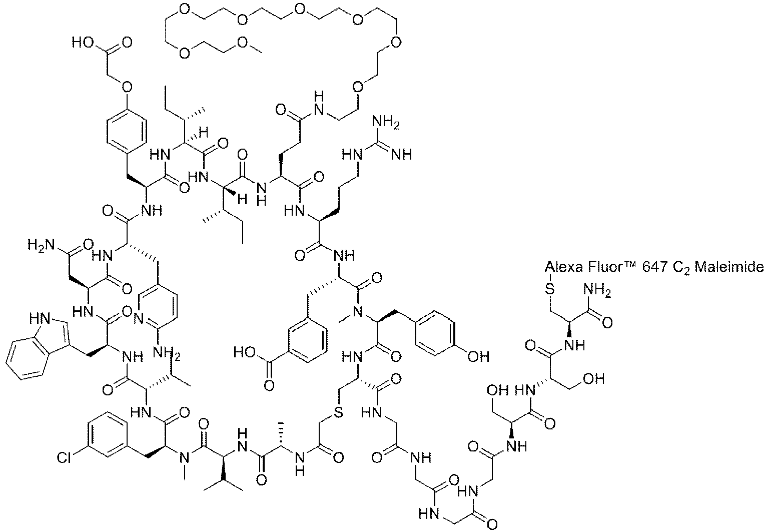

894_3228 (5 mg, 1.58 µmol) synthesized in Example 4-14 was dissolved in DMSO (0.3 mL), and triethylamine (2.4 mg, 24.0 µmol) and Alexa Fluor (registered trademark) 647 C2 Maleimide (registered trademark) (1.9 mg, 1.73 µmol) were added and the mixture was stirred at room temperature all night.

The crude product was purified using the following conditions (Column: Waters XSelect (registered trademark) C18 5 µm 19x150 mm; Mobile Phase: A = 0.1% TFA in H2O, B = 0.1% TFA in MeCN; Temperature: 60°C; Gradient (%B): 19-49% over 11 min, then 49-60% over 1 min; Flow Rate: 17 mL/min).

The purity of the target product was 93.5%, calculated from the area ratio of the LC/MS (UV wavelength 225 nm) chromatogram under analytical conditions.

Analytical Conditions: Retention Time = 5.01 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1348.24 (M+3H)³⁺

### [Example 5-2]

### Synthesis of 894_3221 (TABLE 6)

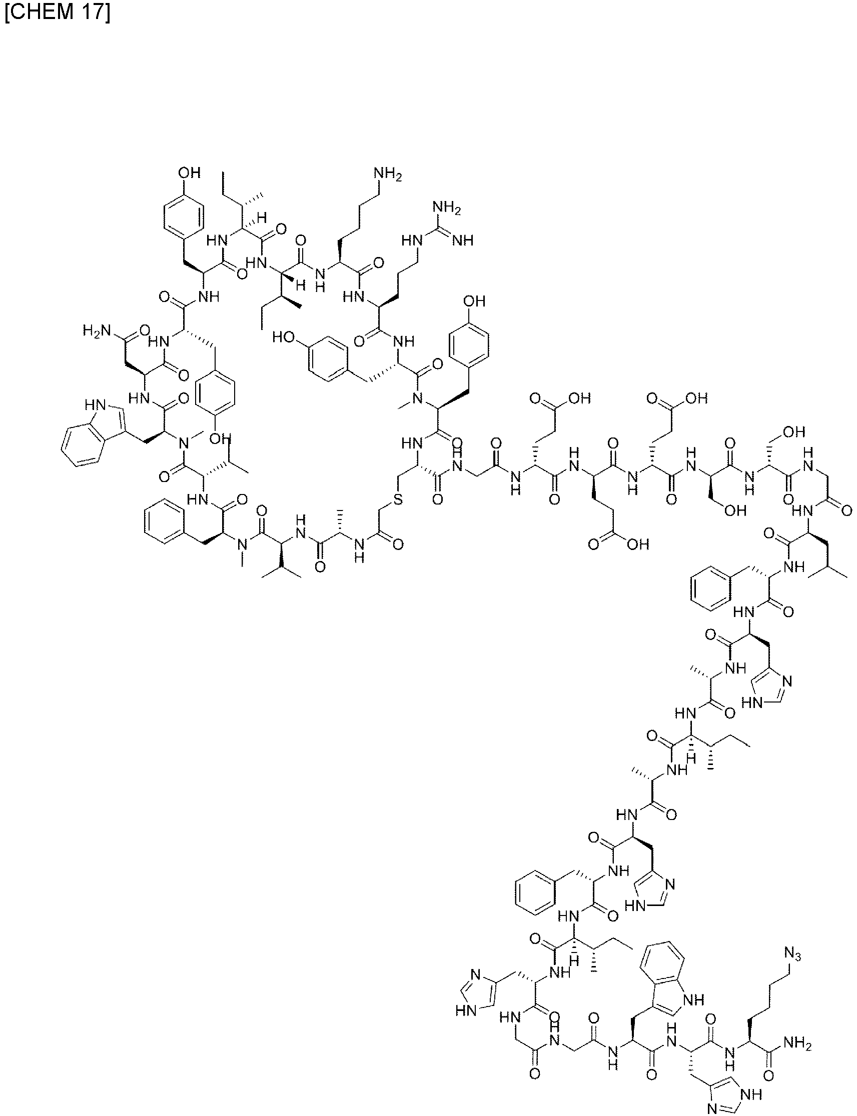

The target peptides were synthesized using Sieber Amide Resin (Watanabe Chemical Industries, Ltd., 0.57 mmol/g, 0.88 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 75°C for 10 min in DMF. However, the 2nd residue and 4th residue were reacted twice at 75°C for 30 min. The 12th residue was reacted twice at 50°C for 15 min. The 13th residue and 21st residue were reacted twice at 75°C for 10 min. The 15th residue, 25th residue, 29th residue, 32nd residue and 36th residue were reacted once at 50°C for 15 min. Fmoc removal was performed by reacting with a DMF solution containing 20% piperidine at 75°C for 3 min. However, Fmoc removal of 2nd residue, 4th residue, 13th residue and 21st residue was carried out by reacting with a DMF solution containing 20% piperidine at 25°C for 5 min, followed by reacting for 5 min. The introduction of the chloroacetyl group was performed by removing the Fmoc group of α-amino group from the solid-phase resin containing the Fmoc-protected peptide obtained in the previous step by the method described above, and then adding ClAcOSu (5 mM), which was prepared by mixing chloroacetic acid (about 10 equivalents), DIPCI (about 10 equivalents), and HOSu (about 10 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (30 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in 10% hydrous DMSO so that the final concentration of the peptide becomes 5 mM based on the mol number of the solid-phase resin, and then 10 equivalents of triethylamine was added and stirred at 25°C for 4 hours, then the reaction was quenched with acetic acid. The reaction solution was concentrated under reduced pressure using Genevac HT-12.

The resulting crude product was purified using the following conditions (Column: Waters Xbridge (registered trademark) C18 5µm 50x150 mm; Mobile Phase: A = 0.1% TFA in H2O, B = 0.1% TFA in MeCN; Temperature: 40°C; Gradient (%B): 35-35% over 3 min, then 35-40% over 8 min, then 40-60% over 1 min; Flow Rate: 20mL/min over 1 min, then 20-120mL/min over 2 min, then 120mL/min). After lyophilization, the product was purified using the following conditions (Column: Waters XSelect Fluoro-Phenyl (registered trademark) 5µm 10x150 mm; Mobile Phase: A = 1.0% AcOH in H2O, B = 1.0% AcOH in MeCN; Temperature: 50°C; Gradient (%B): 5-20% over 3 min, then 20-25% over 8 min, then 25-60% over 1 min; Flow Rate: 5mL/min).

The purity of the target product was 98.7%, calculated from the area ratio of the LC/MS (UV wavelength 225 nm) chromatogram under analytical conditions.

Analytical Conditions: Retention Time = 4.04 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1135.17 (M+4H)⁴⁺

### [Example 5-3]

### Synthesis of 894_3222 (TABLE 6)

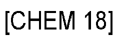

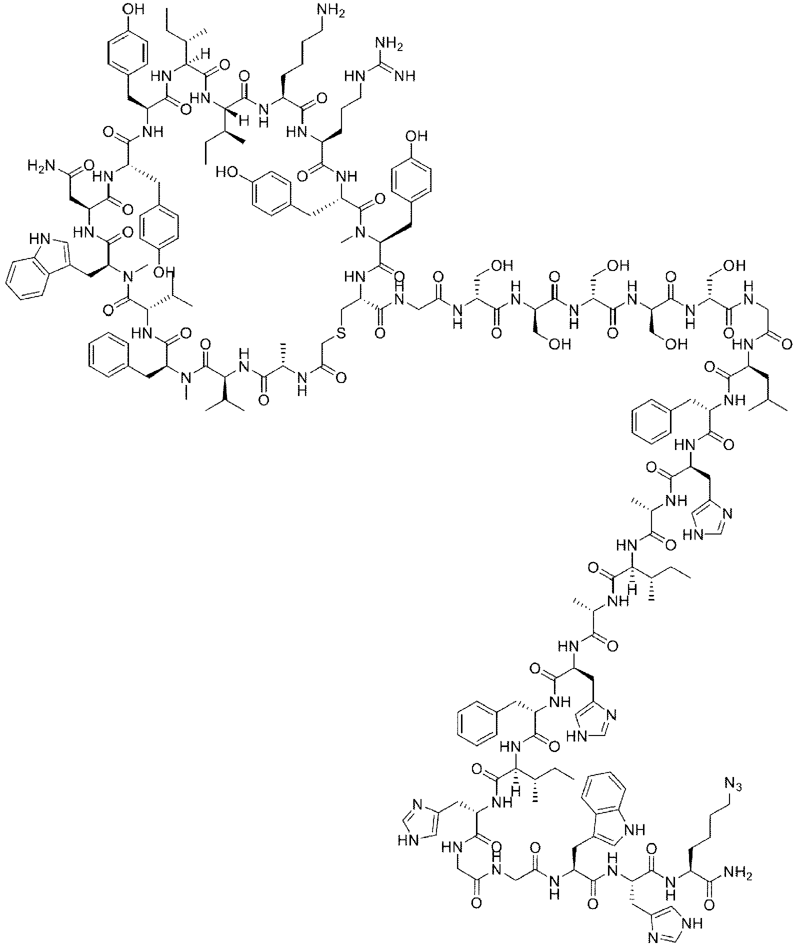

The target peptides were synthesized using Sieber Amide Resin (Watanabe Chemical Industries, Ltd., 0.57 mmol/g, 0.88 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out once at 75°C for 10 min in DMF. However, the 2nd residue and 4th residue were reacted twice at 75°C for 30 min. The 12th residue was reacted twice at 50°C for 15 min. The 13th residue and 21st residue were reacted twice at 75°C for 20 min. The 15th residue, 25th residue, 29th residue, 32nd residue and 36th residue were reacted once at 50°C for 15 min. Fmoc removal was performed by reacting with a DMF solution containing 20% piperidine at 75°C for 3 min. However, Fmoc removal of 2nd residue, 4th residue, 13th residue and 21st residue was carried out by reacting with a DMF solution containing 20% piperidine at 25°C for 5 min, followed by reacting for 5 min. The introduction of the chloroacetyl group was performed by removing the Fmoc group of α-amino group from the solid-phase resin containing the Fmoc-protected peptide obtained in the previous step by the method described above, then adding ClAcOSu (5 mM), which was prepared by mixing chloroacetic acid (about 10 equivalents), DIPCI (about 10 equivalents), and HOSu (about 10 equivalents) and shaking in DCM for60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (30 mL, mixture of TFA/H2O/TIS/DODT in a volume ratio of 92.5/2.5/2.5/2.5) was added, and the vessel was shaken at room temperature for 90 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in 10% hydrous DMSO so that the final concentration of the peptide becomes 5 mM based on the mol number of the solid-phase resin, and then 10 equivalents of triethylamine was added and stirred at 25°C for 4 hours, then the reaction was quenched with acetic acid. The reaction solution was concentrated under reduced pressure using Genevac HT-12.

The resulting crude product was purified using the following conditions (Column: Waters Xbridge (registered trademark) C18 5µm 50x150 mm; Mobile Phase: A = 0.1% TFA in H2O, B = 0.1% TFA in MeCN; Temperature: 40°C; Gradient (%B): 34-34% over 3 min, then 34-39% over 8 min, then 39-60% over 1 min; Flow Rate: 20mL/min over 1 min, then 20-120mL/min over 2 min, then 120mL/min). After lyophilization, the product was purified using the following conditions (Column: Waters XSelect Fluoro-Phenyl (registered trademark) 5µm 10x150 mm; Mobile Phase: A = 1.0% AcOH in H2O, B = 1.0% AcOH in MeCN; Temperature: 50°C; Gradient (%B): 5-19% over 3 min, then 19-24% over 8 min, then 24-60% over 1 min; Flow Rate: 5mL/min).

The purity of the target product was 98.8%, calculated from the area ratio of the LC/MS (UV wavelength 225 nm) chromatogram under analytical conditions.

Analytical Conditions: Retention Time = 4.43 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1103.64 (M+4H)⁴⁺

### [Example 5-4]

### Synthesis of 894_33808 (TABLE 6)

In this example, a peptide conjugate was synthesized in which the following signal peptide (sequence number 395: G-L-F-H-A-I-A-H-F-I-H-G-G-W-H) was bound as the payload. The payload is bound to the side chain of dk in the linker.

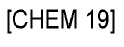

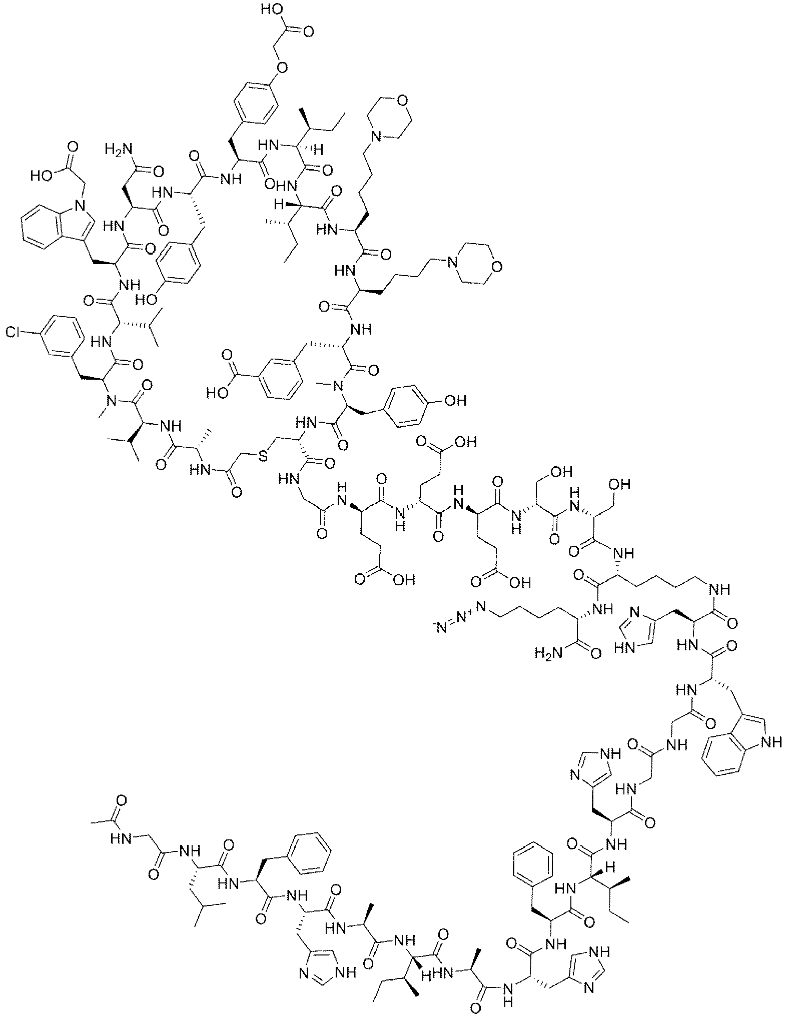

The target peptides were synthesized using Sieber Amide Resin (Watanabe Chemical Industries, Ltd., 0.57 mmol/g, 0.49 g), starting with the removal of the Fmoc group by the general method described above. Liberty Blue (CEM Inc.) was used as the solid-phase synthesizer, and the synthesis was performed according to the manufacturer's manual. For the introduction of each residue, Fmoc-AA/DIPCI/Oxyma Pure (4.2 equivalents/8 equivalents/4 equivalents) was used per 1 equivalent of resin, and the reaction was carried out in DMF. Fmoc removal was performed by reacting with a DMF solution containing 10% pyrrolidine. Fmoc-KN3-OH was introduced into the resin by one reaction at 90°C for 3 min, and then the Fmoc group was removed by reaction at 90°C for 1 min. The peptide was then reacted with Alloc-dk(Fmoc)-OH once at 90°C for 3 min for introducing it, and the Fmoc group was then removed by reacting for 1 min at 90°C, followed by peptide elongation from the amino group of the lysine side chain. Amino acids other than Fmoc-His(Trt)-OH was introduced by reacting once at 90°C for 3 min. Fmoc-His(Trt)-OH was introduced by reacting once at 50°C for 15 min. The Fmoc group of each amino acid was removed by reacting at 90°C for 1 min. After lysine side chain elongation, the resin was washed three times with DMF, reacted with 5% acetic anhydride in dichloromethane solution at room temperature for 30 min, and washed with DMF and dichloromethane. The resulting resin was suspended in dichloromethane and permeated with 0.2 equivalents of tetrakistriphenylphosphine palladium complex(0) and 10 equivalents of phenylsilane at room temperature for 1 hour and washed with dichloromethane and DMF. Peptide elongation was performed from the lysine α-position amino group of the resulting resin. Fmoc-ds(tBu)-OH, Fmoc-de(tBu)-OH, Fmoc-Gly-OH, and Fmoc-MeY(tBu)-OH were introduced by reacting once at 90°C for 10 min. Fmoc group was removed by reacting at 75°C for 1 min. Fmoc-C(Trt)-OH was introduced by reacting at 50°C for 15 min. Fmoc group was removed by reacting at 75°C for 1 min. Fmoc-F3COO(tBu)-OH was introduced by reacting twice at 90°C for 10 min. Fmoc group was removed by reacting at 75°C for 1 min. Other amino acids were introduced by reacting once or twice at 30°C for 40 min. In particular, the reaction was performed twice when N-terminal alanine was the 1st residue and the 2nd Fmoc-V-OH residue and the 9th Fmoc-I-OH residue were introduced. The Fmoc group was removed by reacting at 25°C for 1 min followed by reacting for 1 min. The introduction of the chloroacetyl group was performed by removing the Fmoc group of the α-amino group from the solid-phase resin holding the Fmoc-protected peptide obtained in the previous step by the method described above, and then adding ClAcOSu (20 mM), which was prepared by mixing chloroacetic acid (about 5 equivalents), DIPCI (about 5 equivalents), and HOSu (about 5 equivalents) and shaking in DCM for 60 min followed by adding the same volume of DMF as DCM, and shaking at 25°C for 60 min. For deprotection of the side chains and removal from the solid-phase resin, the resin obtained after the chloroacetyl group introduction step was first washed 5 times with DMF and 3 times with methylene chloride, followed by washing with diethyl ether and drying under reduced pressure. Then, to the reaction vessel containing the solid-phase resin, reactant cocktail-A (8.6 mL, mixture of TFA/H2O/TIS/DODT in a volume ratio of 92.5/2.5/2.5/2.5) was added, and the vessel was shaken at room temperature for 70 min. The reaction solution was collected by filtration through a frit. The solid-phase resin remaining in the reaction vessel was shaken again with the cut-out cocktail, and the solution component was collected from the frit and mixed with the aforementioned filtrate. The filtrate was added to an excess of diisopropyl ether/hexane (1/1) mixed solvent cooled to 0°C, and a cloudy precipitate was formed. The mixture was centrifuged (9000 rpm, 2 min) and the solution was decanted. The resulting solid was washed again with a small amount of diethyl ether cooled to 0°C and dried under reduced pressure. The solid obtained was used for the next cyclization reaction. For the cyclization reaction of the peptide, the peptide was dissolved in 10% hydrous DMSO so that the final concentration of the peptide becomes 5 mM based on the mol number of the solid-phase resin, and then 10 equivalents of triethylamine was added and stirred at 25°C for 15 hours. The reaction solution was concentrated under reduced pressure using Genevac HT-12.

The resulting crude product was purified using the following conditions (Column: Waters Xbridge (registered trademark) C18 5µm 50x250 mm; Mobile Phase: A = 0.1% TFA in H2O, B = 0.1% TFA in MeCN; Temperature: 50°C; Gradient (%B): 9.3-5.6% over 0.1 min, then 5.6-5.6% over 4.9 min, then 5.6-9.3% over 2 min, then 9.3-34.7% over 3 min, then 34.7-39.8% over 15 min, then 39.8-60% over 3 min; Flow Rate: 118-18mL/min over 0.1 min, then 18mL/min-18mL/min over 4.9 min, then 18-118mL/min over 2 min, then 118mL/min). After lyophilization, the product was purified using the following conditions (Column: Kinetex EVO C18 2.6µm 30x150 mm; Mobile Phase: A = 1.0% AcOH in H2O, B = 1.0% AcOH in MeCN; Temperature: 40°C; Gradient (%B): 5-25% over 3 min, then 25-30% over 8 min, then 30-60% over 1 min; Flow Rate: 45mL/min).

The purity of the target product was 99.9%, calculated from the area ratio of the LC/MS (UV wavelength 225 nm) chromatogram under analytical conditions.

Analytical Conditions: Retention Time = 4.36 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1246.89 (M+4H)⁴⁺

### [Example 5-5]

### Synthesis of 894_3372 (TABLE 6)

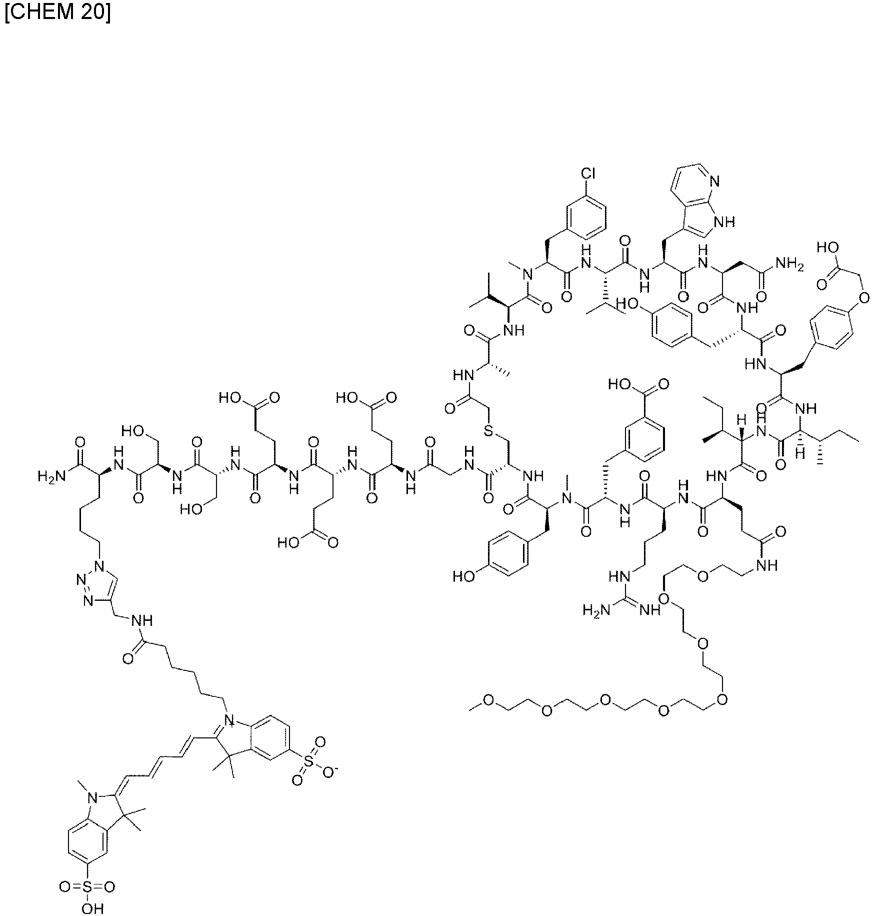

894_3326 (2 mg, 0.56 µmol) synthesized in Example 4-15 was suspended in DMF (75 pL)/water (25 pL), and then tetrakis(acetonitrile)copper(I) hexafluorophosphate (0.21 mg, 0.56 µmol, CAS: 64443-05-6), tris((1-benzyl-1H-1,2,3-triazol-4-yl)methyl)amine (0.30 mg, 0.56 µmol, CAS: 510758-28-8) and Sulfo-Cy5-alkyne (0.40 mg, 0.56 µmol, CAS: 1617572-09-4) was added and the mixture was stirred at room temperature for 16 hours.

The crude product was purified using the following conditions (Column: Waters XSelect (registered trademark) C18 5µm 19x150 mm; Mobile Phase: A = 0.1% TFA in H2O, B = 0.1% TFA in MeCN; Temperature: 60°C; Gradient (%B): 5-30% over 3 min, then 30-35% over 8 min, then 35-60% over 1 min; Flow Rate: 17mL/min).

The purity of the target product was 98.4%, calculated from the area ratio of the LC/MS (UV wavelength 225 nm) chromatogram under analytical conditions.

Analytical Conditions: Retention Time = 4.21 min; Column: Kinetex EVO C18 2.6 µm 2.1x150 mm, 100Å; Mobile Phase: A = 0.025% TFA in H2O, B = 0.025% TFA in MeCN; Temperature: 60°C; Gradient (% B cone): 20-60% over 7.15 min, then 60-95% over 0.3 min, then 95-95% over 1.55 min; Flow Rate: 0.5 mL/min.

ESI-MS (+) observed m/z = 1337.07 (M+3H)³⁺

### [Example 6]

### Intermolecular Interaction Evaluation Test Between Peptide Conjugate and hTfR Using Surface Plasmon Resonance (SPR)

Peptide-fluorescent conjugates 894_0501 and 894_0502 (TABLE 6), synthesized in the same manner as Example 5-1, were tested for intermolecular interaction of the peptide to hTfR using surface plasmon resonance (SPR), by the same method as Example 3. The obtained KD values were designated A for KD values less than 1 nM, B for KD values between 1 nM and 100 nM, C for KD values between 100 nM and 1 µM, and D for KD values greater than 1 µM). As a result, the synthesized peptide-antibody substance conjugates both had B binding activity to hTfR, indicating that the peptides of the present invention showed significant binding activity to human TfR, even when the linker was given or/and the payload was bound.

New amino acids were synthesized as follows.

### [Synthesis Example 1-1]

### Synthesis of Fmoc-F3COO(allyl)-OH

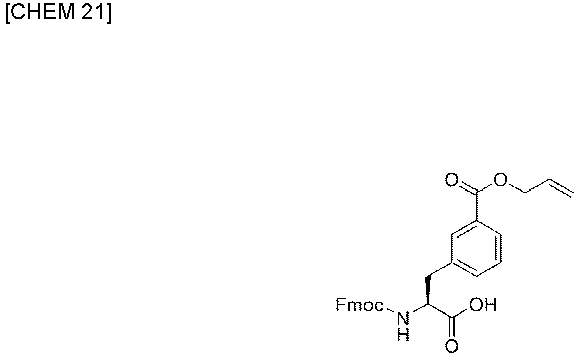

Under nitrogen atmosphere, zinc (11.5 g, 175.0 mmol) was suspended in DMF (600 mL), then iodine (1.18 g, 4.7 mmol) was added and the mixture was stirred at room temperature for 10 min. Methyl(2R)-2-[[(9H-fluoren-9-ylmethoxy)carbonyl]amino]-3-lodopropanoate (31.6 g, 70.0 mmol) and iodine (1.2 g, 4.7 mmol) were added and the mixture was stirred at room temperature for 30 minutes. Tert-butyl-3-bromobenzoate (15 g, 58.3 mmol), tris(dibenzylideneacetone)dipalladium(0)-chloroform adduct (1.8 g, 1.8 mmol) and 2-dicyclohexylphosphino-2',6'-dimethoxybiphenyl (1.2 g, 2.9 mmol) was added and the mixture was stirred at 50°C for 2 hours. The solid was removed by filtration and the filtrate was quenched with water (100 mL). The reaction solution was extracted three times with ethyl acetate (100 mL). The organic layers were combined, washed three times with saturated saline solution (100 mL), and dried with anhydrous sodium sulphate. Filtration was performed and the filtrate was concentrated under reduced pressure and purified by silica gel column chromatography (ethyl acetate/petroleum ether = 1/10). The resulting product (20 g, 39.9 mmol) was stirred in a dioxane solution of 4N HCl at room temperature for 12 hours. The reaction solution was concentrated under reduced pressure, dried, and the resulting product (15.5 g, 34.8 mmol) was dissolved in acetonitrile (150 mL) and stirred with DIEA (9.0 g, 69.6 mmol) and allyl bromide (6.3 g, 52.2 mmol) at room temperature for 12 hours. The reaction solution was diluted with ethyl acetate (100 mL), washed three times with saturated saline solution (100 mL), dried by anhydrous sodium sulphate, filtered, and the filtrate was concentrated under reduced pressure. The resulting residue was purified by silica gel column chromatography (ethyl acetate/petroleum ether = 1/6). The resulting product (9.0 g, 18.5 mmol) was dissolved in isopropanol (120 mL) and water (40 mL), and calcium chloride (32.9 g, 296.6 mmol) was added at 0°C in several portions. Lithium hydroxide (1.8 g, 74.1 mmol) was then added at 0°C in several portions, and the mixture was stirred at room temperature for 1 hour. The reaction solution was diluted with aqueous solution of citric acid (50 mL) and extracted 3 times with ethyl acetate (100 mL). The organic layers were combined and washed three times with saturated saline solution (100 mL). The mixture was dried by anhydrous sodium sulphate, filtered, and concentrated under reduced pressure. The resulting residue was purified by preparative HPLC (Column: C18 silica gel, acetonitrile/water = 0/100-38/62 (over 20 min)). The titled compound was obtained. ESI-MS (+) observed m/z = 494.10 (M+Na)⁺

### [Synthesis Example 1-2]

### Synthesis of Fmoc-F4aao(allyl)-OH

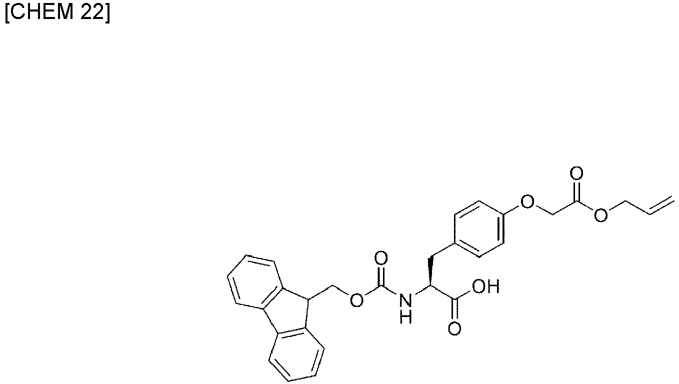

Tert-butyl(2S)-2-[(tert-butoxycarbonyl)amino]-3-(4-hydroxyphenyl) propanoate (20 g, 59.3 mmol) was dissolved in acetonitrile (240 mL) and then potassium carbonate (16.4 g, 118.5 mmol) and allyl chloroacetate (10,3 g, 76.5 mmol) were added and the mixture was stirred at 90°C overnight. The reaction solution was concentrated under reduced pressure, and the resulting residue was purified by silica gel column chromatography (ethyl acetate/petroleum ether = 1/100-1/5). The resulting product (16.1 g, 37.0 mmol) was dissolved in dichloromethane (50 mL) and TFA (70 mL) and stirred at room temperature for 1 day. The reaction solution was concentrated under reduced pressure and dried. A portion of the obtained product (5.2 g, 13.8 mmol) was dissolved in water (25 mL) and sodium hydrogen carbonate (3.9 g, 46.6 mmol) was added. A solution of N-(9-fluorenylmethoxycarbonyloxy)succinimide (6.3 g, 18.6 mmol) in dioxane (25 mL) was added dropwise at 0°C to the reaction solution while stirring, and the mixture was stirred at room temperature for 2 hours. Aqueous solution of citric acid (40 mL) was added to quench the reaction, and the mixture was extracted 3 times with ethyl acetate (50 mL). The organic layers were combined, washed once with saturated saline solution (50 mL), dried with sodium sulphate, filtered, and the filtrate was concentrated under reduced pressure. The resulting residue was purified by preparative HPLC (Column: C18 silica gel, acetonitrile containing 1% formic acid/water = 45/55-75/25 (over 40 minutes). The titled compound was obtained. ESI-MS (+) observed m/z = 524.10 (M+Na)⁺

### [Synthesis Example 1-3]

### Synthesis of Fmoc-MeF3COO(allyl)-OH

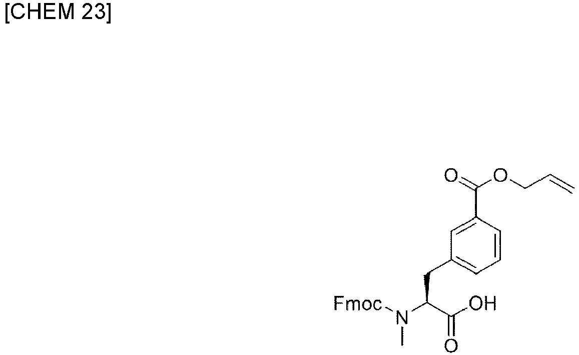

(S)-2-((((9H-fluoren-9-yl)methoxy)carbonyl)amino-3-(3-((aryloxy)carbonyl)phenyl)propanoic acid (7.5 g, 15.8 mmol), synthesized by abovementioned method is mixed with acetic acid (7.5 mL, 124.4 mmol), CSA (0.74 g, 3.2 mmol) and paraformaldehyde (4.8 g, 158.4 mmol) in acetic acid (7.5 mL, 124.4 mmol) and stirred at 110°C for 1 hour in toluene (70 mL). After concentration under reduced pressure, the resulting residue was purified by silica gel column chromatography (ethyl acetate/petroleum ether = 4/1). The resulting product (4.3 g, 8.9 mmol) was dissolved in DCM (24 mL) and TFA (24 mL) was added dropwise at 0°C while stirring. Triisopropylsilane (4.2 g, 26.7 mmol) was added dropwise at 0°C while stirring the reaction solution. The reaction solution was stirred at room temperature for 2 days. After concentrating under reduced pressure, the mixture was dissolved in ethyl acetate (50 mL) and water (50 mL) was added. Sodium hydrogen carbonate was added to the mixture to adjust the pH to 6, and the mixture was extracted 4 times with ethyl acetate (50 mL). The organic layers were combined, washed three times with water (50 mL), and then washed three times with saturated saline solution (50 mL). The mixture was dried with sodium sulphate, filtered, and the filtrate was concentrated under reduced pressure. The resulting residue was purified by preparative HPLC (Column: C18 silica gel, acetonitrile containing 0.1% formic acid/water containing 0.1% formic acid = 50/50-80/20 (over 30 min)). The titled compound was obtained. ESI-MS (+) observed m/z = 486.10 (M+H)⁺

### [Synthesis Example 1-4]

### Synthesis of Fmoc-QhEt-OH

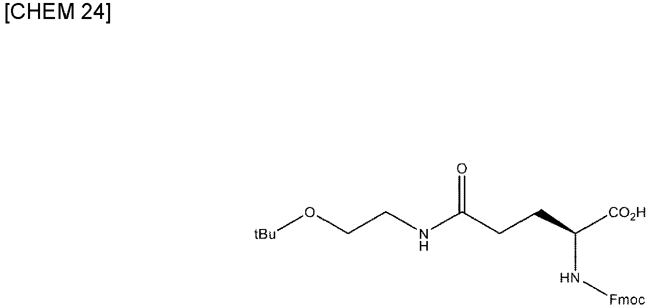

N-[(9H-fluoren-9-ylmethoxy)carbonyl]-L-glutamic acid-1-allyl (15.0 g, 36.6 mmol) and 2-(tert-butoxy)ethanamine (4.9 g, 42.1 mmol) were dissolved in DMF (200 mL), and then HATU (16.7 g, 44.0 mmol) and DIEA (7.1 g, 55.0 mmol) were added at 0°C, and the mixture was stirred at room temperature for 2 hours. The reaction solution was quenched with water and extracted 3 times with ethyl acetate (500 mL). The organic layers were combined and washed 3 times with saturated saline solution (500 mL). The mixture was dried with anhydrous sodium sulphate, filtered, and the filtrate was concentrated under reduced pressure. The resulting residue was purified by silica gel column chromatography (ethyl acetate/petroleum ether = 6/4). A portion of the obtained product (7.0 g, 13.8 mmol) was dissolved in THF (150 mL), and morpholine (2.4 g, 27.5 mmol) and tetrakis(triphenylphosphine)palladium(0) (1.6 g, 1.4 mmol) were added, then the mixture was stirred at room temperature for 30 min. The reaction solution was diluted with ethyl acetate (100 mL), and the pH was adjusted to 5 by adding 2M hydrochloric acid. The mixture was extracted twice with ethyl acetate (200 mL). The organic layers were combined, washed 3 times with saturated saline solution (300 mL), and dried with anhydrous sodium sulphate. Filtration is performed and the filtrate was concentrated under reduced pressure and the resulting residue was purified by preparative HPLC (Column: C18 silica gel, Gradient: CH3CN/H2O = 5/95-100/0 over 40 min). The titled compound was obtained. ESI-MS (+) observed m/z = 469.15 (M+H)⁺

### [Synthesis Example 1-5]

### Synthesis of Fmoc-Qpipzaa-OH

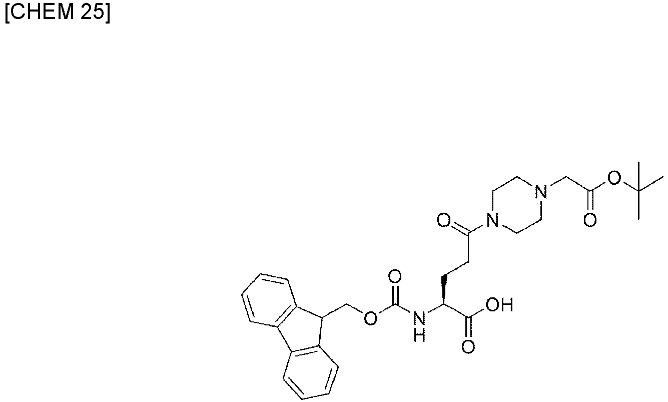

N-[(9H-fluoren-9-ylmethoxy)carbonyl]-L-glutamic acid-1-allyl (15.0 g, 36.6 mmol) was dissolved in DMF (500 mL), and *tert*-butyl2-(piperazin-1-yl)acetate (8.8 g, 44.0 mmol), HATU (27.9 g, 73.3 mmol), and DIEA (25.5 mL, 197.5 mmol) were added and then the mixture was stirred at room temperature for 15 hours. The reaction solution was diluted with ethyl acetate (2 L) and washed 3 times with saturated saline solution (500 mL). The organic layer was dried with anhydrous sodium sulphate, filtered, and the filtrate was concentrated under reduced pressure. The resulting residue was purified by silica gel column chromatography (ethyl acetate/petroleum ether = 1/1). A portion of the obtained product (10.0 g, 16.9 mmol) was dissolved in THF (200 mL), and tetrakis(triphenylphosphine)palladium(0) (2.0 g, 1.7 mmol) and morpholine (2.9 g, 33.8 mmol) were added, then the mixture was stirred at room temperature for 30 min. Citric acid solution was added to adjust the pH to 5-6, and the mixture was extracted 3 times with ethyl acetate (500 mL). The organic layers were combined, washed 3 times with 300 mL of saturated saline solution, and dried with anhydrous sodium sulphate. Filtration is performed and the filtrate was concentrated under reduced pressure. The resulting residue was purified by silica gel column chromatography (ethyl acetate/petroleum ether = 1/1). The titled compound was obtained. ESI-MS (+) observed m/z = 552.30 (M+H)⁺

### [Synthesis Example 1-6]

### Synthesis of Fmoc-W1aa(allyl)-OH

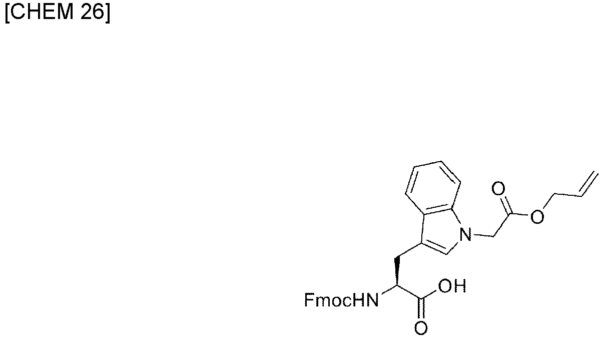

Under a nitrogen atmosphere, N-tert-butoxycarbonyl-L-tryptophan (20 g, 65.7 mmol) was dissolved in THF/DMF (100 mL/100 mL), then potassium tert-butoxide (14.8g, 131.9 mmol) was added slowly at 0°C, and the mixture was stirred at 0°C for 10 min. While stirring the reaction mixture at 0°C, allyl chloroacetate (7.9 mL, 67.8 mmol) was added dropwise and stirred for 5 minutes, followed by stirring at room temperature overnight. The reaction was quenched with aqueous solution of citric acid and extracted 3 times with ethyl acetate (50 mL). The organic layers were combined, washed 3 times with saturated saline solution (50 mL), dried with anhydrous sodium sulphate, filtered, and the filtrate was concentrated under reduced pressure. The resulting residue was purified by silica gel column chromatography (ethyl acetate/petroleum ether = 1/1). A portion of the product obtained (15.0 g, 37.2 mmol) was dissolved in dichloromethane (50 mL) and TFA (50 mL) and stirred at room temperature for 1 hour. The mixture was concentrated under reduced pressure and dried. A portion of the product obtained (4.0 g, 13.2 mmol) was dissolved in dioxane (80 mL), and then water (40 mL), sodium hydrogen carbonate (4.5 g, 53.0 mmol) and N-(9-fluorenylmethoxycarbonyloxy)sucinimide (6.7 g, 19.8 mmol) were added and the mixture was stirred at room temperature all night. The solid was filtered and citric acid was added to adjust the pH to 5. The mixture was extracted 3 times with ethyl acetate (100 mL). The organic layers were combined and washed 3 times with saturated saline solution (100 mL). The mixture was dried with anhydrous sodium sulphate, filtered, and the filtrate was concentrated under reduced pressure. The resulting residue was purified by silica gel column chromatography (ethyl acetate/petroleum ether = 1/1) followed by purification by preparative HPLC (Column: C18 silica gel, acetonitrile/water = 1/9-6/4). The titled compound was obtained. ESI-MS (+) observed m/z = 525.25 (M+H)⁺

### [Synthesis Example 1-7]

### Synthesis of Fmoc-dkCOmeglumine-OH

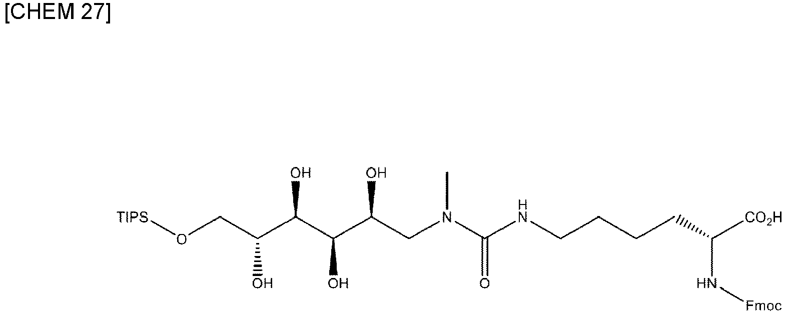

N2-[(9H-fluoren-9ylmethoxy)carbonyl]-D-lysine-2-propene-1-yl ester hydrochloride (5.0 g, 11.2 mmol) was dissolved in dichloromethane (80 mL). Triphosgene (1.7 g, 5.6 mmol) was added at 0°C, then DIEA (6.3 mL, 36 mmol) was added to the reaction solution dropwise and stirred at 0°C for 30 minutes. Dioxane (80 mL) was added to the reaction solution, and then 0.5N sodium hydrogen carbonate solution (80 mL) containing meglumine (8.8 g, 44.9 mmol) was added. The reaction solution was stirred at 0°C overnight. The reaction solution was diluted with ethyl acetate (300 mL), and the organic layer was washed with water, 1M hydrochloric acid, saturated sodium hydrogen carbonate solution and saturated saline solution, then dried over sodium sulphate. The mixture was filtered, and the filtrate was concentrated under reduced pressure. The resulting residue was purified by silica gel column chromatography (dichloromethane/methanol = 100/0-60/40). The resulting product was dissolved in dichloromethane (80 mL), and imidazole (1.9 g, 28.1 mmol) and triisopropylchlorosilane (3.0 g, 14.1 mmol) were added at 0°C and stirred for 5 days. The mixture was diluted with ethyl acetate and washed 3 times with water. The mixture was dried with sodium sulphate, filtered, and the filtrate was concentrated under reduced pressure. The resulting residue was purified by silica gel column chromatography (ethyl acetate/hexane = 0/100-60/40, then dichloromethane/methanol = 100/0-90/10). The resulting product (6.2 g, 7.9 mmol) was dissolved in dichloromethane (40 mL), and phenylsilane (1.9 mL, 15.8 mmol) and tetrakis(triphenylphosphine)palladium(0) (0.2 g, 0.2 mmol) were added, then the mixture was stirred at room temperature for 2 hours. After concentration, the product was purified by silica gel column chromatography (ethyl acetate/hexane = 100/0-60/40, then dichloromethane/methanol = 100/0-90/10). The titled compound was obtained. ESI-MS (+) observed m/z = 746.6 (M+H)⁺

### [Synthesis Example 1-8]

### Synthesis of Fmoc-F3COO(PEG4Me)-OH

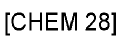

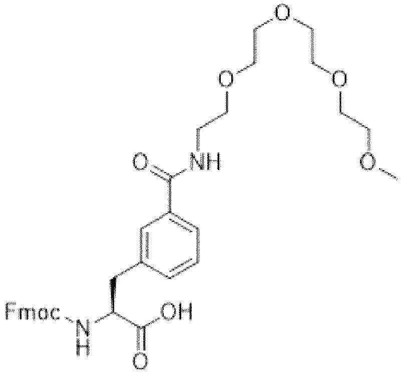

3-lodobenzoic acid (31.5 g, 96.5 mmol) was dissolved in DMF (400 mL), and HATU (44.0 g, 116 mmol) and DIEA (24.9 g, 193 mmol) were added at room temperature, then 2,5,8,11-tetraoxatridecan-13-amine (20 g, 96.5 mmol, CAS: 85030-56-4) was added and stirred for 3 hours. The mixture was diluted with water (1000 mL) and extracted 3 times with ethyl acetate (200 mL). The mixed organic layer was washed 3 times with saturated saline solution (200 mL) and dried with sodium sulphate. After filtration and concentration under reduced pressure, the resulting residue was purified by silica gel column chromatography (petroleum ether/ethyl acetate = 50/50) and 3-iodo N-(2,5,8,11-tetraoxatridecan-13-yl)benzamide was obtained. At room temperature, zinc (15.7 g, 240 mmol) was suspended in DMF and iodine (12.2 g, 48.0 mmol) was added, then the mixture was stirred for 10 minutes. Methyl(R)-((((9H-fluoren-9-yl)methoxy)carbonyl)amino)-3-iodopropanoate (36.1 g, 80.0 mmol, CAS: 847413-82-5) was added to the reaction solvent and stirred at room temperature for 1 hour. 3-iodo N-(2,5,8,11-tetraoxatridecan-13-yl)benzamide (35 g, 80.0 mmol), Pd₂(dba)₃ (4.1 g, 4.0 mmol), and SPhos (6.6 g, 16.0 mmol) were added to the mixture at room temperature and stirred at 50°C for 3 hours. The mixture was diluted with water (1000 mL) and the solid was filtered. The filtrate was extracted 3 times with ethyl acetate (300 mL). The mixed organic layer was washed 3 times with water (500 mL) and twice with saturated saline solution (500 mL), dried with sodium sulphate, and concentrated under reduced pressure. The resulting residue was purified by silica gel column chromatography (petroleum ether/ethyl acetate = 30/70). A portion of the obtained product (17.3 g, 27.3 mmol) was dissolved in isopropanol (180 mL), and calcium chloride (48.4 g, 436 mmol) was added at 0°C, followed by adding lithium hydroxide monohydrate (4.6 g, 109 mmol) solution (60 mL) dropwise. After the drop was completed, the reaction mixture was stirred at room temperature for 2 hours. The reaction mixture was cooled to 0°C, and sodium dihydrogen phosphate solution was added to bring pH = 4. The resulting solid was filtered, and the filtrate was extracted 3 times with ethyl acetate (200 mL). The mixed organic layer was washed 3 times with saturated saline solution (200 mL), dried with sodium sulphate, filtered, and concentrated under reduced pressure. The resulting residue was purified by silica gel column chromatography (petroleum ether/ethyl acetate = 50/50). The titled compound was obtained. ESI-MS (+) observed m/z = 621.3 (M+H)⁺

### [Synthesis Example 1-9]

### Synthesis of Fmoc-F3COO(PEG8Me)-OH

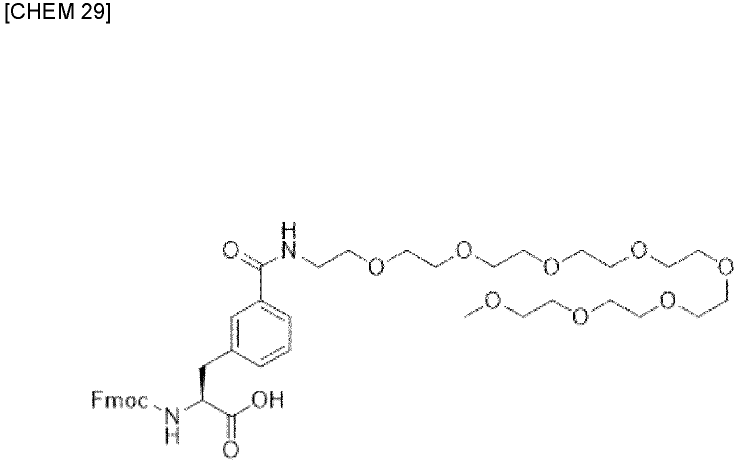

The titled compound was obtained by using 2,5,8,11,14,17,20,23-octaoxapentacosane-25-amine (CAS: 869718-81-0) instead of 2,5,8,11-tetraoxatridecan-13-amine and performing the synthesis in the same manner as Fmoc-F3COO(PEG4Me)-OH. ESI-MS (+) observed m/z = 797.6 (M+H)⁺

### [Synthesis Example 1-10]

### Synthesis of Fmoc-Kmor-OH Hydrochloride

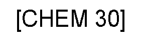

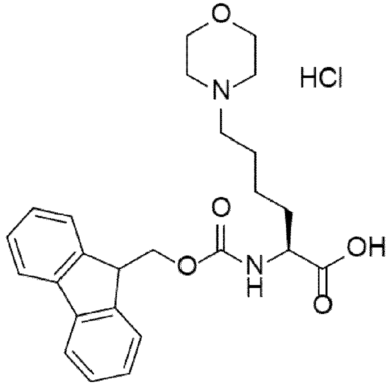

Methyl ((benzyloxy)carbonyl)-L-lysinate hydrochloride (15 g, 45.3 mmol, CAS: 26348-68-5) was dissolved in DMF (91 mL). Potassium carbonate (25.1 g, 181 mmol), 1-bromo-2-(2-bromoethoxy)ethane (12.6 g, 54.4 mmol, CAS: 5414-19-7) was added at room temperature, and the reaction solution was stirred at 60°C for 6 hours. After quenching the reaction solution with ammonium chloride solution, the mixture was extracted twice with ethyl acetate (100 mL), and the mixed organic layer was washed with water and saturated saline solution and dried with sodium sulphate. The mixture was filtered, and the filtrate was concentrated under reduced pressure. The obtained residue was purified by silica gel column chromatography (dichloromethane/methanol = 97/3-60/40). The resulting product (15 g, 41.2 mmol) was dissolved in methanol (206 mL), and 10% Pd/C (4.4 g, 4.12 mmol) and triethylsilane (26.3 mL, 162 mmol) were added at 0°C and stirred for 1 hour. The mixture was filtered through Celite, and the solid was washed with methanol, then the filtrate was concentrated under reduced pressure. The resulting residue was diluted with ethyl acetate and washed 3 times with water. It was dried with sodium sulphate, filtered, and the filtrate was concentrated under reduced pressure. The resulting residue was dissolved in 1,4-dioxane (62 mL), and sodium hydrogen carbonate (13.8 g, 165 mmol) and water (82 mL) were added, then Fmoc-OSu (13.2 g, 39.1 mmol) in 1,4-dioxane (62 mL) was added at 0°C. The mixture was stirred overnight. The reaction solution was extracted twice with ethyl acetate and mixed. The organic layer was washed with saturated saline solution, dried with sodium sulphate, filtered, and concentrated under reduced pressure. The resulting residue was diluted with water, and 1N hydrochloric acid was added until pH reached 1-2, and the mixture was washed with ethyl acetate. Sodium hydrogen carbonate was added until the aqueous layer reached pH 6, extracted twice with ethyl acetate, mixed, and washed with saturated saline solution. After drying with sodium sulphate, the mixture was filtered and concentrated under reduced pressure. The resulting residue was dissolved in 1,4-dioxane (85 mL), then 4N hydrogen chloride 1,4-dioxane solution (85 mL) and water (85 mL) were added, and the mixture was stirred at 60°C for 4 hours. After concentration, 4N hydrogen chloride 1,4-dioxane solution (30 mL) was added and the mixture was stirred at 60°C overnight. The reaction mixture was concentrated under reduced pressure and azeotroped with toluene. N-hexane was added to the resulting residue and concentrated under reduced pressure. The titled compound was obtained. ESI-MS (+) observed m/z = 439.4 (M+H)⁺

### [Synthesis Example 1-11]

### Synthesis of Fmoc-MeF3COO(PEG4Me)-OH

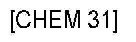

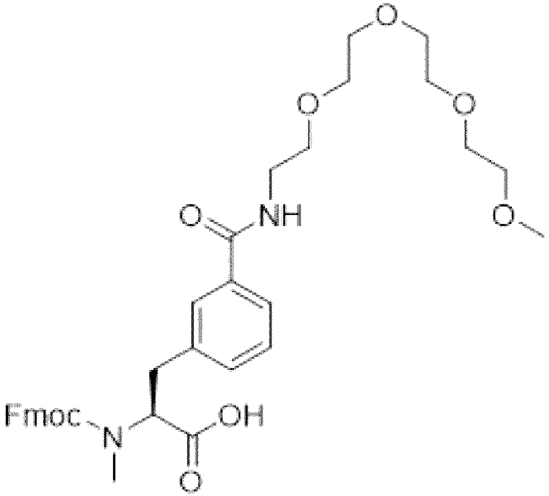

(2S)-3-[3-(tert-butoxycarbonyl)phenyl]-2-{[(9H-fluorene-9-ylmethoxy)carbonyl]amino}propanoic acid (20 g, 41.0 mmol, CAS: 210282-33-0) was suspended in toluene (250 mL), and, at room temperature, [7,7-dimethyl-2-oxobicyclo[2,2,1]heptan-1-yl]methanesulfonic acid (0.57 g, 2.46 mmol, CAS: 3144-16-9) and paraformaldehyde (6.2 g, 205 mmol) were added. The mixture was stirred at 110°C for 1.5 hours. The reaction was quenched by cooling to 0°C with sodium hydrogen carbonate solution, and extracted 3 times with ethyl acetate. The mixed organic layer was washed 3 times with saturated saline solution, dried with sodium sulphate, filtered, and concentrated under reduced pressure. The resulting residue was dissolved in dichloromethane (100 mL), then TFA (100 mL) was added and the mixture was stirred at room temperature for 1 hour. The mixture was concentrated under reduced pressure. The resulting residue was dissolved in DMF (200 mL), then DIPEA (15.7 g, 121.8 mmol) and HATU (18.5 g, 48.7 mmol) were added at room temperature, and 2,5,8,1 1-tetraoxatridecan-13-amine (8.4 g, 40.6 mmol, CAS: 85030-56-4) was added and stirred at room temperature for 3 hours. The reaction was quenched with water at 0°C and extracted 3 times with ethyl acetate. The mixed organic layer was washed 3 times with water, dried with sodium sulphate, filtered, and concentrated under reduced pressure. The resulting residue was purified by silica gel column chromatography (petroleum ether/ethyl acetate = 20/80). The resulting product (21.0 g, 33.2 mmol) was dissolved in dichloroethane (130 mL), and TFA (130 mL) and tris(propan-2-yl)silane (15.8 g, 99.6 mmol) were added at room temperature and stirred at 50°C for 3 hours. The reaction mixture was concentrated under reduced pressure and azeotroped 3 times with toluene. The resulting residue was purified by silica gel column chromatography (dichloromethane/methanol = 95/5). The titled compound was obtained. ESI-MS (+) observed m/z = 635.3 (M+H)⁺

### [Synthesis Example 1-12]

### Synthesis of Fmoc-MeF4COO(PEG4Me)-OH

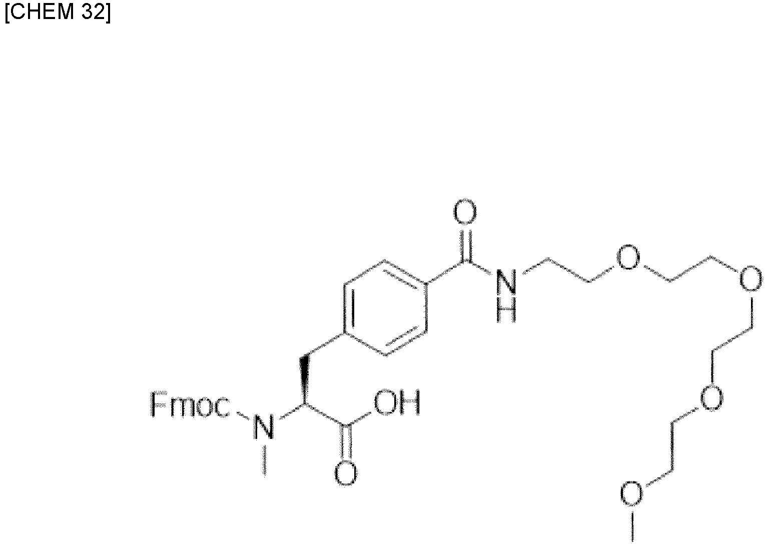

Instead of (2S)-3-[3-(tert-butoxycarbonyl)phenyl]-2-{[(9H-fluorene-9-ylmethoxy)carbonyl]amino}propanoic acid, (2S)-3-[4-(tert-butoxycarbonyl)phenyl]-2-{[(9H-fluorene-9-ylmethoxy)carbonyl]amino}propanoic acid (CAS: 183070-44-2) was used as a starting material and synthesis was performed as the same manner as Fmoc-MeF3COO(PEG4Me)-OH, then the titled compound was obtained. ESI-MS (+) observed m/z = 635.2 (M+H)⁺

### [Synthesis Example 1-13]

### Synthesis of Fmoc-MeF4COO(PEG8Me)-OH

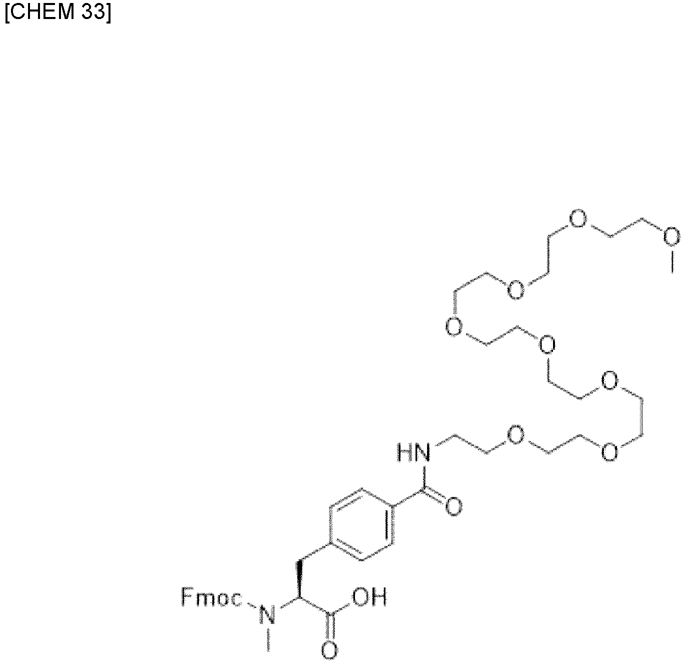

Instead of (2S)-3-[3-(tert-butoxycarbonyl)phenyl]-2-{[(9H-fluorene-9-ylmethoxy)carbonyl]amino}propanoic acid, (2S)-3-[4-(tert-butoxycarbonyl)phenyl]-2-{[(9H-fluorene-9-ylmethoxy)carbonyl]amino}propanoic acid was used as a starting material, and instead of 2,5,8,11-tetraoxatridecan-13-amine, 2,5,8,11,14,17,20,23-octaoxapentacosane-25-amine (CAS: 869718-81-0) was used for synthesis performed as the same manner as Fmoc-MeF3COO(PEG4Me)-OH, then the titled compound was obtained. ESI-MS (+) observed m/z = 811.7 (M + H)⁺

### [Example 7]

### Amino Acid Residue Possessed by Peptide That Bind Strongly to hTfR

Peptides with SEQ ID NOs: 254-262 were chemically synthesized. The sequences of the synthesized peptides are listed in TABLE 4. The synthesized peptides were analyzed as in Example 2, and their structures were confirmed by ESI-MS (+) in mass spectrometry. For each of the synthesized peptides, the surface plasmon resonance (SPR) intermolecular interaction of the peptide with the transferrin receptor (hTfR) was tested by the method shown in Example 3. The results obtained in this way were compared with the peptide "894-3151" (SEQ ID NO:13) synthesized and tested in the above example. The results showed that when the 1st alanine, 4th valine, 6th asparagine, and 9th isoleucine of SEQ ID NO:13 were replaced with different amino acid residues, the KD value increased significantly and the binding ability decreased significantly. These results suggested that these residues are important for maintaining the high binding ability of the peptide to hTfR.

**[TABLE 4]**

| [TABLE 4-1] | | | | | | | | | | | | | | | | | |
|---|---|---|---|---|---|---|---|---|---|---|---|---|---|---|---|---|---|
| Position of Substitution | SEQ ID No. | Name | Seq | | | | | | | | | | | | | | |
| | | | 1 | 2 | 3 | 4 | 5 | 6 | 7 | 8 | 9 | 10 | 11 | 12 | 13 | 14 | 15 |
| Substitution of 1st A | 13 | 894_3151 | A | V | MeF | V | W | N | Y | Y | I | I | R | R | Y | MeY | C |
| | 254 | 894_3m_1F | **F** | V | MeF | V | W | N | Y | Y | I | I | R | R | Y | MeY | C |
| | 255 | 894_3m_1S | S | V | MeF | V | W | N | Y | Y | I | I | R | R | Y | MeY | C |
| | 256 | 894_3m_1G | G | V | MeF | V | W | N | Y | Y | I | I | R | R | Y | MeY | C |
| Substitution of 4th V | 13 | 894_3151 | A | V | MeF | V | W | N | Y | Y | I | I | R | R | Y | MeY | C |
| | 257 | 894_3m_4S | A | V | MeF | S | W | N | Y | Y | I | I | R | R | Y | MeY | C |
| | 258 | 894_3m_4A | A | V | MeF | A | W | N | Y | Y | I | I | R | R | Y | MeY | C |
| | 259 | 894_3m_4G | A | V | MeF | G | W | N | Y | Y | I | I | R | R | Y | MeY | C |
| Substitution of 6th N | 13 | 894_3151 | A | V | MeF | V | W | N | Y | Y | I | I | R | R | Y | MeY | C |
| | 260 | 894_3m_6D | A | V | MeF | V | W | D | Y | Y | I | I | R | R | Y | MeY | C |
| | 261 | 894_3m_6A | A | V | MeF | V | W | A | Y | Y | I | I | R | R | Y | MeY | C |
| Substitution of 9th I | 13 | 894_3151 | A | V | MeF | V | W | N | Y | Y | I | I | R | R | Y | MeY | C |
| | 262 | 894_3m_9F | A | V | MeF | V | W | N | Y | Y | F | I | R | R | Y | MeY | C |

**[TABLE 4-2]**

| Position of Substitution | SEQ ID No. | Linker | | KD (nM) | KD (Each Peptide)/ KD(894_3151) |
|---|---|---|---|---|---|
| Substitution of 1st A | 13 | G | KN3 | 1.3 | 1.0 |
| | 254 | PEG12c | | 110 | 84.6 |
| | 255 | PEG12c | | 24.4 | 18.8 |
| | 256 | PEG12c | | 18.6 | 14.3 |
| Substitution of 4th V | 13 | G | KN3 | 1.3 | 1.0 |
| | 257 | PEG12c | | 164 | 126.2 |
| | 258 | PEG12c | | 177 | 136.2 |
| | 259 | PEG12c | | 952 | 732.3 |
| Substitution of 6th N | 13 | G | KN3 | 1.3 | 1.0 |
| | 260 | PEG12c | | 72.1 | 55.5 |
| | 261 | PEG12c | | 279 | 214.6 |
| Substitution of 9th I | 13 | G | KN3 | 1.8 | 1.4 |
| | 262 | PEG12c | | 218 | 167.7 |

**[TABLE 5]**

| [TABLE 5-1-1] | | | | | | | | | | | | | | | | |
|---|---|---|---|---|---|---|---|---|---|---|---|---|---|---|---|---|
| SEQ ID No. | Name | Seq | | | | | | | | | | | | | | |
| | | 1 | 2 | 3 | 4 | 5 | 6 | 7 | 8 | 9 | 10 | 11 | 12 | 13 | 14 | 15 |
| 263 | 894_3202 | A | V | MeF | V | W | N | Y | Y | I | I | R | R | Y | MeY | C |
| 264 | 894_3203 | A | V | MeF | V | W | N | Y | Y | I | I | R | R | Y | MeY | C |
| 265 | 894_3204 | A | V | MeF3C | V | W1aa | N | Y | F4aao | I | I | R | R | F | MeY | C |
| 266 | 894_3205 | A | V | MeF3C | V | W1aa | N | Y | F4aao | I | I | R | R | F | MeY | C |
| 267 | 894_3206 | A | V | MeF | V | MeW | N | Y | Y | I | I | K | R | Y | MeY | C |
| 268 | 894_3207 | A | V | MeF3C | V | W1aa | N | Y | F4aao | I | I | E(PEG8Me) | R | 4Py | MeY | C |
| 269 | 894_3208 | A | V | MeF3C | V | W | N | 3Py6NH2 | F4aao | I | I | E(PEG8Me) | R | F3COO | MeY | C |
| 270 | 894_3209 | A | V | MeF3C | V | W | N | 3Py6NH2 | F4aao | I | I | E(PEG8Me) | R | F3COO | MeY | C |
| 271 | 894_3210 | A | V | MeF3C | V | W | N | 3Py6NH2 | F4aao | I | I | E(PEG8Me) | R | F3COO | MeY | C |
| 272 | 894_3211 | A | V | MeF3C | V | W | N | 3Py6NH2 | F4aao | I | I | E(PEG8Me) | R | F3COO | MeY | C |
| 273 | 894_3212 | A | V | MeF3C | V | W1aa | N | Y | F4aao | I | I | Kmor | Kmor | F3COO | MeY | C |
| 274 | 894_3213 | A | V | MeF | V | MeW | N | Y | Y | I | I | K | R | Y | MeY | C |
| 275 | 894_3214 | A | V | MeF | V | MeW | N | Y | Y | I | I | K | R | Y | MeY | C |
| 276 | 894_3215 | A | V | MeF | V | MeW | N | Y | Y | I | I | K | R | Y | MeY | C |
| 277 | 894_3216 | A | V | MeF3C | V | W1aa | N | Y | F4aao | I | I | Q | R | F3COO | MeY | C |
| 278 | 894_3217 | A | V | MeF3C | V | W1aa | N | Y | F4aao | I | I | Q | R | F3COO | MeY | C |
| 279 | 894_3218 | A | V | MeF3C | V | W1aa | N | Y | F4aao | I | I | Q | R | F3COO | MeY | C |
| 280 | 894_3219 | A | V | MeF3C | V | W1aa | N | Y | F4aao | I | I | Q | R | F3COO | MeY | C |
| 281 | 894_3220 | A | V | MeF3C | V | W7N | N | Y | F4OMe | I | I | R | R | 4Py | MeY | C |
| 282 | 894_3223 | A | V | MeF3C | V | W1aa | N | Y | F4aao | I | I | Kmor | Kmor | F3COO | MeY | C |
| 283 | 894_3224 | A | V | MeF3C | V | W1aa | N | Y | F4aao | I | I | Kmor | Kmor | F3COO | MeY | C |
| 284 | 894_3231 | A | V | MeF3C | V | W1aa | N | Y | F4aao | I | I | R | R | F | MeY | C |
| 285 | 894_3232 | A | V | MeF3C | V | W1aa | N | Y | F4aao | I | I | Q | R | F3COO | MeY | C |
| 286 | 894_3233 | A | V | MeF3C | V | W7N | N | Y | F4aao | I | I | E(PEG8Me) | R | F3COO | MeY | C |

**[TABLE 5-1-2]**

| SEQ ID No. | Linker | | | | | | | | | | | | m/z | [M+XH]X+ | SPR |
|---|---|---|---|---|---|---|---|---|---|---|---|---|---|---|---|
| 263 | G | G | G | G | S | S | KN3 | | | | | | 1327.36 | 2 | B |
| 264 | G | C | | | | | | | | | | | 1129.09 | 2 | B |
| 265 | G | de | de | de | ds | ds | K | | | | | | 993.41 | 3 | B |
| 266 | G | ds | ds | ds | ds | ds | K | | | | | | 951.42 | 3 | B |
| 267 | G | C | | | | | | | | | | | 1122.12 | 2 | B |
| 268 | G | KN3 | | | | | | | | | | | β28.03 | 3 | C |
| 269 | G | G | G | G | S | S | KN3 | | | | | | 1038.25 | 3 | B |
| 270 | G | MeG | MeG | MeG | MeG | MeG | KN3 | | | | | | 1041.53 | 3 | B |
| 271 | G | KN3 | | | | | | | | | | | 923.08 | 3 | B |
| 272 | G | de | de | de | ds | ds | KN3 | | | | | | 1110.28 | 3 | B |
| 273 | G | KN3 | | | | | | | | | | | 1285.98 | 2 | B |
| 274 | G | G | dr | G | dr | ds | KN3 | | | | | | 936.63 | 3 | B |
| 275 | G | G | G | G | S | S | KN3 | | | | | | 1320.33 | 2 | B |
| 276 | G | MeG | MeG | MeG | MeG | MeG | ICΠt3 | | | | | | 883.97 | 3 | B |
| 277 | G | de | de | de | ds | ds | KN3 | | | | | | 1007.33 | 3 | B |
| 278 | G | G | dr | G | dr | ds | KN3 | | | | | | 1486.72 | 2 | B |
| 279 | G | G | G | G | S | S | KN3 | | | | | | 1402.59 | 2 | B |
| 280 | G | MeG | MeG | MeG | MeG | MeG | KN3 | | | | | | 1407.75 | 2 | B |
| 281 | G | KN3 | | | | | | | | | | | 1171.93 | 2 | A |
| 282 | G | PEG24c | K | | | | | | | | | | 1225.09 | 3 | B |
| 283 | G | ds | ds | ds | ds | ds | K | | | | | | 994.19 | 3 | B |
| 284 | G | ds | ds | ds | ds | ds | ds | ds | ds | ds | ds | K | 1096.49 | 3 | B |
| 285 | G | ds | ds | ds | ds | ds | K | | | | | | 957.06 | 3 | B |
| 286 | G | K | | | | | | | | | | | 914.67 | 3 | A |

**[TABLE 5-2-1]**

| SEQ ID No. | Name | Seq | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 287 | 894_3234 | A | V | MeF3C | V | W1aa | N | Y | F4aao | I | I | Q | R | F3COO | MeY | C |
| 288 | 894_3235 | A | V | MeF3C | V | W1aa | N | Y | F4aao | I | I | R | R | F | MeY | C |
| 289 | 894_3237 | A | V | MeF | V | W | N | Y | Y | I | I | R | R | Y | MeY | C |
| 290 | 894_3238 | A | V | MeF3C | V | W1aa | N | Y | F4aao | I | I | Q | R | F3COO | MeY | C |
| 291 | 894_3239 | A | V | MeF3C | V | W1aa | N | Y | F4aao | I | I | Q | R | F3COO | MeY | C |
| 292 | 894_3240 | A | V | MeF3C | V | W1aa | N | Y | F4aao | I | I | Kmor | Kmor | F3COO | MeY | C |
| 293 | 894_3241 | A | V | MeF3C | V | W1aa | N | Y | F4aao | I | I | Kmor | Kmor | F3COO | MeY | C |
| 294 | 894_3242 | A | V | MeF3C | V | W1aa | N | Y | F4aao | I | I | R | R | F | MeY | C |
| 295 | 894_3243 | A | V | MeF3C | V | W1aa | N | Y | F4aao | I | I | R | R | F | MeY | C |
| 296 | 894_3244 | A | V | MeF | V | W | N | Y | Y | I | I | R | R | Y | MeY | C |
| 297 | 894_3245 | A | V | MeF | V | W | N | Y | Y | I | I | R | R | Y | MeY | C |
| 298 | 894_3246 | A | V | MeF | V | W | N | Y | Y | I | I | R | R | Y | MeY | C |
| 299 | 894_3247 | A | V | MeF | V | MeW | N | Y | Y | I | I | K | R | Y | MeY | C |
| 300 | 894_3248 | A | V | MeF | V | MeW | N | Y | Y | I | I | K | R | Y | MeY | C |
| 301 | 894_3249 | A | V | MeF | V | MeW | N | Y | Y | I | I | K | R | Y | MeY | C |
| 302 | 894_3250 | A | V | MeF3C | V | W1aa | N | Y | F4aao | I | I | R | R | F | MeY | C |
| 303 | 894_3251 | A | V | MeF3C | V | W1aa | N | 3Py6NH2 | F4aao | I | I | R | R | 4Py | MeY | C |
| 304 | 894_3252 | A | V | MeF3C | V | W1aa | N | 3Py6NH2 | F4aao | I | I | E(PEG8Me) | R | 4Py | MeY | C |
| 305 | 894_3253 | A | V | MeF3C | V | W1aa | N | 3Py6NH2 | F4aao | I | I | E(PEG8Me) | R | F3COO | MeY | C |
| 306 | 894_3255 | A | V | MeF3C | V | W1aa | N | Y | F4aao | I | I | E(PEG8Me) | Kmor | 4Py | MeY | C |
| 307 | 894_3256 | A | V | MeF3C | V | W1aa | N | Y | F4aao | I | I | Kmor | Kmor | F3COO | MeY | C |
| 308 | 894_3257 | A | V | MeF3C | V | W1aa | N | Y | F4aao | I | I | E(PEG8Me) | Kmor | 4Py | MeY | C |
| 309 | 894_3258 | A | V | MeF3C | V | W1aa | N | Y | F4aao | I | I | Kmor | Kmor | F3COO | MeY | C |
| 310 | 894_3259 | A | V | MeF3C | V | W1aa | N | Y | F4aao | I | I | R | R | F | MeY | C |

**[TABLE 5-2-2]**

| SEQ ID No. | Linker | | | | | | | | | | | | m/z | [M+XH]X+ | SPR |
|---|---|---|---|---|---|---|---|---|---|---|---|---|---|---|---|
| 287 | G | K | | | | | | | | | | | 1216.88 | 2 | A |
| 288 | G | de | de | de | ds | ds | K(Ac) | | | | | | 1007.37 | 3 | B |
| 289 | G | de | de | de | ds | ds | K(Maleimide) | | | | | | 1021.6 | 3 | B |
| 290 | G | K(Maleimide) | | | | | | | | | | | 1326.55 | 2 | B |
| 291 | G | G | G | G | S | S | K(Maleimide) | | | | | | 999.82 | 3 | B |
| 292 | G | K(Maleimide) | | | | | | | | | | | 922.1 | 3 | B |
| 293 | G | G | G | G | S | S | K(Maleimide) | | | | | | 1037.21 | 3 | B |
| 294 | G | G | G | G | S | S | K(Ac) | | | | | | 1402.56 | 2 | B |
| 295 | G | MeG | MeG | MeG | MeG | MeG | K(Ac) | | | | | | 1407.65 | 2 | B |
| 296 | G | V | Cit | KN3 | | | | | | | | | 1282.76 | 2 | B |
| 297 | G | E | V | Cit | KN3 | | | | | | | | 1347.34 | 2 | B |
| 298 | G | G | G | F | G | KN3 | | | | | | | 1313.75 | 2 | B |
| 299 | G | V | Cit | KN3 | | | | | | | | | 1275.76 | 2 | B |
| 300 | G | E | V | Cit | KN3 | | | | | | | | 893.88 | 3 | B |
| 301 | G | G | G | F | G | KN3 | | | | | | | 1306.79 | 2 | B |
| 302 | G | ds | ds | ds | ds | ds | ds | ds | ds | ds | ds | K(Ac) | 1110.9 | 3 | B |
| 303 | G | KN3 | | | | | | | | | | | 815.68 | 3 | B |
| 304 | G | KN3 | | | | | | | | | | | 928.11 | 3 | B |
| 305 | G | KN3 | | | | | | | | | | | 942.45 | 3 | B |
| 306 | G | de | de | de | ds | ds | K | | | | | | 1120.62 | 3 | B |
| 307 | G | de | de | de | ds | ds | K | | | | | | 1036.15 | 3 | B |
| 308 | G | ds | ds | ds | ds | ds | K | | | | | | 1078.85 | 3 | B |
| 309 | G | ds | ds | ds | ds | ds | K(Ac) | | | | | | 1008.49 | 3 | B |
| 310 | G | ds | ds | ds | de | de | K(Ac) | | | | | | 993.49 | 3 | B |

**[TABLE 5-3-2]**

| SEQ ID No. | Name | Seq | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 311 | 894_3260 | A | V | MeF3C | V | W1aa | N | Y | F4aao | I | I | R | R | F | MeY | C |
| 312 | 894_3261 | A | V | MeF3C | V | W1aa | N | Y | F4aao | I | I | R | R | F | MeY | C |
| 313 | 894_3262 | A | V | MeF3C | V | W1aa | N | Y | F4aao | I | I | R | R | F | MeY | C |
| 314 | 894_3263 | A | V | MeF3C | V | W1aa | N | Y | F4aao | I | I | R | R | F | MeY | C |
| 315 | 894_3264 | A | V | MeF3C | V | W1aa | N | Y | F4aao | I | I | R | R | F | MeY | C |
| 316 | 894_3265 | A | V | MeF3C | V | W1aa | N | Y | F4aao | I | I | R | R | F | MeY | C |
| 317 | 894_3266 | A | V | MeF3C | V | W1aa | N | Y | F4aao | I | I | R | R | F | MeY | C |
| 318 | 894_3267 | A | V | MeF3C | V | W1aa | N | Y | F4aao | I | I | R | R | F | MeY | C |
| 319 | 894_3268 | A | V | MeF3C | V | W1aa | N | Y | F4aao | I | I | R | R | F | MeY | C |
| 320 | 894_3269 | A | V | MeF3C | V | W1aa | N | Y | F4aao | I | I | R | R | F | MeY | C |
| 321 | 894_3270 | A | V | MeF3C | V | W1aa | N | Y | F4aao | I | I | R | R | F | MeY | C |
| 322 | 894_3271 | A | V | MeF3C | V | W1aa | N | Y | F4aao | I | I | R | R | F | MeY | C |
| 323 | 894_3272 | A | V | MeF3C | V | W1aa | N | Y | F4aao | I | I | R | R | F | MeY | C |
| 324 | 894_3273 | A | V | MeF3C | V | W1aa | N | Y | F4aao | I | I | R | R | F | MeY | C |
| 325 | 894_3280 | A | V | MeF | V | W | N | Y | Y | I | I | R | R | Y | MeY | C |
| 326 | 894_3282 | A | V | MeF3C | V | W1aa | N | Y | F4aao | I | I | R | R | F | MeY | C |
| 327 | 894_3283 | A | V | MeF3C | V | W1aa | N | Y | F4aao | I | I | R | R | F | MeY | C |
| 328 | 894_3284 | A | V | MeF3C | V | W1aa | N | Y | F4aao | I | I | R | R | F | MeY | C |
| 329 | 894_3285 | A | V | MeF3C | V | W1aa | N | Y | F4aao | I | I | R | R | F | MeY | C |
| 330 | 894_3286 | A | V | MeF3C | V | W1aa | N | Y | F4aao | I | I | R | R | F | MeY | C |
| 331 | 894_3287 | A | V | MeF3C | V | W1aa | N | Y | F4aao | I | I | R | R | F | MeY | C |
| 332 | 894_3288 | A | V | MeF3C | V | W1aa | N | Y | F4aao | I | I | R | R | F | MeY | C |
| 333 | 894_3289 | A | V | MeF3C | V | W1aa | N | Y | F4aao | I | I | R | R | F | MeY | C |
| 334 | 894_3290 | A | V | MeF3C | V | W1aa | N | Y | F4aao | I | I | R | R | F | MeY | C |

**[TABLE 5-3-2]**

| SEQ ID No. | Linker | | | | | | | | m/z | [M+XH]X+ | SPR |
|---|---|---|---|---|---|---|---|---|---|---|---|
| 311 | G | ds | ds | de | de | ds | K(Ac) | | 993.81 | 3 | B |
| 312 | G | ds | ds | de | ds | de | K(Ac) | | 993.8 | 3 | A |
| 313 | G | de | ds | ds | de | ds | K(Ac) | | 993.9 | 3 | B |
| 314 | G | ds | de | ds | de | ds | K(Ac) | | 993.48 | 3 | B |
| 315 | G | de | ds | ds | ds | ds | K(Ac) | | 979.79 | 3 | B |
| 316 | G | ds | ds | de | ds | ds | K(Ac) | | 979.52 | 3 | B |
| 317 | G | ds | ds | ds | ds | de | K(Ac) | | 979.84 | 3 | A |
| 318 | G | de | ds | ds | ds | de | K(Ac) | | 1489.7 | 2 | B |
| 319 | G | de | de | ds | ds | ds | K(Ac) | | 1489.74 | 2 | B |
| 320 | G | ds | de | de | ds | ds | K(Ac) | | 1489.7 | 2 | B |
| 321 | G | ds | de | ds | ds | de | K(Ac) | | 1489.72 | 2 | B |
| 322 | G | de | ds | de | ds | ds | K(Ac) | | 1489.74 | 2 | B |
| 323 | G | ds | de | ds | ds | ds | K(Ac) | | 979.78 | 3 | B |
| 324 | G | ds | ds | ds | de | ds | K(Ac) | | 1468.66 | 2 | B |
| 325 | G | de | de | de | ds | ds | K | | 1422.37 | 2 | B |
| 326 | G | ds | de | de | de | ds | K(Ac) | | 1007.51 | 3 | B |
| 327 | G | ds | ds | de | de | de | K(Ac) | | 1007.49 | 3 | B |
| 328 | G | de | de | ds | ds | de | K(Ac) | | 1007.49 | 3 | B |
| 329 | G | de | ds | ds | de | de | K(Ac) | | 1007.52 | 3 | B |
| 330 | G | de | de | ds | de | ds | K(Ac) | | 1007.52 | 3 | B |
| 331 | G | de | ds | de | de | ds | K(Ac) | | 1007.49 | 3 | B |
| 332 | G | ds | de | de | ds | de | K(Ac) | | 1007.49 | 3 | B |
| 333 | G | ds | de | ds | de | de | K(Ac) | | 1007.47 | 3 | B |
| 334 | G | de | ds | de | ds | de | K(Ac) | | 1007.48 | 3 | A |

**[TABLE 5-4-1]**

| SEQ ID No. | Name | Seq | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 335 | 894_3309 | A | V | MeF3C | V | W | N | 3Py6NH2 | F4aao | I | I | E(PEG8Me) | R | F3COO | MeY | C |
| 336 | 894_3313 | A | V | MeF3C | V | W | N | 3Py6NH2 | F4aao | I | I | E(PEG8Me) | R | F3COO | MeY | C |
| 337 | 894_3314 | A | V | MeF3C | V | W | N | 3Py6NH2 | F4aao | I | I | E(PEG8Me) | R | F3COO | MeY | C |
| 338 | 894_3315 | A | V | MeF3C | V | W | N | 3Py6NH2 | F4aao | I | I | E(PEG8Me) | R | F3COO | MeY | C |
| 339 | 894_3316 | A | V | MeF3C | V | W | N | 3Py6NH2 | F4aao | I | I | E(PEG8Me) | R | F3COO | MeY | C |
| 340 | 894_3317 | A | V | MeF3C | V | W | N | 3Py6NH2 | F4aao | I | I | E(PEG8Me) | R | F3COO | MeY | C |
| 341 | 894_3318 | A | V | MeF3C | V | W | N | 3Py6NH2 | F4aao | I | I | E(PEG8Me) | R | F3COO | MeY | C |
| 342 | 894_3319 | A | V | MeF3C | V | W | N | 3Py6NH2 | F4aao | I | I | E(PEG8Me) | R | F3COO | MeY | C |
| 343 | 894_3320 | A | V | MeF3C | V | W | N | 3Py6NH2 | F4aao | I | I | E(PEG8Me) | R | F3COO | MeY | C |
| 344 | 894_3321 | A | V | MeF3C | V | W | N | 3Py6NH2 | F4aao | I | I | E(PEG8Me) | R | F3COO | MeY | C |
| 345 | 894_3323 | A | V | MeF3C | V | W7N | N | 3Py6NH2 | F4aao | I | I | E(PEG8Me) | R | F3COO | MeY | C |
| 346 | 894_3325 | A | V | MeF3C | V | Wlaa | N | Y | F4aao | I | I | Q | Kmor | 4Py | MeY | C |
| 347 | 894_3326 | A | V | MeF3C | V | W7N | N | Y | F4aao | I | I | E(PEG8Me) | R | F3COO | MeY | C |
| 348 | 894_3328 | A | V | MeF3C | V | Wlaa | N | Y | F4aao | I | I | Kmor | Kmor | F3COO | MeY | C |
| 349 | 894_3329 | A | V | MeF | V | W | N | Y | Y | I | I | R | R | Y | MeY | C |
| 350 | 894_3331 | A | V | MeF | V | W | N | Y | Y | I | I | R | R | Y | MeY | C |
| 351 | 894_3358 | A | V | MeF3C | V | Wlaa | N | Y | F4aao | I | I | Q | Kmor | 4Py | MeY | C |
| 352 | 894_3367 | A | V | MeF | V | MeW | N | Y | Y | I | I | K | R | Y | MeY | C |
| 353 | 894_3384 | A | V | MeF3C | V | Wlaa | N | Y | F4aao | I | I | E(PEG8Me) | R | 4Py | MeY | C |
| 354 | 894_3385 | A | V | MeF3C | V | Wlaa | N | Y | F4aao | I | I | Kmor | Kmor | F3COO | MeY | C |
| 355 | 894_3386 | A | V | MeF3C | V | W1aa | N | Y | F4aao | I | I | Q | R | F3COO | MeY | C |
| 356 | 894_3389 | A | V | MeF3C | V | W1aa | N | Y | F4aao | I | I | Q | R | F3COO | MeY | C |
| 357 | 894_3392 | A | V | MeF3C | V | W | N | 3Py6NH2 | F4OMe | I | I | R | R | 4Py | MeY | C |
| 358 | 894_3393 | A | V | MeF | V | MeW | N | Y | Y | I | I | K | R | Y | MeY | C |

**[TABLE 5-4-2]**

| SEQ ID No. | Linker | | | | | | | | m/z | [M+XH]X+ | SPR |
|---|---|---|---|---|---|---|---|---|---|---|---|
| 335 | G | ds | ds | ds | ds | ds | KN3 | | 1068.57 | 3 | B |
| 336 | G | de | de | ds | ds | ds | dk | | 1087.61 | 3 | B |
| 337 | G | de | ds | ds | ds | ds | dk | | 1073.57 | 3 | B |
| 338 | G | G | G | G | S | S | dk | | 1029.53 | 3 | B |
| 339 | G | G | G | G | ds | ds | dk | | 1029.53 | 3 | B |
| 340 | G | G | ds | G | ds | G | dk | | 1029.52 | 3 | B |
| 341 | G | ds | ds | ds | ds | ds | dk | | 1059.59 | 3 | B |
| 342 | G | S | S | S | S | S | dk | | 1059.6 | 3 | B |
| 343 | G | ds | ds | ds | de | de | dk | | 1087.58 | 3 | B |
| 344 | G | ds | ds | ds | ds | de | dk | | 1073.57 | 3 | B |
| 345 | G | de | de | de | ds | ds | KN3 | | 1110.58 | 3 | C |
| 346 | G | de | de | de | ds | ds | KN3 | | 1007.13 | 3 | B |
| 347 | G | de | de | de | ds | ds | KN3 | | 1110.6 | 3 | B |
| 348 | G | de | de | de | ds | ds | KN3 | | 1044.89 | 3 | B |
| 349 | G | R | R | R | KN3 | | | | 926.65 | 3 | B |
| 350 | G | dr | dr | dr | KN3 | | | | 926.65 | 3 | B |
| 351 | G | KN3 | | | | | | | 1229.43 | 2 | B |
| 352 | G | G | G | G | S | S | C | | 864.01 | 3 | B |
| 353 | G | ds | ds | ds | ds | ds | KN3 | | 1073.25 | 3 | B |
| 354 | G | ds | ds | ds | ds | ds | KN3 | | 1002.88 | 3 | B |
| 355 | G | ds | ds | ds | ds | ds | KN3 | | 1447.65 | 2 | B |
| 356 | G | KN3 | | | | | | | 1230.38 | 2 | A |
| 357 | G | G | G | G | S | S | K(Maleimide) | | 961.18 | 3 | ND |
| 358 | G** | | | | | | | | 1071.06 | 2 | ND |

**[TABLE 5-5-1]**

| SEQ ID No. | Name | Seq | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 359 | 894_3394 | A | V | MeF3C | V | W1aa | N | Y | F4aao | I | I | E(PEG8Me) | R | F | MeY | C |
| 360 | 894_3226 | A | V | MeF | V | MeW | N | Y | Y | I | I | K | R | Y | MeY | C |
| 361 | 894_3227 | A | V | MeF3C | V | W1aa | N | Y | F4aao | I | I | Q | R | F3COO | MeY | C |
| 362 | 894_3228 | A | V | MeF3C | V | W | N | 3Py6NH2 | F4aao | I | I | E(PEG8Me) | R | F3COO | MeY | C |
| 363 | 894_3236 | A | V | MeF3C | V | W1aa | N | Y | F4aao | I | I | R | R | F | MeY | C |
| 364 | 894_3291 | A | V | MeF3C | V | W1aa | N | Y | F4aao | I | I | E(PEG8Me) | R | F | MeY | C |
| 365 | 894_3292 | A | V | MeF3C | V | W | N | Y | F4aao | I | I | E(PEG4Me) | COpipza | F | MeY | C |
| 366 | 894_3293 | A | V | MeF3C | V | W1aa | N | Y | F4aao | I | I | E(PEG4Me) | Kmor | F | MeY | C |
| 367 | 894_3295 | A | V | MeF3C | V | W1aa | N | Y | F4aao | I | I | E(PEG8Me) | Kmor | 4Py | MeY | C |
| 368 | 894_3300 | A | V | MeF3C | V | W7N | N | 3Py6NH2 | F4aao | I | I | E(PEG8Me) | R | F3COO | MeY | C |
| 369 | 894_3305 | A | V | MeF3C | V | W | N | 3Py6NH2 | F4aao | I | I | E(PEG8Me) | R | F3COO | MeY | C |
| 370 | 894_3306 | A | V | MeF3C | V | W | N | 3Py6NH2 | F4aao | I | I | E(PEG8Me) | R | F3COO | MeY | C |
| 371 | 894_3307 | A | V | MeF3C | V | W | N | 3Py6NH2 | F4aao | I | I | E(PEG8Me) | R | F3COO | MeY | C |
| 372 | 894_3308 | A | V | MeF3C | V | W | N | 3Py6NH2 | F4aao | I | I | E(PEG8Me) | R | F3COO | MeY | C |
| 373 | 894_3310 | A | V | MeF3C | V | W | N | 3Py6NH2 | F4aao | I | I | E(PEG8Me) | R | F3COO | MeY | C |
| 374 | 894_3311 | A | V | MeF3C | V | W | N | 3Py6NH2 | F4aao | I | I | E(PEG8Me) | R | F3COO | MeY | C |
| 375 | 894_3312 | A | V | MeF3C | V | W | N | 3Py6NH2 | F4aao | I | I | E(PEG8Me) | R | F3COO | MeY | C |
| 376 | 894_3347 | A | V | MeF | V | W | N | Y | Y | I | I | R | R | Y | MeY | C |
| 377 | 894_3349 | A | V | MeF3C | V | W1aa | N | Y | F4aao | I | I | Q | R | F3COO | MeY | C |
| 378 | 894_3350 | A | V | MeF3C | V | W1aa | N | Y | F4aao | I | I | Q | R | F3COO | MeY | C |
| 379 | 894_3351 | A | V | MeF3C | V | W1aa | N | Y | F4aao | I | I | Q | R | F3COO | MeY | C |
| 380 | 894_3352 | A | V | MeF3C | V | W | N | 3Py6NH2 | F40Me | I | I | R | R | 4Py | MeY | C |
| 381 | 894_3353 | A | V | MeF3C | V | W | N | 3Py6NH2 | F4OMe | I | I | R | R | 4Py | MeY | C |
| 382 | 894_3354 | A | V | MeF3C | V | W | N | 3Py6NH2 | F4OMe | I | I | R | R | 4Py | MeY | C |

**[TABLE 5-5-2]**

| SEQ ID No. | Linker | | | | | | | | m/z | [M+XH]X+ | SPR |
|---|---|---|---|---|---|---|---|---|---|---|---|
| 359 | G | de | de | de | ds | ds | KN3 | | 1114.85 | 3 | ND |
| 360 | G | de | de | de | ds | ds | C | | 1402.96 | 2 | ND |
| 361 | G | G | G | G | S | S | C | | 1377.08 | 2 | ND |
| 362 | G | G | G | G | S | S | C | | 1021.25 | 3 | ND |
| 363 | G | ds | ds | ds | ds | ds | K(Ac) | | 965.35 | 3 | ND |
| 364 | G | de | de | de | ds | ds | dk | | 1106.63 | 3 | ND |
| 365 | G | de | de | de | ds | ds | dk | | 1075.9 | 3 | ND |
| 366 | G | de | de | de | ds | ds | dk | | 1061.92 | 3 | ND |
| 367 | G | de | de | de | ds | ds | dk | | 1120.94 | 3 | ND |
| 368 | G | de | de | de | ds | ds | dk | | 1102.34 | 3 | ND |
| 369 | G | de | de | ds | ds | ds | KN3 | | 1096.62 | 3 | ND |
| 370 | G | de | ds | ds | ds | ds | KN3 | | 1082.58 | 3 | ND |
| 371 | G | G | G | G | ds | ds | KN3 | | 1038.49 | 3 | ND |
| 372 | G | G | ds | G | ds | G | KN3 | | 1038.51 | 3 | ND |
| 373 | G | S | S | S | S | S | KN3 | | 1068.52 | 3 | ND |
| 374 | G | ds | ds | ds | de | de | KN3 | | 1096.54 | 3 | ND |
| 375 | G | ds | ds | ds | ds | de | KN3 | | 1082.61 | 3 | ND |
| 376 | G | de | de | de | ds | ds | dk | | 948.66 | 3 | ND |
| 377 | G | G | G | G | S | S | K(C5Mal) | | 991.19 | 3 | ND |
| 378 | PEG4c | K(C5Mal) | | | | | | | 939.47 | 3 | ND |
| 379 | G | de | de | de | ds | ds | K(C5Mal) | | 1063.22 | 3 | ND |
| 380 | G | G | G | G | S | S | K(C5Mal) | | 1427.74 | 2 | ND |
| 381 | PEG4c | K(C5Mal) | | | | | | | 1350.17 | 2 | ND |
| 382 | G | de | de | de | ds | ds | K(C5Mal) | | 768.47 | 4 | ND |

**[TABLE 5-6-1]**

| SEQ ID No. | Name | Seq | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 383 | 894_3355 | A | V | MeF | V | W | N | Y | Y | I | I | R | R | Y | MeY | C |
| 384 | 894_3360 | A | V | MeF3C | V | Wlaa | N | 3Py6NH2 | F4aao | I | I | R | R | 4Py | MeY | C |
| 385 | 894_3361 | A | V | MeF3C | V | Wlaa | N | 3Py6NH2 | F4aao | I | I | E(PEG8Me) | R | 4Py | MeY | C |
| 386 | 894_3362 | A | V | MeF3C | V | Wlaa | N | 3Py6NH2 | F4aao | I | I | E(PEG8Me) | R | F3COO | MeY | C |
| 387 | 894_3364 | A | V | MeF3C | V | W | N | 3Py6NH2 | F4aao | I | I | E(PEG8Me) | R | F3COO | MeY | C |
| 388 | 894_3366 | A | V | MeF3C | V | W | N | Y | F4OMe | I | I | R | R | F | MeY | C |
| 389 | 894_3376 | A | V | MeF3C | V | Wlaa | N | Y | F4aao | I | I | Q | R | F3COO | MeY | C |
| 390 | 894_3377 | A | V | MeF3C | V | W | N | 3Py6NH2 | F4OMe | I | I | R | R | 4Py | MeY | C |
| 391 | 894_3378 | A | V | MeF3C | V | W7N | N | Y | F4OMe | I | I | R | R | 4Py | MeY | C |
| 392 | 894_3381 | A | V | MeF3C | V | Wlaa | N | Y | F4aao | I | I | Kmor | Kmor | F3COO | MeY | C |
| 393 | 894_3382 | A | V | MeF3C | V | Wlaa | N | Y | F4aao | I | I | Kmor | Kmor | F3COO | MeY | C |
| 394 | 894_3383 | A | V | MeF3C | V | Wlaa | N | Y | F4aao | I | I | Kmor | Kmor | F3COO | MeY | C |

| | | | | | | | | | | | | | | | | |
|---|---|---|---|---|---|---|---|---|---|---|---|---|---|---|---|---|
| **: C-terminus is -OH | | | | | | | | | | | | | | | | |

**[TABLE 5-6-2]**

| SEQ ID No. | Linker | | | | | | | | m/z | [M+XH]X+ | SPR |
|---|---|---|---|---|---|---|---|---|---|---|---|
| 383 | G | de | de | de | ds | ds | K(Ac) | | 1443.56 | 2 | ND |
| 384 | G | de | de | de | ds | ds | dk | | 994.16 | 3 | ND |
| 385 | G | de | de | de | ds | ds | dk | | 1107.03 | 3 | ND |
| 386 | G | de | de | de | ds | ds | dk | | 1120.86 | 3 | ND |
| 387 | G | de | de | de | ds | ds | dk | | 1101.54 | 3 | ND |
| 388 | G | de | de | de | ds | ds | dk | | 959.39 | 3 | ND |
| 389 | G | PEG24c | K | | | | | | 1187.74 | 3 | ND |
| 390 | G | de | de | de | ds | ds | dk | | 960.17 | 3 | ND |
| 391 | G | de | de | de | ds | ds | dk | | 960.49 | 3 | ND |
| 392 | G | de | de | de | ds | ds | K(C5Mal) | | 1100.59 | 3 | ND |
| 393 | G | G | G | G | S | S | K(C5Mal) | | 1028.55 | 3 | ND |
| 394 | PEG4c | K(C5Mal) | | | | | | | 976.8 | 3 | ND |

**[TABLE 6]**

| [TABLE 6-1] | | | | | | | | | | | | | | | |
|---|---|---|---|---|---|---|---|---|---|---|---|---|---|---|---|
| Name | Seq | | | | | | | | | | | | | | |
| | 1 | 2 | 3 | 4 | 5 | 6 | 7 | 8 | 9 | 10 | 11 | 12 | 13 | 14 | 15 |
| 894_3368 | A | V | MeF3C | V | W | N | 3Py6NH2 | F4aao | I | I | E(PEG8Me) | R | F3COO | MeY | C |
| 894_3369 | A | V | MeF3C | V | W7N | N | 3Py6NH2 | F4aao | I | I | E(PEG8Me) | R | F3COO | MeY | C |
| 894_3370 | A | V | MeF3C | V | W | N | Y | F4aao | I | I | E(PEG4Me) | KCOpipzaa | F | MeY | C |
| 894_3371 | A | V | MeF3C | V | W1aa | N | Y | F4aao | I | I | Q | Kmor | 4Py | MeY | C |
| 894_3372 | A | V | MeF3C | V | W7N | N | Y | F4aao | I | I | E(PEG8Me) | R | F3COO | MeY | C |
| 894_3373 | A | V | MeF3C | V | W1aa | N | Y | F4aao | I | I | E(PEG8Me) | R | 4Py | MeY | C |
| 894_3374 | A | V | MeF3C | V | W1aa | N | Y | F4aao | I | I | Kmor | Kmor | F3COO | MeY | C |
| 894_3375 | A | V | MeF3C | V | W1aa | N | Y | F4aao | I | I | Q | R | F3COO | MeY | C |
| 894_0501 | A | V | MeF | V | MeW | N | Y | Y | I | I | K | R | Y | MeY | C |
| 894_0502 | A | V | MeF3C | V | W | N | 3Py6NH2 | F4aao | I | I | E(PEG8Me) | R | F3COO | MeY | C |
| 894_3221 | A | V | MeF | V | MeW | N | Y | Y | I | I | K | R | Y | MeY | C |
| 894_3222 | A | V | MeF | V | MeW | N | Y | Y | I | I | K | R | Y | MeY | C |
| 894_33808 | A | V | MeF3C | V | W1aa | N | Y | F4aao | I | I | Kmor | Kmor | F3COO | MeY | C |
| 894_33809 | A | V | MeF3C | V | W1aa | N | Y | F4aao | I | I | Kmor | Kmor | F3COO | MeY | C |

**[TABLE 6-2]**

| Linker | | | | | | | | Payload | m/z | [M+XH]X+ |
|---|---|---|---|---|---|---|---|---|---|---|
| G | de | de | de | ds | ds | KN3 | | Cy5SAlk | 1336.8 | 3 |
| G | de | de | de | ds | ds | KN3 | | Cy5SAlk | 1337.1 | 3 |
| G | de | de | de | ds | ds | KN3 | | Cy5SAlk | 1310.8 | 3 |
| G | de | de | de | ds | ds | KN3 | | Cy5SAlk | 1233.7 | 3 |
| G | de | de | de | ds | ds | KN3 | | Cy5SAlk | 1337.1 | 3 |
| G | de | de | de | ds | ds | KN3 | | Cy5SAlk | 1341.8 | 3 |
| G | de | de | de | ds | ds | KN3 | | Cy5SAlk | 1271.4 | 3 |
| G | de | de | de | ds | ds | KN3 | | Cy5SAlk | 1234.0 | 3 |
| G | de | de | de | ds | ds | C | | Maleimide-AF647 | 1262.7 | * |
| G | G | G | G | S | S | C | | Maleimide-AF647 | 1348.2 | * |
| G | de | de | de | ds | ds | | | Signal Peptide-KN3-NH2 | 1135.2 | 4 |
| G | ds | ds | ds | ds | ds | | | Signal Peptide-KN3-NH2 | 1471.2 | 3 |
| G | de | de | de | ds | ds | dk(@) | KN3 | Signal Peptide | 1246.9 | 4 |
| G | de | de | de | ds | ds | | | Signal Peptide-KN3-NH2 | 1204.8 | 4 |

## Claims

1. A peptide comprising an amino acid sequence described in Ala-Val-MePhe-Val-Trp-Asn-Tyr-Tyr-Ile-Ile-K(MePEG4c)-Arg-Phe-MeTyr-Cys (SEQ ID NO: 1), or the peptide comprising one or more substitutions in the amino acid sequence described in SEQ ID NO:1, said substitutions being selected from the following group:
(I) substitution of the 1st alanine residue in the SEQ ID NO: 1 for a MeA residue;
(II) substitution of the 3rd N-methylphenylalanine residue in the SEQ ID NO: 1 for an amino acid which contain an aromatic ring, which may have a substituent, in a side chain, or an N-methylamino acid;
(III) substitution of the 5th tryptophan residue in the SEQ ID NO: 1 for tryptophan which has a substituent or N-methyltryptophan;
(IV) substitution of the 6th asparagine residue of the SEQ ID NO: 1 for alanine or a positively charged amino acid;
(V) substitution of the 7th tyrosine residue of the SEQ ID NO: 1 for an amino acid having an aromatic ring, which may have a substituent, in a side chain, or an alkyl group of C₄ or more;
(VI) substitution of the 8th tyrosine residue of the SEQ ID NO: 1 for an amino acid having an aromatic ring, which may have a substituent which may have a linker, in a side chain, or N-methylamino acid;
(VII) substitution of the 9th isoleucine residue of the SEQ ID NO: 1 for an aliphatic amino acid;
(VIII) substitution of the 10th isoleucine residue of the SEQ ID NO: 1 for an amino acid having a butyl group, which may have a substituent, in a side chain;
(IX) substitution of the 11th K(MePEG4c) residue of the SEQ ID NO: 1 for Aib, or a hydrophilic amino acid which may have a linker or N-methylamino acid;
(X) substitution of the 12th arginine residue of the SEQ ID NO: 1 for a hydrophilic amino acid (including D-body) which may have a linker, or N-methylamino acid;
(XI) substitution of the 13th phenylalanine of the SEQ ID NO: 1 for an amino acid having an aromatic ring, which may have a linker, in a side chain; or
(XII) substitution of the 14th N-methyltyrosine residue of the SEQ ID NO: 1 for N-methylamino acid having an aromatic ring, which may have a linker, in the side chain.

2. The peptide according to claim 1, wherein the peptide is a peptide having an amino acid sequence with one or more substitution selected from the following group:
(I) substitution of the 3rd N-methylphenylalanine residue of SEQ ID NO: 1 for one amino acid selected from the group consisting of F, MeF3C, MeF4F, Me3Py, Me4Py and MeF3COO;
(II) substitution of the 5th tryptophan residue of SEQ ID NO: 1 for one amino acid selected from the group consisting of W5C, MeW, W1aa and W7N;
(III) substitution of the 6th asparagine residue of SEQ ID NO: 1 for alanine or Ndm;
(IV) substitution of the 7th tyrosine residue of SEQ ID NO: 1 for 3Py6NH2;
(V) substitution of the 8th tyrosine residue of SEQ ID NO: 1 for one amino acid selected from the group consisting of 4Py, F4OMe, F4aao, 3Py, W and F4aao which may have a linker;
(VI) substitution of the 9th isoleucine residue of SEQ ID NO: 1 for alanine;
(VII) substitution of the 10th isoleucine residue in SEQ ID NO: 1 for E;
(VIII) substitution of the 11th K(MePEG4c) residue of SEQ ID NO: 1 for one amino acid selected from the group consisting of Aib, RMeR, K(Mecar), Kmor, K which may have a linker, E(Glucamine), QhEt, Qpipzaa, Q which may have a linker, and E which may have a linker;
(IX) substitution of the 12th arginine residue of SEQ ID NO: 1 with one amino acid selected from the group consisting of da, dr, A4paa, QhEt, Qpipzaa, E(Glucamine), K(Mecar), Kmor, KdMe, KCOpipzaa, Q which may have a linker, E which may have a linker and K to which a linker is attached,
(X) substitution of the 13th phenylalanine of SEQ ID NO: 1 for one amino acid selected from the group consisting of Y, 4Py, F3COO which may have a linker and E which may have a linker; and
(XI) substitution of the 14th N-methyltyrosine residue of SEQ ID NO: 1 for one amino acid selected from the group consisting of Me3Py, MeF4COO which may have a linker and MeF3COO which may have a linker.

3. The peptide according to claim 1 or 2, wherein the peptide comprises an amino acid sequence satisfying at least one of the following (I) to (III):
(I) the 1st amino acid of SEQ ID NO: 1 is alanine residue;
(II) the 6th amino acid of SEQ ID NO: 1 is asparagine residue, and
(III) the 9th amino acid of SEQ ID NO: 1 is isoleucine residue.

4. The peptide according to any one of claims 1 to 3,
wherein the peptide comprising an amino acid sequence moiety which comprises: 1st-15th amino acid sequence of the amino acid sequence described in any one of the SEQ ID NOs: 1 to 241, or 1st-15th amino acid sequence of a conjugate of the amino acid sequence described in any one of the SEQ ID NOs: 1 to 241 with a linker, and
wherein the amino acid sequence moiety has a cyclic structure.

5. The peptide according to any one of claims 1 to 4, wherein the peptide is a cyclic peptide.

6. The peptide according to claim 1, wherein the peptide has hTfR binding activity.

7. The peptide according to claim 1, wherein a linker is attached to the 8th, 11th, 12th, 13th, 14th or 15th amino acid residue of SEQ ID NO: 1.

8. The peptide according to claim 7, wherein the peptide is described in any one of SEQ ID NOs: 10 to 241.

9. The peptide according to claim 7, wherein the linker comprising:
polyethylene glycol (PEG): or
an amino acid sequence indicated by any one of SEQ ID NOs: 242 to 253.

10. A complex comprising the peptide according to claim 7 and a substance bound to the linker.

11. The complex according to claim 10, wherein the linker comprises a sequence indicated by any one of SEQ ID NOs: 242 to 253 and 263 to 394.

12. The complex according to claim 10, wherein the linker is polyethylene glycol (PEG).

13. A composition comprising the peptide according to any one of claims 1 to 9.

14. A composition for pharmaceutical or diagnostic use comprising the peptide according to any one of claims 1 to 9.

15. A composition comprising the complex according to claim 10.

16. A composition for pharmaceutical or diagnostic use comprising the complex according to claim 10.

17. A method for manufacturing a composition for pharmaceutical or diagnostic use, comprising a process of obtaining the complex according to claim 10.

18. The method according to claim 17, wherein the linker is:
polyethylene glycol (PEG); or
a linker comprising a sequence indicated by any one of SEQ ID NOs: 242 to 253 and 263 to 394.

19. A test method for peptide, wherein the peptide is tested at least one of:
a) solubility in a solvent;
b) binding ability to hTfR;
c) toxicity to a cell and/or a tissue; and
d) toxicity to experimental animal,
wherein the peptide has an amino acid sequence in which one to three amino acid residues are deleted, substituted, inserted and/or added in the amino acid sequence possessed by the peptide according to claim 1.
